# EUROPEAN PATENT APPLICATION

(11) **EP 4 671 269 A1**
(43) Date of publication of application: **31.12.2025**
(21) Application number: 24759667.9
(22) Date of filing: 20.02.2024
(51) Int. Cl.: C07K 16/00, C07K 16/28, C07K 14/71, A61K 39/395, A61P 35/00, A61K 38/17

(54) **FUSION PROTEIN COMPRISING TGF-?RII EXTRACELLULAR REGION FRAGMENT, PHARMACEUTICAL COMPOSITION THEREOF AND USE THEREOF**

(30) Priority: 20.02.2023 CN 202310142861
(71) Applicant: Akeso Biopharma Co., Ltd., Zhongshan, Guangdong 528437 (CN)
(72) Inventor: LI, Baiyong, Zhongshan, Guangdong 528437 (CN); XIA, Yu, Zhongshan, Guangdong 528437 (CN)
(74) Representative: Santarelli
(86) International application number: PCT/CN2024/077727
(87) International publication number: WO 2024/174990

(57) **Abstract**

The present disclosure pertains to the fields of biology and pharmaceutics and relates to a fusion protein comprising a TGF-βRII extracellular region fragment, a pharmaceutical composition thereof, and use thereof. Specifically, the fusion protein of the present disclosure comprises: a first protein functional region targeting an immune checkpoint and a second protein functional region with TGF-β binding activity, wherein the second protein functional region is a variant of a TGF-βRII extracellular region fragment, and the variant of the TGF-βRII extracellular region fragment is: the first serine at the N-terminus of the TGF-βRII extracellular region fragment is replaced with alanine, glycine, or threonine, and/or a fragment comprising the amino acid sequence set forth in SEQ ID NO: 59 is deleted from the TGF-βRII extracellular region fragment. The fusion protein of the present disclosure can simultaneously inhibit TIGIT and reduce TGF-β levels, demonstrating good potential for preparing anti-tumor drugs.

## Description

### TECHNICAL FIELD

The present disclosure pertains to the fields of biology and pharmaceutics and relates to a fusion protein comprising a TGF-βRII extracellular region fragment, a pharmaceutical composition thereof, and use thereof.

### BACKGROUND

The transforming growth factor-β (TGF-β) superfamily is a class of functionally diverse cytokines and is further divided into subfamilies such as TGF-β, activins, inhibins, growth differentiation factors (GDFs), glial cell line-derived neurotrophic factors (GDNFs), Nodal, Lefty, and anti-Müllerian hormone (Table A). The three proteins of the TGF-β subfamily are known as TGF-β1, TGF-β2, and TGF-β3, respectively, and TGF-β1 is the most highly expressed subtype. TGF-β1, TGF-β2, and TGF-β3, upon binding to receptors, mediate a range of biological reactions through the Smad and non-Smad signaling pathways, including promotion of epithelial-mesenchymal transitions (EMTs), promotion of tissue fibrosis, promotion of angiogenesis, promotion of tumor immune escape, cancer inhibition and promotion dual effects, etc. (J Massagué. TGFbeta in Cancer [J]. Cell, 2008, 134(2):215-230).

TGF-β receptors (TGF-βRs) are widely distributed on the surface of normal and tumor cells in humans and include three receptor superfamilies: the classical TGF-β receptor family type I includes ALK1-7, and TGF-βRI (also written as TβRI) is also known as ALK5; the classical TGF-β receptor family type II includes TGF-βRII (also written as TβRII), ActRII, ActRIIB, AMHRII, and BMPRII; the TGF-β receptor superfamily type III includes Betaglycan (also known as TGF-βRIII) and Endoglin. TGF-βRI, TGF-βRII, and TGF-βRIII are each able to bind to TGF-β1, TGF-β2, and TGF-β3 (Pawlak John B, Blobe Gerard C, TGF-β superfamily co-receptors in cancer. DevDyn, 2021). The TGF-β family and receptors are shown in Table A (summarized based on the review Carl-Henrik Heldin1 and Aristidis Moustakas. Cold Spring Harb Perspect Biol. 2016). TGF-βRI and TGF-βRII are serine/threonine protein kinase receptors. TGF-βRII, as a key molecule of signal transduction in the TGF-β signaling pathways, can bind to TGF-β with a high affinity and then form a heterotetramer receptor complex with a TGF-βRI dimer. Through the self-phosphorylation of TGF-βRII, TGF-βRI is further phosphorylated and activated. The activated TGF-βRI phosphorylates downstream Smad pathway-related proteins and regulates the transcription and translation of downstream target genes, thereby causing disease-related biological reactions. TGF-βRIII has a weaker affinity for TGF-β than TGF-βRI and TGF-βRII and has no intracellular segment. Thus, it cannot be connected to downstream signaling pathways. Its function is to capture and present TGF-β to TGF-βRII. Other TGF-β receptors can also bind to TGF-β (Sang, X.H., et al., Advances in research on small-molecule inhibitors targeting TGF-β and receptors [J]. Journal of Pharmacy, 2019(9)).

**Table A: The TGF-β family and receptors**

| **Protein name** | **Gene name** | **Other names** | **Receptor** | |
|---|---|---|---|---|
| **TGF-β1** | **TGFB1** | **CIF-A (cartilage-inducing factor-A), differentiation-inhibiting factor** | **ActRL1/ALK-1; TβRI/ALK-5** | **TβRII** |
| **TGF-β2** | **TGFB2** | **G-TsF (glioblastoma-derived T-cell suppresso rfactor), BSC-1GI (BSC-1 cell-growth inhibitor), polyergin, CIF-B (cartilage-inducingfactor-B)** | | |
| **TGF-β3** | **TGFB3** | | | |
| **Inhibinα** | **INHA** | **InhibinA** | **ActRIB/ALK-4** | **ActRIIB; ActRII** |
| **InhibinβA** | **INHBA** | **FRP (follicle-stimulating hormone-releasing protein), EDF (erythroid differentiation factor), XTC-MIF (Xenopus XTC cell mesoderm-inducing factor)** | **ActRIB/ALK-4; ActRIC/ALK-7** | |
| **InhibinβB** | **INHBB** | **InhibinB and activinB or AB,a,b XTC-MIF** | | |
| **InhibinβC** | **INHBC** | **ActivinCc** | | |
| **InhibinβE** | **INHBE** | **ActivinEc** | | |
| **Nodal** | **NODAL** | **BMP-16** | **ActRIB/ALK-4** | **ActRIIB; ActRII** |
| **Myostatin** | **MSTN** | **GDF-8** | **TβRI/ALK-5; ActRIB/ALK-4** | **ActRIIB** |
| **BMP-2** | **BMP2** | | **BMPRIA/ALK-3; BMPRIB/ALK-6** | **BMPRII; ActRIIB; ActRII** |
| **BMP-3** | **BMP3** | **Osteogenin** | | |
| **BMP-4** | **BMP4** | **BMP-2B** | **BMPRIA/ALK-3; BMPRIB/ALK-6** | **BMPRII; ActRIIB; ActRII** |
| **BMP-5** | **BMP5** | | **BMPRIB/ALK-6; BMPRIA/ALK-3; ActRIA/ALK-2** | **BMPRII; ActRIIB; ActRII** |
| **BMP-6** | **BMP6** | **Vgr1 (Vg1-related protein)** | **BMPRIB/ALK-6; BMPRIA/ALK-3; ActRIA/ALK-2** | **BMPRII; ActRIIB; ActRII** |
| **BMP-7** | **BMP7** | **OP-1 (osteogenic protein-1)** | **BMPRIB/ALK-6; BMPRIA/ALK-3; ActRIA/ALK-2** | **BMPRII; ActRIIB; ActRII** |
| **BMP-8A** | **BMP8A** | **OP-2** | **BMPRIB/ALK-6; BMPRIA/ALK-3; ActRIA/ALK-2** | **BMPRII; ActRIIB; ActRII** |
| **BMP-8B** | **BMP8B** | **OP-3** | **BMPRIB/ALK-6; BMPRIA/ALK-3; ActRIA/ALK-2** | **BMPRII; ActRIIB; ActRII** |
| **BMP-9** | **GDF2** | **GDF-2** | **BMPRIB/ALK-6; ActRL1/ALK-1** | **BMPRII; ActRIIB; ActRII** |
| **BMP-10** | **BMP10** | | **BMPRIB/ALK-6; ActRL1/ALK-1** | **BMPRII; ActRIIB; ActRII** |
| **GDF-1** | **GDF1** | | **ActRIB/ALK-4; ActRIC/ALK-7** | **ActRIIB; ActRII** |
| **GDF-3** | **GDF3** | **Vgr2** | **ActRIB/ALK-4; ActRIC/ALK-7** | **ActRIIB; ActRII** |
| **GDF-5** | **GDF5** | **CDMP-1 (cartilage-derived morpho genetic protein-1), BMP-14** | **BMPRIB/ALK-6; BMPRIA/ALK-3** | **BMPRII; ActRIIB; ActRII** |
| **GDF-6** | **GDF6** | **CDMP-2, BMP-13** | **BMPRIB/ALK-6; BMPRIA/ALK-3** | **BMPRII; ActRIIB; ActRII** |
| **GDF-7** | **GDF7** | **CDMP-3, BMP-12** | **BMPRIB/ALK-6; BMPRIA/ALK-3** | **BMPRII; ActRIIB; ActRII** |
| **GDF-9** | **GDF9** | **GDF-9A** | **TβRI/ALK-5; ActRIB/ALK-4; ActRIC/ALK-7** | **ActRIIB** |
| **GDF-9B** | **BMP15** | **BMP-15** | **BMPRIB/ALK-6** | |
| **GDF-10** | **GDF10** | **BMP-3b** | **ActRIB/ALK-4; ActRIB/ALK-4** | **ActRII** |
| **GDF-11** | **GDF11** | **BMP-11** | **TβRI/ALK-5; ActRIB/ALK-4** | **ActRIIB; ActRII** |
| **GDF-15** | **GDF15** | **Placental TGF-β, placental BMP (PLAB), PDF (prostate-derivedfactor), NAG-1 (nonsteroidalanti-inflammatorydrug-activatedgene-1), MIC-1 (macrophage inhibitory cytokine-1)** | **BMPRIA/ALK-3** | **ActRIIB** |
| **MIS (Miillerian-inhibiting substance)** | **AMH** | **AMH (anti-Müllerian hormone)** | **ActRIA/ALK-2;** | **AMURII** |
| **LeftyA** | **LEFTY2** | **EBAF (endometrial bleeding-associated factor), TGF-β4, Stra3** | | |
| **LeftyB** | **LEFTY1** | | | |

During tumor progression, tumor cells, mesenchymal fibroblasts, and other cells secrete large quantities of TGF-β in the tumor microenvironment (TME), mediating immunosuppression. TGF-β inhibits the differentiation of naive T cells to Th1, which mediates anti-tumor activity, and TGF-βRII-deficient T cells have enhanced Th1 responses (Eduard, Battle, Joan, et al., Transforming Growth Factor-β Signaling in Immunity and Cancer [J]. Immunity, 2019). Granzyme A, granzyme B, perforin, γ-interferon (IFN-y), and FasL of effector T cells having tumor killing activity have reduced expression levels under the action of TGF-β, resulting in immune escape of tumor cells. Tregs are key cells for the tumor microenvironment (TME) to mediate tumor immunosuppression and can inhibit the functioning of effector T cells with tumor killing activity. The TGF-β produced by tumor cells induces the emergence of a large number of Tregs in the TME, enhancing the tolerance of tumor antigens and promoting the generation of tumor immune escape (Chen Dan, Ran Yan. Advances in research on the regulation of tumor cells and tumor-associated immune cells by TGF-β [J]. Modern Medicine & Health, 2020, 36(09):1354-1358). Indications for TGF-β molecule-targeting investigational antibody-based drugs that are undergoing clinical trials include melanoma, renal cell carcinoma, breast cancer, cervical cancer, advanced non-small cell lung cancer, prostate cancer, pancreatic ductal adenocarcinoma, advanced solid tumors, and metastatic solid tumors.

TIGIT (T cell Ig and ITIM domain, also known as WUCAM, Vstm3, or VSIG9) is a member of the poliovirus receptor (PVR)/Nectin family. TIGIT consists of an extracellular immunoglobulin variable region (IgV) domain, a type I transmembrane domain, and an intracellular domain with a classical immunoreceptor tyrosine-based inhibitory motif (ITIM) and an immunoglobulin tail tyrosine (ITT) motif. TIGIT is highly expressed in lymphocytes, especially in effector and regulatory CD4⁺ T cells (Treg cells), follicular helper CD4⁺ T cells, and effector CD8⁺ T cells, as well as natural killer (NK) cells (Yu X, Harden K, Gonzalez L C, et al., The surface protein TIGIT suppresses T cell activation by promoting the generation of mature immunoregulatory dendritic cells [J]. Nature immunology, 2009, 10(1):48).

CD155 (also known as PVR, Necl5, or Tage4), CD112 (also known as PVRL2/nectin 2), and CD113 (also known as PVRL3) are ligands to which TIGIT binds (Martinet L, Smyth M J., Balancing natural killer cell activation through paired receptors [J]. Nature Reviews Immunology, 2015, 15(4):243-254). CD155 is a high-affinity ligand for TIGIT. In NK cells, TIGIT binding to the ligands CD155 and CD112 can inhibit the killing effect of NK cells on cells with high CD155 and CD112 expression (Stanietsky N, Simic H, Arapovic J, et al., The interaction of TIGIT with PVR and PVRL2 inhibits human NK cell cytotoxicity [J]. Proceedings of the National Academy of Sciences, 2009, 106(42):17858-17863). There is a report that the killing effect of CD8+ T cells can be enhanced when PD-1 and TIGIT are blocked simultaneously (Johnston R J, Comps-Agrar L, Hackney J, et al., The immunoreceptor TIGIT regulates anti-tumor and antiviral CD8+ T cell effector function [J]. Cancer cell, 2014, 26(6):923-937). Recent research reveals that TIGIT, as an immune checkpoint of NK cells, can cause NK cell exhaustion during tumor development and demonstrates that anti-TIGIT monoclonal antibodies can reverse NK cell exhaustion and be used for immunotherapy of a variety of tumors such as non-small cell lung cancer, small cell lung cancer, breast cancer, ovarian cancer, colorectal cancer, melanoma, pancreatic cancer, cervical tumor, multiple myeloma, non-Hodgkin's lymphoma, B-lymphoma, and plasma cell cancer (Zhang Q, Bi J, Zheng X, et al., Blockade of the checkpoint receptor TIGIT prevents NK cell exhaustion and elicits potent anti-tumor immunity [J]. Nature immunology, 2018, 19(7):723-732).

In addition, there is a report that TIGIT blockers used alone or in combination with PD-1 blockers plus CD96 blockers can significantly reduce the growth of B16 melanoma in wild-type and CD155-/- mouse models (Li X-Y, Das I, Lepletier A, et al., Cd155 loss enhances tumor suppression via combined host and tumor-intrinsic mechanisms. J Clin Invest, 2018, 128:2613-25). CD112R blockers used alone or in combination with TIGIT blockers and/or PD-1 blockers can increase the ability of TILs to produce cytokines in ovarian cancer, endometrial cancer, and lung tumors (Whelan S, Ophir E, Kotturi MF, et al., PVRIG and PVRL2 Are Induced in Cancer and Inhibit CD8+ T-cell function. Cancer Immunol Res, 2019, 7:257-68).

At present, there is still a need to develop a new fusion protein containing a TGF-β receptor, particularly a TGF-βRII extracellular region fragment; for example, the fusion protein further contains an anti-TIGIT antibody.

### SUMMARY

The inventors prepared a fusion protein comprising an anti-TIGIT antibody and a TGF-βR through intensive research and creative effort and surprisingly found that the fusion protein can effectively bind to TIGIT and TGF-β at the same time and even overcomes the defects of easy cleavage or degradation, demonstrating potential for preparing anti-tumor drugs. The present disclosure is detailed below:
One aspect of the present disclosure relates to a fusion protein comprising: a first protein functional region targeting an immune checkpoint, and a second protein functional region with TGF-β binding activity,
wherein:
   the second protein functional region is a variant of a TGF-βRII extracellular region fragment, and the variant of the TGF-βRII extracellular region fragment is:
   the first serine at the N-terminus of the TGF-βRII extracellular region fragment is replaced with alanine, glycine, or threonine, and/or a fragment comprising the amino acid sequence set forth in SEQ ID NO: 59 is deleted from the TGF-βRII extracellular region fragment.

In some embodiments of the present disclosure, the fusion protein is provided, wherein the amino acid sequence of the TGF-βRII extracellular region fragment is set forth in SEQ ID NO: 33 or SEQ ID NO: 37.

In some embodiments of the present disclosure, the fusion protein is provided, wherein the amino acid sequence of the deleted fragment comprising the amino acid sequence set forth in SEQ ID NO: 59 is set forth in any one of SEQ ID NO: 59 to SEQ ID NO: 65.

In some embodiments of the present disclosure, the fusion protein is provided, wherein:
the amino acid sequence of the variant of the TGF-βRII extracellular region fragment is set forth in any one of SEQ ID NO: 49 to SEQ ID NO: 58.

In some embodiments of the present disclosure, the fusion protein is provided, wherein the immune checkpoint is selected from one or more of PD-1, PD-L1, CTLA-4, LAG3, and TIGIT.

In some embodiments of the present disclosure, the fusion protein is provided, wherein the first protein functional region is an anti-PD-1 antibody or an antigen-binding fragment thereof.

In some embodiments of the present disclosure, the fusion protein is provided, wherein the first protein functional region is an anti-PD-L1 antibody or an antigen-binding fragment thereof.

In some embodiments of the present disclosure, the fusion protein is provided, wherein the first protein functional region is an anti-CTLA-4 antibody or an antigen-binding fragment thereof.

In some embodiments of the present disclosure, the fusion protein is provided, wherein the first protein functional region is an anti-LAG3 antibody or an antigen-binding fragment thereof.

In some embodiments of the present disclosure, the fusion protein is provided, wherein the first protein functional region is an anti-TIGIT antibody or an antigen-binding fragment thereof.

In some embodiments of the present disclosure, the fusion protein is provided, wherein:
the anti-TIGIT antibody comprises a heavy chain variable region comprising HCDR1 to HCDR3 and a light chain variable region comprising LCDR1 to LCDR3, wherein:
the amino acid sequence of HCDR1 is set forth in SEQ ID NO: 3, the amino acid sequence of HCDR2 is set forth in SEQ ID NO: 4, and the amino acid sequence of HCDR3 is set forth in SEQ ID NO: 5, and
the amino acid sequence of LCDR1 is set forth in SEQ ID NO: 8, the amino acid sequence of LCDR2 is set forth in SEQ ID NO: 9, and the amino acid sequence of LCDR3 is set forth in SEQ ID NO: 10.

In some embodiments of the present disclosure, the fusion protein is provided, wherein the amino acid sequence of the heavy chain variable region of the anti-TIGIT antibody is selected from SEQ ID NO: 1, SEQ ID NO: 11, SEQ ID NO: 13, SEQ ID NO: 15, and SEQ ID NO: 17; and
the amino acid sequence of the light chain variable region of the anti-TIGIT antibody is selected from SEQ ID NO: 6, SEQ ID NO: 19, SEQ ID NO: 21, SEQ ID NO: 23, and SEQ ID NO: 25.

In some embodiments of the present disclosure, the fusion protein is provided, wherein:
the amino acid sequence of the heavy chain variable region of the anti-TIGIT antibody is set forth in SEQ ID NO: 1, and the amino acid sequence of the light chain variable region is set forth in SEQ ID NO: 6;
the amino acid sequence of the heavy chain variable region of the anti-TIGIT antibody is set forth in SEQ ID NO: 11, and the amino acid sequence of the light chain variable region is set forth in SEQ ID NO: 19;
the amino acid sequence of the heavy chain variable region of the anti-TIGIT antibody is set forth in SEQ ID NO: 17, and the amino acid sequence of the light chain variable region is set forth in SEQ ID NO: 19;
the amino acid sequence of the heavy chain variable region of the anti-TIGIT antibody is set forth in SEQ ID NO: 13, and the amino acid sequence of the light chain variable region is set forth in SEQ ID NO: 21;
the amino acid sequence of the heavy chain variable region of the anti-TIGIT antibody is set forth in SEQ ID NO: 13, and the amino acid sequence of the light chain variable region is set forth in SEQ ID NO: 23;
the amino acid sequence of the heavy chain variable region of the anti-TIGIT antibody is set forth in SEQ ID NO: 15, and the amino acid sequence of the light chain variable region is set forth in SEQ ID NO: 21;
the amino acid sequence of the heavy chain variable region of the anti-TIGIT antibody is set forth in SEQ ID NO: 15, and the amino acid sequence of the light chain variable region is set forth in SEQ ID NO: 23;
the amino acid sequence of the heavy chain variable region of the anti-TIGIT antibody is set forth in SEQ ID NO: 11, and the amino acid sequence of the light chain variable region is set forth in SEQ ID NO: 25; or
the amino acid sequence of the heavy chain variable region of the anti-TIGIT antibody is set forth in SEQ ID NO: 17, and the amino acid sequence of the light chain variable region is set forth in SEQ ID NO: 25.

In some embodiments of the present disclosure, the fusion protein is provided, wherein the anti-TIGIT antibody or the antigen-binding fragment thereof is selected from Fab, Fab', F(ab')2, Fd, Fv, dAb, a complementarity determining region fragment, a single chain fragment variable, a humanized antibody, a chimeric antibody, and a diabody.

In some embodiments of the present disclosure, the fusion protein is provided, wherein:
the anti-TIGIT antibody comprises a non-CDR region, and the non-CDR region is derived from a non-murine species, e.g., from a human antibody.

In some embodiments of the present disclosure, the fusion protein is provided, wherein a heavy chain constant region of the anti-TIGIT antibody is an Ig gamma-1 chain C region or an Ig gamma-4 chain C region, and a light chain constant region is an Ig kappa chain C region.

In some embodiments of the present disclosure, the fusion protein is provided, wherein the fusion protein has a stronger ability to bind to ligand CD155-hFc-Biotin than control antibody RG6058(hG4).

In some embodiments of the present disclosure, the fusion protein is provided, wherein the fusion protein binds to TIGIT-mFc with an EC₅₀ of less than 0.08 nM or less than 0.10 nM; preferably, the EC₅₀ is measured by indirect ELISA.

In some embodiments of the present disclosure, the fusion protein is provided, wherein the fusion protein binds to TGF-β1, TGF-β2, and/or TGF-β3 with an EC₅₀ of less than 0.3 nM, less than 0.4 nM, less than 0.5 nM, or less than 0.6; preferably, the EC₅₀ is measured by indirect ELISA.

In some embodiments of the present disclosure, the fusion protein is provided, wherein the anti-TIGIT antibody is an antibody produced by hybridoma cell line LT019, and the hybridoma cell line LT019 is deposited at the China Center for Type Culture Collection (CCTCC) under CCTCC NO. C2020208.

In some embodiments of the present disclosure, the fusion protein is provided, wherein according to the EU numbering system,
when the heavy chain constant region of the anti-TIGIT antibody is IgG1, the heavy chain constant region has one of the following mutation combinations:
   L234A and L235A;
   L234A and G237A;
   L235A and G237A; or
   L234A, L235A, and G237A;
when the heavy chain constant region of the anti-TIGIT antibody is IgG4, the heavy chain constant region has one of the following mutation combinations:
   F234A and L235A;
   F234A and G237A;
   L235A and G237A; or
   F234A, L235A, and G237A.

In some embodiments of the present disclosure, the fusion protein is provided, wherein:
the amino acid sequence of a heavy chain of the anti-TIGIT antibody is set forth in SEQ ID NO: 27 or SEQ ID NO: 31, and the amino acid sequence of a light chain thereof is set forth in SEQ ID NO: 29.

In some embodiments of the present disclosure, the fusion protein is provided, wherein the first protein functional region and the second protein functional region are linked directly or by a linker fragment.

In some embodiments of the present disclosure, the fusion protein is provided, wherein the linker fragment is a polypeptide set forth in SEQ ID NO: 69 or SEQ ID NO: 70, or a polypeptide obtained by concatenating a plurality of (e.g., 2, 3, 4, 5, or 6) polypeptides set forth in SEQ ID NO: 69, or a polypeptide obtained by concatenating a plurality of (e.g., 2, 3, 4, or 5) polypeptides set forth in SEQ ID NO: 69 and further concatenating the concatenated polypeptides and a polypeptide set forth in SEQ ID NO: 70; preferably, the amino acid sequence of the linker fragment is set forth in SEQ ID NO: 44 or SEQ ID NO: 66.

In some embodiments of the present disclosure, the fusion protein is provided, wherein the first protein functional region and the second protein functional region are independently 1, 2, or more in number.

In some embodiments of the present disclosure, the fusion protein is provided, wherein the second protein functional region is linked to the C-terminus of the first protein functional region.

In some embodiments of the present disclosure, the fusion protein is provided, wherein the second protein functional region is linked to the C-terminus of the heavy chain of the anti-TIGIT antibody.

The present disclosure relates to a fusion protein comprising:
a first protein functional region targeting TIGIT, and
a second protein functional region with TGF-β binding activity,
wherein:
the first protein functional region is 1 in number, and the second protein functional region is 2 in number;
the first protein functional region is an anti-TIGIT antibody or an antigen-binding fragment thereof, and the second protein functional region is a variant of a TGF-βRII extracellular region fragment;
the amino acid sequence of a heavy chain of the anti-TIGIT antibody is set forth in SEQ ID NO: 27 or SEQ ID NO: 31, and the amino acid sequence of a light chain thereof is set forth in SEQ ID NO: 29;
the amino acid sequence of the variant of the TGF-βRII extracellular region fragment is set forth in any one of SEQ ID NO: 49 to SEQ ID NO: 58;
the second protein functional region is linked to the C-terminus of the heavy chain of the anti-TIGIT antibody by a linker fragment;
preferably, the amino acid sequence of the linker fragment is set forth in SEQ ID NO: 44 or SEQ ID NO: 66.

Without being bound by theory, some TGF-βR fusion proteins are unstable and have the problem of TGF-βR cleavage, resulting in incomplete fusion protein structures, which brings a certain difficulty to the process and quality control in the production process of the fusion proteins, thereby affecting the purity and quality uniformity of the fusion proteins and further causing problems in drug safety and effectiveness. In actual production and application, it is also necessary to develop a more stable antibody/TGF-βR fusion protein.

In some embodiments of the present disclosure, the fusion protein is provided, wherein compared to a fusion protein using a TGF-βRII extracellular region fragment (e.g., SEQ ID NO: 33 or SEQ ID NO: 37), the variant of the TGF-βRII extracellular region fragment undergoes less cleavage and/or degradation during recombinant expression.

In some embodiments of the present disclosure, the fusion protein is used for treating and/or preventing a tumor;
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

In a broad sense, the fusion protein of the present disclosure may also be referred to as an antibody.

Another aspect of the present disclosure relates to an isolated nucleic acid molecule encoding the fusion protein according to any embodiment of the present disclosure.

Yet another aspect of the present disclosure relates to a vector comprising the isolated nucleic acid molecule of the present disclosure.

Yet another aspect of the present disclosure relates to a host cell comprising the isolated nucleic acid molecule of the present disclosure or the vector of the present disclosure.

Yet another aspect of the present disclosure relates to a conjugate comprising a fusion protein moiety and a conjugated moiety, wherein the fusion protein moiety is the fusion protein according to any embodiment of the present disclosure, and the conjugated moiety is a detectable label;
preferably, the conjugated moiety is a radioisotope, a fluorescent substance, a luminescent substance, a colored substance, or an enzyme.

In some embodiments of the present disclosure, the conjugate is used for treating and/or preventing a tumor;
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

Yet another aspect of the present disclosure relates to use of the fusion protein of the present disclosure or the conjugate of the present disclosure in the preparation of a kit for detecting the presence or level of TIGIT and/or TGF-β in a sample.

Yet another aspect of the present disclosure relates to a pharmaceutical composition comprising the fusion protein according to any embodiment of the present disclosure or the conjugate of the present disclosure; optionally, the pharmaceutical composition further comprises one or more pharmaceutically acceptable auxiliary materials (e.g., carriers and/or excipients); optionally, the pharmaceutical composition further comprises one or more anti-tumor chemotherapeutic drugs.

In some embodiments of the present disclosure, the pharmaceutical composition is provided, wherein a unit dose of the pharmaceutical composition is 100 mg-1500 mg, 200 mg-1000 mg, 200 mg-800 mg, 300 mg-600 mg, 400 mg-500 mg, or 450 mg, as calculated based on the mass of the fusion protein therein.

In some embodiments of the present disclosure, the pharmaceutical composition is an injection.

In some embodiments of the present disclosure, the pharmaceutical composition is provided, wherein the fusion protein according to any embodiment of the present disclosure or the conjugate of the present disclosure is the only active ingredient. (Note: Sometimes the examiner will request the definition of the pharmaceutical composition. If it is defined as "comprising the fusion protein as the only active ingredient and an excipient", it is equivalent to a semi-closed definition [compared to "consisting of the fusion protein and an excipient"].)

In some embodiments of the present disclosure, the pharmaceutical composition consists of the fusion protein according to any embodiment of the present disclosure or the conjugate of the present disclosure and one or more pharmaceutically acceptable auxiliary materials.

Yet another aspect of the present disclosure relates to use of the fusion protein according to any embodiment of the present disclosure or the conjugate of the present disclosure in the preparation of a medicament for treating and/or preventing a tumor;
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

Yet another aspect of the present disclosure relates to a method for treating and/or preventing a tumor, comprising a step of administering to a subject in need thereof an effective amount of the fusion protein according to any embodiment of the present disclosure or the conjugate of the present disclosure;
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

In some embodiments of the present disclosure, the method is provided, wherein the step of administering to a subject in need thereof an effective amount of the fusion protein according to any embodiment of the present disclosure or the conjugate of the present disclosure is before or after surgical treatment, and/or before or after radiotherapy.

In some embodiments of the present disclosure, the method is provided, wherein:
a single-administration dose of the fusion protein of the present disclosure is 0.1-100 mg, preferably 1-10 mg, per kg body weight; alternatively, a single-administration dose of the fusion protein of the present disclosure is 10-1000 mg, preferably 50-500 mg, 100-400 mg, 150-300 mg, 150-250 mg, or 200 mg, per subject;
preferably, administration is performed once every 3 days, 4 days, 5 days, 6 days, 10 days, 1 week, 2 weeks, or 3 weeks;
preferably, a route of administration is intravenous drip infusion or intravenous injection.

It should be noted that the dose may vary depending on the type and severity of the symptom to be treated. Furthermore, those skilled in the art will appreciate that for any particular patient, the particular dosing regimen should be adjusted over time according to the needs of the patient and the professional judgment of the physician; the dose ranges provided herein are for illustrative purposes only and do not limit the use or scope of the fusion protein or pharmaceutical composition of the present disclosure.

In the present disclosure, the subject may be a mammal, such as a human.

Yet another aspect of the present disclosure relates to a variant of a TGF-βRII extracellular region fragment, and the variant is:
the first serine at the N-terminus of the TGF-βRII extracellular region fragment is replaced with alanine, glycine, or threonine; or,
a fragment comprising the amino acid sequence set forth in SEQ ID NO: 59 is deleted from the TGF-βRII extracellular region fragment;
preferably, the amino acid sequence of the TGF-βRII extracellular region fragment is set forth in SEQ ID NO: 33 or SEQ ID NO: 37.

In some embodiments of the present disclosure, the variant of the TGF-βRII extracellular region fragment is provided, wherein the amino acid sequence of the deleted fragment comprising the amino acid sequence set forth in SEQ ID NO: 59 is set forth in any one of SEQ ID NO: 59 to SEQ ID NO: 65.

In some embodiments of the present disclosure, the variant of the TGF-βRII extracellular region fragment is provided, wherein:
the amino acid sequence of the variant of the TGF-βRII extracellular region fragment is set forth in any one of SEQ ID NO: 49 to SEQ ID NO: 58.

The variable regions of the light and heavy chains determine antigen binding; the variable region of each chain contains three hypervariable regions called complementarity determining regions (CDRs), wherein the CDRs of the heavy chain (H) include HCDR1, HCDR2, and HCDR3, and the CDRs of the light chain (L) include LCDR1, LCDR2, and LCDR3. In the present disclosure, the CDRs are defined by the IMGT numbering system. See Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF [J]. Nucleic acids research, 2009; 38(suppl_1):D301-D307.

The amino acid sequences of the CDRs of the following monoclonal antibody are analyzed by technical means well known to those skilled in the art, for example, according to the IMGT definition, and the results are as follows:
The HCDRs and LCDRs of a murine TIGIT monoclonal antibody
HCDR1: GHSFTSDYA (SEQ ID NO: 3)
HCDR2: ISYSDST (SEQ ID NO: 4)
HCDR3: ARLDYGNYGGAMDY (SEQIDNO: 5)
LCDR1: QHVSTA (SEQIDNO: 8)
LCDR2: SAS (SEQIDNO: 9)
LCDR3: QQHYITPWT (SEQIDNO: 10)
The three HCDRs and three LCDRs of a humanized TIGIT monoclonal antibody are the same as those of the murine TIGIT monoclonal antibody.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196:901-917; Chothia et al., Nature, 1989; 342:878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF [J]. Nucleic acids research, 2009; 38(suppl_1):D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C., Continuous cultures of fused cells secreting antibody of predefined specificity [J]. Nature, 1975; 256(5517):495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) is replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522 525; Reichmann et al., Nature, 1988; 332:323329; Presta, Curr. Op. Struct. Biol., 1992; 2:593-596; and Clark, Immunol. Today, 2000; 21:397 402. In some cases, the antigen-binding fragments of the antibodies are diabodies, in which the V_{H} and V_{L} domains are expressed on a single polypeptide chain. However, the linker used is too short to allow the pairing of the two domains on the same chain. Thus, the domains are forced to pair with the complementary domains on the other chain, and two antigen-binding sites are generated (see, e.g., Holliger P. et al., Proc. Natl. Acad. Sci. USA, 1993; 90:6444-6448 and Poljak R.J. et al., Structure, 1994; 2:1121-1123).

The fusion protein described herein is a protein product of co-expression of two genes by DNA recombination. Methods for producing and purifying antibodies and antigen-binding fragments are well known in the art, for example, those described in chapters 5-8 and 15 of Antibodies: A Laboratory Manual, Cold Spring Harbor Press.

As used herein, the term "single chain fragment variable (ScFv)" refers to a molecule comprising an antibody heavy chain variable region (V_{H}) and an antibody light chain variable region (V_{L}) linked via a linker. The V_{L} and V_{H} domains are paired to form a monovalent molecule by a linker that enables them to produce a single polypeptide chain (see, e.g., Bird et al., Science, 1988; 242:423-426 and Huston et al., Proc. Natl. Acad. Sci. USA, 1988; 85:5879-5883). Such scFv molecules may have the following general structures: NH2-V_{L}-linker fragment-V_{H}-COOH or NH2-V_{H}-linker fragment-V_{L}-COOH. A suitable linker in the prior art consists of a repeated GGGGS (SEQ ID NO: 69) amino acid sequence or a variant thereof. For example, a linker having the amino acid sequence (GGGGS)4 (SEQ ID NO: 66) may be used, but variants thereof may also be used (Holliger et al., Proc. Natl. Acad. Sci. USA, 1993; 90: 6444-6448). Other linkers that can be used in the present disclosure are described in Alfthan et al., Protein Eng., 1995; 8: 725-731, Choi et al., Eur. J. Immunol., 2001; 31:94-106, Hu et al., Cancer Res., 1996; 56: 3055-3061, Kipriyanov et al., J. Mol. Biol., 1999; 293: 41-56 and Roovers et al., Cancer Immunology, Immunotherapy, 2001, 50(1): 51-59.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, GS cells, COS cells, NSO cells, HeLa cells, BHK cells, HEK293 cells, or human cells.

In the present disclosure, the term "ADCP" refers to antibody-dependent cellular phagocytosis. The Fc fragment of an antibody that binds to a cell surface antigen binds to the Fc receptor of a phagocytically active cell, such as a macrophage, which in turn mediates phagocytosis of the antibody-bound cells by phagocytic cells.

In the present disclosure, the term "ADCC" refers to antibody-dependent cell-mediated cytotoxicity. The Fab fragment of an antibody binds to an epitope of a virus-infected cell or a tumor cell, and the Fc fragment of the antibody binds to an Fc receptor (FcR) on the surface of a killer cell (NK cell, macrophage, etc.) to mediate direct killing of the target cells by killer cells.

In the present disclosure, the term "CDC" refers to complement-dependent cytotoxicity. When an antibody specifically binds to a corresponding antigen on a cell membrane surface, a complex is formed and the complement system is activated, and then an MAC is formed on the surface of a target cell, resulting in subsequent target cell lysis. Complements may cause cell lysis of various bacteria and other pathogenic organisms, and are an important defense mechanism against pathogenic organism infections.

As used herein, the term "specifically bind" refers to a non-random binding reaction between two molecules, such as a reaction between an antibody and an antigen it targets. In certain embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, an antibody binds to an antigen (e.g., TIGIT protein) with a dissociation equilibrium constant (K_{D}) of less than about 10⁻⁵ M, e.g., less than about 10⁻⁶ M, 10⁻⁷ M, 10⁻⁸ M, 10⁻⁹ M, or 10⁻¹⁰ M or less. K_{D} can be determined using methods known to those skilled in the art, e.g., using a ForteBio molecular interaction instrument.

As used herein, the terms "monoclonal antibody" and "mAb" have the same meaning and are used interchangeably; the terms "polyclonal antibody" and "pAb" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable auxiliary material" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to: pH regulators, surfactants, adjuvants and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

In the present disclosure, the terms "first" (e.g., first protein functional region, or first product) and "second" (e.g., second protein functional region, or second product) are used for distinguishing or clarity in expression and do not carry typical sequential meanings, unless otherwise specified.

### Beneficial Effects of the Present Disclosure

The present disclosure achieves any one or more of the following technical effects (1)-(8):
(1) The fusion protein of the present disclosure can simultaneously inhibit TIGIT and reduce TGF-β levels.
(2) The fusion protein of the present disclosure can very specifically bind to TIGIT, and can very effectively block the binding of TIGIT to CD155, and specifically remove the immunosuppression of the organism by TIGIT.
(3) The fusion protein of the present disclosure can very specifically bind to TGF-β, and can very effectively block the binding of TGF-β to a TGF-β receptor, and specifically remove the immunosuppression of the organism by TGF-β and activate immune responses.
(4) The fusion protein of the present disclosure has good potential for preparing anti-tumor drugs.
(5) The first protein functional region and the second protein functional region of the fusion protein of the present disclosure have a synergistic effect that is superior to the effect of using either of the protein functional regions alone, e.g., to the effect of using the anti-TIGIT antibody alone.
(6) The fusion protein of the present disclosure can effectively block TIGIT-induced immunosuppression of immune cells and induce cells to secrete IL-2.
(7) The fusion protein of the present disclosure can effectively block TGF-β-induced immunosuppression of immune cells and induce cells to secrete INF-γ.
(8) The fusion protein of the present disclosure overcomes the defects of easy cleavage or degradation.

### BRIEF DESCRIPTION OF THE DRAWINGS

FIG. 1: the binding activity of TF01, TF02, 26B12H2L2(hG4DM), and RG6058(hG4) to antigen TIGIT-mFc.
FIG. 2: the binding activity of TF01, TF02, and TGF-βRII-mFc to TGF-β1.
FIG. 3: the binding activity of TF01, TF02, and TGF-βRII-mFc to TGF-β2.
FIG. 4: the binding activity of TF01, TF02, and TGF-βRII-mFc to TGF-β3.
FIG. 5: the activity of anti-TIGIT antibody-TGF-βR fusion proteins in competing with TGF-βRII-His-Biotin for binding to human TGF-β1.
FIG. 6: the activity of anti-TIGIT antibody-TGF-βR fusion proteins in competing with TGF-βRII-His-Biotin for binding to human TGF-β3.
FIG. 7: the activity of anti-TIGIT antibody-TGF-βR fusion proteins in competing with human CD155-hFc-Biotin for binding to human TIGIT-mFC.
FIG. 8: the binding activity of an anti-TIGIT antibody-TGF-βR fusion protein to 293T-TIGIT membrane surface TIGIT.
FIG. 9: the competition between TF01 and CD155 for binding to cell membrane surface antigen TIGIT.
FIG. 10: the competition between TF01 and CD112 for binding to cell membrane surface antigen TIGIT.
FIG. 11: the affinity constant of TF01 for FcγRI.
FIG. 12: the affinity constant of 26B12H2L2(hG1WT) for FcyRI. FIG. 13: the affinity constant of TF01 for FcγRIIIa_V158.
FIG. 14: the affinity constant of 26B12H2L2(hG1WT) for FcγRIIIa_V158.
FIG. 15: the affinity constant of TF01 for FcγRIIIa_F158.
FIG. 16: the affinity constant of 26B12H2L2(hG1WT) for FeyRIIIa_F158.
FIG. 17: the affinity constant of TF01 for FcγRIIa_H131.
FIG. 18: the affinity constant of 26B12H2L2(hG1WT) for FcγRIIa_H131.
FIG. 19: the affinity constant of TF01 for FcγRIIa_R131.
FIG. 20: the affinity constant of 26B12H2L2(hG1WT) for FcγRIIa_R131.
FIG. 21: the affinity constant of TF01 for FcyRIIb.
FIG. 22: the affinity constant of 26B12H2L2(hG1WT) for FcγRIIb.
FIG. 23: the affinity constant of TF01 for C1q.
FIG. 24: the affinity constant of 26B12H2L2(hG1WT) for C1q.
FIG. 25: the ADCP effects of RG6058(hG1WT), RG6058(hG4WT), 26B12H2L2(hG1WT), and TF01.
FIG. 26: the blocking activity of anti-TIGIT antibody-TGF-βR fusion proteins against the inhibition of IL-2 secretion in a co-culture system of Jurkat-TIGIT and THP-1 cells by CD112.
FIG. 27: the blocking activity of an anti-TIGIT antibody-TGF-βR fusion protein against the inhibition of IL-2 secretion in a co-culture system of Jurkat-TIGIT and THP-1 cells by CD155.
FIG. 28: the IL-2 secretion promoting bioactivity of anti-TIGIT antibody-TGF-βR fusion proteins in a co-culture system of Jurkat-TIGIT and HT1080-aCD3scFv cells.
FIG. 29: the blocking activity of an anti-TIGIT antibody-TGF-βR fusion protein against the inhibition of IFN-y secretion by TGF-β1 during the secondary immune response of PBMCs to the CMV antigen.
FIG. 30: the efficacy of an anti-TIGIT antibody-TGF-βR fusion protein in a Scid Beige mouse MDA-MB-231 xenograft tumor model. The results are expressed as mean ± standard error, and a two-way analysis of variance was performed, followed by a Bonferroni test. Compared to the isotype control group: *p < 0.05, **p < 0.01, ***p < 0.001.
FIG. 31: the effect of an anti-TIGIT antibody-TGF-βR fusion protein on body weight in a Scid Beige mouse MDA-MB-231 xenograft tumor model.
FIG. 32: the IL-2 secretion promoting bioactivity of antibodies TF01A, TF01G, TF01T, TF01t31, and TF01t37 in a co-culture system of Jurkat-TIGIT and HT1080-aCD3scFv cells.
FIG. 33: the neutralizing effects of anti-TIGIT antibody-TGF-βR fusion proteins on the inhibition of IL-4-induced TF-1 proliferation by TGF-β1.
FIG. 34: the neutralizing effects of anti-TIGIT antibody-TGF-βR fusion proteins on the inhibition of IL-4-induced TF-1 proliferation by TGF-β3.
FIG. 35: the results of a reporter gene assay assessment of the blocking of the interaction between TGF-β1 and TGF-βR by anti-TIGIT antibody-TGF-βR fusion proteins.
FIG. 36: the results of a reporter gene assay assessment of the blocking of the interaction between TGF-β3 and TGF-βR by anti-TIGIT antibody-TGF-βR fusion proteins.
FIG. 37: the results of a reporter gene assay assessment of the blocking of the interaction between TIGIT and PVR by anti-TIGIT antibody-TGF-βR fusion proteins.
FIG. 38: the molecular weight of anti-TIGIT antibody-TGF-βR fusion protein TF01.
FIG. 39: the molecular weight of anti-TIGIT antibody-TGF-βR fusion protein TF01A.
FIG. 40: the molecular weight of anti-TIGIT antibody-TGF-βR fusion protein TF01G.
FIG. 41: the molecular weight of anti-TIGIT antibody-TGF-βR fusion protein TF01T.
FIG. 42: the molecular weight of anti-TIGIT antibody-TGF-βR fusion protein TF01t31.
FIG. 43: the molecular weight of anti-TIGIT antibody-TGF-βR fusion protein TF01t37.
FIG. 44: the results of an SEC-HPLC analysis of anti-TIGIT antibody-TGF-βR fusion protein TF01.
FIG. 45: the results of an SEC-HPLC analysis of anti-TIGIT antibody-TGF-βR fusion protein TF01T.
FIG. 46: the binding activity of anti-TIGIT antibody-TGF-βR fusion proteins to TGF-β1.
FIG. 47: the determination of the IFN-γ secretion promoting bioactivity of an anti-TIGIT antibody-TGF-βR fusion protein using a mixed lymphocyte reaction.

### Deposited biological material:

Hybridoma cell line LT019 (TIGIT-26B12) was deposited at the China Center for Type Culture Collection (CCTCC) on October 23, 2020. The CCTCC designation was CCTCC NO. C2020208, and the deposition address was Wuhan University, Wuhan, China, postal code: 430072.

Some sequences involved in the present disclosure are as follows:
1. The amino acid sequence of 26B12VH 
EVQLQESGPGLVKPSQSLSLTCTVTGHSFTSDYAWNWIRQFPGNR
LEWMGYISYSDSTNYNPSLKSRISITRDTSKNQFFLQMNSVTTEDT
ATYYCARLDYGNYGGAMDYWGQGTSVTVSS (SEQ ID NO: 1)
2. The nucleotide sequence of 26B12VH 
GAGGTGCAGCTGCAGGAGTCTGGACCTGGCCTGGTGAAACCC
TCTCAGTCTCTGTCCCTCACCTGCACTGTCACTGGCCACTCATT
CACCAGTGATTATGCCTGGAACTGGATCCGGCAGTTTCCAGGA
AACAGACTGGAGTGGATGGGCTACATAAGCTACAGTGATAGCA
CTAACTACAACCCATCTCTCAAAAGTCGAATCTCTATCACTCGA
GACACATCCAAGAACCAGTTCTTCTTGCAGATGAATTCTGTGA
CTACTGAGGACACAGCCACATATTACTGTGCAAGATTGGACTA
TGGTAACTACGGTGGGGCTATGGACTACTGGGGTCAAGGAC
CTCAGTCACCGTCTCCTCA (SEQ ID NO: 2)
3. HCDR1:
   GHSFTSDYA (SEQ ID NO: 3)
4. HCDR2:
   ISYSDST (SEQ ID NO: 4)
5. HCDR3:
   ARLDYGNYGGAMDY (SEQ ID NO: 5)
6. The amino acid sequence of 26B12VL 
DIVLTQSHEFMSTSLRDRVSITCKSSQHVSTAVAWYQQKPGQSPK
LLIYSASYRYTGVPDRFTGSGSGTDFTFTISSVKAEDLAVYYCQQH
YITPWTFGGGTKLEIK (SEQ ID NO: 6)
7. The nucleotide sequence of 26B12VL 
GATATTGTGCTAACTCAGTCTCACGAATTCATGTCCACCTCATT
ACGAGACAGGGTCAGCATCACCTGCAAATCCAGTCAACATGTG
AGTACTGCTGTAGCCTGGTATCAACAGAAACCAGGACAATCTC
CTAAACTACTGATTTACTCGGCATCCTACCGGTACACTGGAGT
CCCTGATCGCTTCACTGGCAGTGGATCTGGGACGGATTTCACT
TTCACCATCAGCAGTGTGAAGGCTGAAGACCTGGCAGTTTATT
ACTGTCAGCAACATTATATTACTCCGTGGACGTTCGGTGGAGG
CACCAAGCTGGAAATAAAA (SEQ ID NO: 7)
8. LCDR1:
   QHVSTA (SEQ ID NO: 8)
9. LCDR2:
   SAS (SEQ ID NO: 9)
10. LCDR3:
   QQHYITPWT (SEQ ID NO: 10)
11. The amino acid sequence of 26B12H1VH 
DVQLQESGPGLVKPSQTLSLTCTVSGHSFTSDYAWNWIRQFPGKG
LEWIGYISYSDSTNYNPSLKSRITISRDTSKNQFFLQLNSVTAADTA
TYYCARLDYGNYGGAMDYWGQGTSVTVSS (SEQ ID NO: 11)
12. The nucleotide sequence of 26B12H1VH 
GATGTGCAGCTGCAGGAGAGCGGCCCCGGACTGGTGAAGCCT
TCCCAGACCCTGTCTCTGACCTGTACAGTGTCTGGCCACAGCT
TCACATCCGACTACGCCTGGAACTGGATCAGGCAGTTTCCAGG
CAAGGGCCTGGAGTGGATCGGCTACATCTCTTATAGCGACTCC
ACCAACTATAATCCCTCTCTGAAGAGCCGGATCACCATCAGCA
GAGATACATCCAAGAACCAGTTCTTTCTGCAGCTGAACAGCGT
GACAGCCGCCGACACCGCCACATACTATTGCGCCCGGCTGGA
CTACGGCAATTATGGCGGAGCCATGGATTACTGGGGCCAGGG
CACCTCCGTGACAGTGAGCTCC (SEQ ID NO: 12)
13. The amino acid sequence of 26B12H2VH 
DVQLQESGPGLVKPSQTLSLTCTVSGHSFTSDYAWSWIRQPPGKG
LEWIGYISYSDSTNYNPSLKSRVTISRDTSKNQFSLKLSSVTAADTA
VYYCARLDYGNYGGAMDYWGQGTSVTVSS (SEQ ID NO: 13)
14. The nucleotide sequence of 26B12H2VH 
GATGTGCAGCTGCAGGAGTCTGGCCCAGGACTGGTGAAGCCA
AGCCAGACCCTGTCCCTGACCTGTACAGTGTCCGGCCACTCTT
TTACAAGCGACTACGCCTGGTCTTGGATCAGGCAGCCCCCTGG
 
CAAGGGACTGGAGTGGATCGGCTACATCTCCTATTCTGACAGC
ACCAACTATAATCCCTCCCTGAAGTCTCGGGTGACCATCTCTA
GAGATACAAGCAAGAACCAGTTCTCCCTGAAGCTGAGCTCCGT
GACCGCAGCAGACACAGCCGTGTACTATTGCGCCCGGCTGGA
CTACGGCAATTATGGCGGAGCCATGGATTACTGGGGCCAGGG
CACCAGCGTGACAGTGTCTAGC (SEQ ID NO: 14)
15. The amino acid sequence of 26B12H3VH 
DVQLQESGPGLVKPSQTLSLTCTVSGHSFTSDYAWSWIRQPPGKG
LEWIGYISYSDSTNYNPSLKSRVTISVDTSKNQFSLKLSSVTAADTA
VYYCARLDYGNYGGAMDYWGQGTSVTVSS (SEQ ID NO: 15)
16. The nucleotide sequence of 26B12H3VH 
GATGTGCAGCTGCAGGAGTCTGGCCCAGGACTGGTGAAGCCA
AGCCAGACCCTGTCCCTGACCTGTACAGTGTCCGGCCACTCTT
TTACAAGCGACTACGCCTGGTCTTGGATCAGACAGCCCCCTGG
CAAGGGACTGGAGTGGATCGGCTACATCTCCTATTCTGACAGC
ACCAACTATAATCCCTCCCTGAAGTCTAGAGTGACCATCTCTG
TGGATACAAGCAAGAACCAGTTCTCCCTGAAGCTGAGCTCCGT
GACCGCAGCAGACACAGCCGTGTACTATTGCGCCCGGCTGGA
CTACGGCAATTATGGCGGAGCCATGGATTACTGGGGCCAGGG
CACCAGCGTGACAGTGTCTAGC (SEQ ID NO: 16)
17. The amino acid sequence of 26B12H4VH 
DVQLQESGPGLVKPSQTLSLTCTVSGHSFTSDYAWNWIRQFPGKG
LEWMGYISYSDSTNYNPSLKSRITISRDTSKNQFFLQLNSVTAADT
ATYYCARLDYGNYGGAMDYWGQGTSVTVSS (SEQ ID NO: 17)
18. The nucleotide sequence of 26B12H4VH 
GATGTGCAGCTGCAGGAGAGCGGCCCCGGACTGGTGAAGCCT
TCCCAGACCCTGTCTCTGACCTGTACAGTGTCTGGCCACAGCT
TCACATCCGACTACGCCTGGAACTGGATCAGGCAGTTTCCAGG
CAAGGGCCTGGAGTGGATGGGCTACATCTCTTATAGCGACTCC
ACCAACTATAATCCCTCTCTGAAGAGCCGGATCACCATCAGCA
GAGATACATCCAAGAACCAGTTCTTTCTGCAGCTGAACAGCGT
GACAGCCGCCGACACCGCCACATACTATTGCGCCCGGCTGGA
CTACGGCAATTATGGCGGAGCCATGGATTACTGGGGCCAGGG
CACCTCCGTGACAGTGAGCTCC (SEQ ID NO: 18)
19. The amino acid sequence of 26B12L1VL 
DIQMTQSPKSLSTSVGDRVTITCRSSQHVSTAVAWYQQKPGKSPK
LLIYSASYRYSGVPDRFSGSGSGTDFTFTISSVQPEDFATYYCQQHY
ITPWTFGGGTKLEIK (SEQ ID NO: 19)
20. The nucleotide sequence of 26B12L1VL 
GACATCCAGATGACCCAGTCCCCTAAGTCCCTGTCTACAAGCG
TGGGCGATCGGGTGACCATCACATGTAGAAGCTCCCAGCACGT
GTCTACCGCAGTGGCATGGTACCAGCAGAAGCCAGGCAAGAG
CCCTAAGCTGCTGATCTATTCCGCCTCTTACAGGTATTCCGGA
GTGCCAGACCGGTTTAGCGGCTCCGGCTCTGGCACCGATTTCA
CCTTTACAATCTCTAGCGTGCAGCCAGAGGACTTCGCCACATA
CTATTGCCAGCAGCACTACATCACCCCATGGACCTTCGGCGGC
GGCACAAAGCTGGAGATCAAG (SEQ ID NO: 20)
21. The amino acid sequence of 26B12L2VL 
DIQMTQSPSSLSASVGDRVTITCRSSQHVSTALAWYQQKPGKSPK
LLIYSASSRYSGVPDRFSGSGSGTDFTFTISSLQPEDFATYYCQQHY
ITPWTFGGGTKLEIK (SEQ ID NO: 21)
22. The nucleotide sequence of 26B12L2VL 
GACATCCAGATGACCCAGTCCCCTAGCTCCCTGTCTGCCAGCG
TGGGCGATAGGGTGACCATCACATGTAGATCTAGCCAGCACGT
GTCTACAGCCCTGGCATGGTACCAGCAGAAGCCAGGCAAGAG
CCCTAAGCTGCTGATCTACTCCGCCTCCTCTAGGTATTCTGGA
GTGCCAGACCGGTTTTCCGGCTCTGGCAGCGGCACCGATTTCA
CCTTTACAATCAGCTCCCTGCAGCCAGAGGACTTCGCCACATA
CTATTGCCAGCAGCACTATATCACCCCATGGACCTTCGGCGGC
GGCACCAAGCTGGAGATCAAG (SEQ ID NO: 22)
23. The amino acid sequence of 26B12L3VL 
DIQMTQSPSSLSASVGDRVTITCRASQHVSTALAWYQQKPGKAPK
LLIYSASSLQSGVPSRFSGSGSGTDFTLTISSLQPEDFATYYCQQHY
ITPWTFGGGTKLEIK (SEQ ID NO: 23)
24. The nucleotide sequence of 26B12L3VL 
GACATCCAGATGACCCAGTCCCCTAGCTCCCTGAGCGCCTCCG
TGGGCGATAGGGTGACCATCACATGTAGAGCCTCTCAGCACGT
GAGCACAGCCCTGGCATGGTACCAGCAGAAGCCAGGCAAGGC
CCCTAAGCTGCTGATCTATAGCGCCTCTAGCCTGCAGTCCGGA
GTGCCATCTCGGTTCTCTGGCAGCGGCTCCGGAACCGACTTTA
CCCTGACAATCTCCTCTCTGCAGCCAGAGGATTTCGCCACATA
CTATTGCCAGCAGCACTACATCACCCCCATGGACCTTCGGCGGC
GGCACCAAGCTGGAGATCAAG (SEQ ID NO: 24)
25. The amino acid sequence of 26B12L4VL 
DIQMTQSPKSMSTSVGDRVTITCRSSQHVSTAVAWYQQKPGKSPK
LLIYSASYRYSGVPDRFSGSGSGTDFTFTISSVQPEDFATYYCQQHY
 ITPWTFGGGTKLEIK (SEQ ID NO: 25)
26. The nucleotide sequence of 26B12L4VL 
GACATCCAGATGACCCAGTCCCCTAAGTCCATGTCTACAAGCG
TGGGCGACAGGGTGACCATCACATGTAGAAGCTCCCAGCACG
TGTCTACCGCAGTGGCATGGTACCAGCAGAAGCCAGGCAAGA
GCCCTAAGCTGCTGATCTATTCCGCCTCTTACAGGTATTCCGG
AGTGCCAGACCGGTTTAGCGGCTCCGGCTCTGGCACCGATTTC
ACCTTTACAATCTCTAGCGTGCAGCCAGAGGACTTCGCCACAT
ACTATTGCCAGCAGCACTACATCACCCCATGGACCTTCGGCGG
CGGCACAAAGCTGGAGATCAAG (SEQ ID NO: 26)
27. The amino acid sequence of the heavy chain of 26B12H2L2(hG4DM) 
DVQLQESGPGLVKPSQTLSLTCTVSGHSFTSDYAWSWIRQPPGKG
LEWIGYISYSDSTNYNPSLKSRVTISRDTSKNQFSLKLSSVTAADTA
VYYCARLDYGNYGGAMDYWGQGTSVTVSSASTKGPSVFPLAPCS
RSTSESTAALGCLVKDYFPEPVTVSWNSGALTSGVHTFPAVLQSS
GLYSLSSVVTVPSSSLGTKTYTCNVDHKPSNTKVDKRVESKYGPP
CPPCPAPEAAGGPSVFLFPPKPKDTLMISRTPEVTCVVVDVSQEDP
EVQFNWYVDGVEVHNAKTKPREEQFNSTYRVVSVLTVLHQDWL
NGKEYKCKVSNKGLPSSIEKTISKAKGQPREPQVYTLPPSQEEMT
KNQVSLTCLVKGFYPSDIAVEWESNGQPENNYKTTPPVLDSDGSF
FLYSRLTVDKSRWQEGNVFSCSVMHEALHNHYTQKSLSLSLG
(SEQ ID NO: 27)
28. The nucleotide sequence of the heavy chain of 26B12H2L2(hG4DM) 
GATGTGCAGCTGCAGGAGTCTGGCCCAGGACTGGTGAAGCCA
AGCCAGACCCTGTCCCTGACCTGTACAGTGTCCGGCCACTCTT
TTACAAGCGACTACGCCTGGTCTTGGATCAGGCAGCCCCCTGG
 
CAAGGGACTGGAGTGGATCGGCTACATCTCCTATTCTGACAGC
ACCAACTATAATCCCTCCCTGAAGTCTCGGGTGACCATCTCTA
GAGATACAAGCAAGAACCAGTTCTCCCTGAAGCTGAGCTCCGT
GACCGCAGCAGACACAGCCGTGTACTATTGCGCCCGGCTGGA
CTACGGCAATTATGGCGGAGCCATGGATTACTGGGGCCAGGG
CACCAGCGTGACAGTGTCTAGCGCCTCCACAAAGGGCCCTC
GGTCTTCCCCCTGGCGCCCTGCTCCAGGAGCACCTCCGAGAGC
ACAGCCGCCCTGGGCTGCCTGGTCAAGGACTACTTCCCCGAAC
CGGTGACGGTGTCGTGGAACTCAGGCGCCCTGACCAGCGGCG
TACACACCTTCCCGGCTGTCCTACAGTCCTCAGGACTCTACTC
CCTCAGCAGCGTGGTGACCGTGCCCTCCAGCAGCTTGGGCAC
GAAGACCTACACCTGCAACGTAGATCACAAGCCCAGCAACACC
AAGGTGGACAAGAGAGTTGAGTCCAAATATGGTCCCCCATGCC
CACCATGCCCAGCACCTGAGGCCGCTGGGGGACCATCAGTCTT
CCTGTTCCCCCCAAAACCCAAGGACACTCTCATGATCTCCCGG
ACCCCTGAGGTCACGTGCGTGGTGGTGGACGTGAGCCAGGAA
GACCCCGAGGTCCAGTTCAACTGGTACGTGGATGGCGTGGAG
GTGCATAATGCCAAGACAAAGCCGCGGGAGGAGCAGTTCAAC
AGCACGTACCGTGTGGTCAGCGTCCTCACCGTCCTGCACCAGG
ACTGGCTGAACGGCAAGGAGTACAAGTGCAAGGTCTCCAACA
AAGGCCTCCCGTCCTCCATCGAGAAAACCATCTCCAAAGCCAA
AGGGCAGCCCCGAGAGCCACAGGTGTACACCCTGCCCCCATC
CCAGGAGGAGATGACCAAGAACCAGGTCAGCCTGACCTGCCT
GGTCAAAGGCTTCTACCCCAGCGACATCGCCGTGGAGTGGGA
GAGCAATGGGCAGCCGGAGAACAACTACAAGACCACGCCTCC
CGTGCTGGACTCCGACGGCTCCTTCTTCCTCTACAGCAGGCTA
ACCGTGGACAAGAGCAGGTGGCAGGAGGGGAATGTCTTCTCA
TGCTCCGTGATGCATGAGGCTCTGCACAACCACTACACACAGA
AGAGCCTCTCCCTGTCTCTGGGT (SEQ ID NO: 28)
29. The amino acid sequence of the light chain of 26B12H2L2(hG4DM) 
DIQMTQSPSSLSASVGDRVTITCRSSQHVSTALAWYQQKPGKSPK
LLIYSASSRYSGVPDRFSGSGSGTDFTFTISSLQPEDFATYYCQQHY
ITPWTFGGGTKLEIKRTVAAPSVFIFPPSDEQLKSGTASVVCLLNN
FYPREAKVQWKVDNALQSGNSQESVTEQDSKDSTYSLSSTLTLSK
ADYEKHKVYACEVTHQGLSSPVTKSFNRGEC (SEQ ID NO: 29)
30. The nucleotide sequence of the light chain of 26B12H2L2(hG4DM) 
GACATCCAGATGACCCAGTCCCCTAGCTCCCTGTCTGCCAGCG
TGGGCGATAGGGTGACCATCACATGTAGATCTAGCCAGCACGT
GTCTACAGCCCTGGCATGGTACCAGCAGAAGCCAGGCAAGAG
CCCTAAGCTGCTGATCTACTCCGCCTCCTCTAGGTATTCTGGA
GTGCCAGACCGGTTTTCCGGCTCTGGCAGCGGCACCGATTTCA
CCTTTACAATCAGCTCCCTGCAGCCAGAGGACTTCGCCACATA
CTATTGCCAGCAGCACTATATCACCCCATGGACCTTCGGCGGC
GGCACCAAGCTGGAGATCAAGCGTACGGTGGCAGCCCCATCT
GTCTTCATTTTCCCCCTAGTGACGAGCAGCTGAAATCCGGAA
CAGCCTCTGTGGTCTGTCTGCTGAACAATTTCTACCCTCGCGA
AGCCAAGGTGCAGTGGAAAGTCGATAACGCTCTGCAGAGTGG
CAATTCACAGGAGAGCGTGACTGAACAGGACTCCAAGGATTCT
ACCTATAGTCTGAGCTCCACTCTGACCCTGTCCAAAGCAGATT
ACGAAAAGCACAAAGTGTATGCCTGTGAGGTCACCCACCAGG
GGCTGAGTTCTCCAGTCACCAAATCCTTCAACAGAGGCGAATG
T(SEQ ID NO: 30)
31. The amino acid sequence of the heavy chain of 26B12H2L2(hG1DM) 
DVQLQESGPGLVKPSQTLSLTCTVSGHSFTSDYAWSWIRQPPGKG
LEWIGYISYSDSTNYNPSLKSRVTISRDTSKNQFSLKLSSVTAADTA
 
VYYCARLDYGNYGGAMDYWGQGTSVTVSSASTKGPSVFPLAPSS
KSTSGGTAALGCLVKDYFPEPVTVSWNSGALTSGVHTFPAVLQSS
GLYSLSSVVTVPSSSLGTQTYICNVNHKPSNTKVDKKVEPKSCDKT
HTCPPCPAPEAAGGPSVFLFPPKPKDTLMISRTPEVTCVVVDVSHE
DPEVKFNWYVDGVEVHNAKTKPREEQYNSTYRVVSVLTVLHQD
WLNGKEYKCKVSNKALPAPIEKTISKAKGQPREPQVYTLPPSRDE
LTKNQVSLTCLVKGFYPSDIAVEWESNGQPENNYKTTPPVLDSDG
SFFLYSKLTVDKSRWQQGNVFSCSVMHEALHNHYTQKSLSLSPG
K (SEQ ID NO: 31)
32. The nucleotide sequence of the heavy chain of 26B12H2L2(hG1DM) 
GATGTGCAGCTGCAGGAGTCTGGCCCAGGACTGGTGAAGCCA
AGCCAGACCCTGTCCCTGACCTGTACAGTGTCCGGCCACTCTT
TTACAAGCGACTACGCCTGGTCTTGGATCAGGCAGCCCCCTGG
CAAGGGACTGGAGTGGATCGGCTACATCTCCTATTCTGACAGC
ACCAACTATAATCCCTCCCTGAAGTCTCGGGTGACCATCTCTA
GAGATACAAGCAAGAACCAGTTCTCCCTGAAGCTGAGCTCCGT
GACCGCAGCAGACACAGCCGTGTACTATTGCGCCCGGCTGGA
CTACGGCAATTATGGCGGAGCCATGGATTACTGGGGCCAGGG
CACCAGCGTGACAGTGTCTAGCGCCTCTACCAAGGGGCCCAG
CGTGTTTCCTCTCGCCCCCTCCTCCAAAAGCACCAGCGGAGGA
ACCGCTGCTCTCGGATGTCTGGTGAAGGACTACTTCCCTGAAC
CCGTCACCGTGAGCTGGAATAGCGGCGCTCTGACAAGCGGAG
TCCATACATTCCCTGCTGTGCTGCAAAGCAGCGGACTCTATTC
CCTGTCCAGCGTCGTCACAGTGCCCAGCAGCAGCCTGGGCAC
CCAGACCTACATCTGTAACGTCAACCACAAGCCCTCCAACACC
AAGGTGGACAAGAAAGTGGAGCCCAAATCCTGCGACAAGACA
CACACCTGTCCCCCCTGTCCTGCTCCCGAAGCTGCTGGAGGCC
CTAGCGTCTTCCTCTTTCCTCCCAAACCCAAGGACACCCTCAT
 
GATCAGCAGAACCCCTGAAGTCACCTGTGTCGTCGTGGATGTC
AGCCATGAGGACCCCGAGGTGAAATTCAACTGGTATGTCGATG
GCGTCGAGGTGCACAACGCCAAAACCAAGCCCAGGGAGGAAC
AGTACAACTCCACCTACAGGGTGGTGTCCGTGCTGACAGTCCT
CCACCAGGACTGGCTGAACGGCAAGGAGTACAAGTGCAAGGT
GTCCAACAAGGCTCTCCCTGCCCCCATTGAGAAGACCATCAGC
AAGGCCAAAGGCCAACCCAGGGAGCCCCAGGTCTATACACTG
CCTCCCTCCAGGGACGAACTCACCAAGAACCAGGTGTCCCTGA
CCTGCCTGGTCAAGGGCTTTTATCCCAGCGACATCGCCGTCGA
GTGGGAGTCCAACGGACAGCCCGAGAATAACTACAAGACCAC
CCCTCCTGTCCTCGACTCCGACGGCTCCTTCTTCCTGTACAGC
AAGCTGACCGTGGACAAAAGCAGGTGGCAGCAGGGAAACGTG
TTCTCCTGCAGCGTGATGCACGAAGCCCTCCACAACCACTACA
CCCAGAAAAGCCTGTCCCTGAGCCCCGGCAAA (SEQ ID NO: 32)
33. The amino acid sequence of a TGF-βRII extracellular region fragment (truncated): 
IPPHVQKSVNNDMIVTDNNGAVKFPQLCKFCDVRFSTCDNQKSC
MSNCSITSICEKPQEVCVAVWRKNDENITLETVCHDPKLPYHDFIL
EDAASPKCIMKEKKKPGETFFMCSCSSDECNDNIIFSEEYNTSNPD
(SEQ ID NO: 33)
34. The amino acid sequence of TGF-βRI: (the sequence of the extracellular region is underlined) 





 






 (SEQ ID NO: 34)
35. The amino acid sequence of the TGF-βRI extracellular region: 
LQCFCHLCTKDNFTCVTDGLCFVSVTETTDKVIHNSMCIAEIDLIP
RDRPFVCAPSSKTGSVTTTYCCNQDHCNKIELPTTVKSSPGLGPVE
L (SEQ ID NO: 35)
36. The amino acid sequence of TGF-βRII: (the sequence of the extracellular region is underlined) 












 (SEQ ID NO: 36)
37. The amino acid sequence of the TGF-βRII extracellular region: 
TIPPHVQKSVNNDMIVTDNNGAVKFPQLCKFCDVRFSTCDNQKSC
MSNCSITSICEKPQEVCVAVWRKNDENITLETVCHDPKLPYHDFIL
EDAASPKCIMKEKKKPGETFFMCSCSSDECNDNIIFSEEYNTSNPD
LLLVIFQ (SEQ ID NO: 37)
38. The amino acid sequence of TGF-βRIII: (the sequence of the extracellular region is underlined) 
MTSHYVIAIFALMSSCLATAGPEPGALCELSPVSASHPVQALMESF
TVLSGCASRGTTGLPQEVHVLNLRTAGQGPGQLQREVTLHLNPIS
SVHIHHKSVVFLLNSPHPLVWHLKTERLATGVSRLFLVSEGSVVQ
FSSANFSLTAETEERNFPHGNEHLLNWARKEYGAVTSFTELKIAR
NIYIKVGEDQVFPPKCNIGKNFLSLNYLAEYLQPKAAEGCVMSSQ
PQNEEVHIIELITPNSNPYSAFQVDITIDIRPSQEDLEVVKNLILILKC
KKSVNWVIKSFDVKGSLKIIAPNSIGFGKESERSMTMTKSIRDDIPS
TQGNLVKWALDNGYSPITSYTMAPVANRFHLRLENNAEEMGDEE
VHTIPPELRILLDPGALPALQNPPIRGGEGQNGGLPFPFPDISRRV
WNEEGEDGLPRPKDPVIPSIQLFPGLREPEEVQGSVDIALSVKCDN
EKMIVAVEKDSFQASGYSGMDVTLLDPTCKAKMNGTHFVLESPL
NGCGTRPRWSALDGVVYYNSIVIQVPALGDSSGWPDGYEDLESGD
NGFPGDMDEGDASLFTRPEIVVFNCSLQQVRNPSSFQEQPHGNITF
NMELYNTDLFLVPSQGVFSVPENGHVYVEVSVTKAEQELGFAIQT
CFISPYSNPDRMSHYTIIENICPKDESVKFYSPKRVHFPIPQADMDK
KRFSFVFKPVFNTSLLFLQCELTLCTKMEKHPQKLPKCVPPDEAC
TSLDASIIWAMMQNKKTFTKPLAVIHHEAESKEKGPSMKEPNPIS
PPIFHGLDTLTVMGIAFAAFVIGALLTGALWYIYSHTGETAGRQQ
VPTSPPASENSSAAHSIGSTQSTPCSSSSTA (SEQ ID NO: 38)
39. The amino acid sequence of the TGF-βRIII extracellular region: 
GPEPGALCELSPVSASHPVQALMESFTVLSGCASRGTTGLPQEVH
VLNLRTAGQGPGQLQREVTLHLNPISSVHIHHKSVVFLLNSPHPL
VWHLKTERLATGVSRLFLVSEGSVVQFSSANFSLTAETEERNFPH
GNEHLLNWARKEYGAVTSFTELKIARNIYIKVGEDQVFPPKCNIG
KNFLSLNYLAEYLQPKAAEGCVMSSQPQNEEVHIIELITPNSNPYS
AFQVDITIDIRPSQEDLEVVKNLILILKCKKSVNWVIKSFDVKGSLK
IIAPNSIGFGKESERSMTMTKSIRDDIPSTQGNLVKWALDNGYSPIT
SYTMAPVANRFHLRLENNAEEMGDEEVHTIPPELRILLDPGALPA
LQNPPIRGGEGQNGGLPFPFPDISRRVWNEEGEDGLPRPKDPVIPS
IQLFPGLREPEEVQGSVDIALSVKCDNEKMIVAVEKDSFQASGYSG
MDVTLLDPTCKAKMNGTHFVLESPLNGCGTRPRWSALDGVVYY
NSIVIQVPALGDSSGWPDGYEDLESGDNGFPGDMDEGDASLFTRP
EIVVFNCSLQQVRNPSSFQEQPHGNITFNMELYNTDLFLVPSQGVF
SVPENGHVYVEVSVTKAEQELGFAIQTCFISPYSNPDRMSHYTIIEN
ICPKDESVKFYSPKRVHFPIPQADMDKKRFSFVFKPVFNTSLLFLQ
CELTLCTKMEKHPQKLPKCVPPDEACTSLDASIIWAMMQNKKTF
TKPLAVIHHEAESKEKGPSMKEPNPISPPIFHGLDTLTV (SEQ ID
NO: 39)
40. The amino acid sequence of the heavy chain of tiragolumab: 
EVQLQQSGPGLVKPSQTLSLTCAISGDSVSSNSAAWNWIRQSPSRG
LEWLGKTYYRFKWYSDYAVSVKGRITINPDTSKNQFSLQLNSVTP
EDTAVFYCTRESTTYDLLAGPFDYWGQGTLVTVSSASTKGPSVFP
LAPSSKSTSGGTAALGCLVKDYFPEPVTVSWNSGALTSGVHTFPA
VLQSSGLYSLSSVVTVPSSSLGTQTYICNVNHKPSNTKVDKKVEPK
SCDKTHTCPPCPAPELLGGPSVFLFPPKPKDTLMISRTPEVTCVVV
DVSHEDPEVKFNWYVDGVEVHNAKTKPREEQYNSTYRVVSVLTV
LHQDWLNGKEYKCKVSNKALPAPIEKTISKAKGQPREPQVYTLPP
SREEMTKNQVSLTCLVKGFYPSDIAVEWESNGQPENNYKTTPPVL
 
DSDGSFFLYSKLTVDKSRWQQGNVFSCSVMHEALHNHYTQKSLS
LSPGK (SEQ ID NO: 40)
41. The amino acid sequence of the light chain of tiragolumab: 
DIVMTQSPDSLAVSLGERATINCKSSQTVLYSSNNKKYLAWYQQK
PGQPPNLLIYWASTRESGVPDRFSGSGSGTDFTLTISSLQAEDVAV
YYCQQYYSTPFTFGPGTKVEIKRTVAAPSVFIFPPSDEQLKSGTAS
VVCLLNNFYPREAKVQWKVDNALQSGNSQESVTEQDSKDSTYSLS
STLTLSKADYEKHKVYACEVTHQGLSSPVTKSFNRGEC (SEQ ID
NO: 41)
42. The amino acid sequence of the heavy chain variable region of RG6058(hG4): 
EVQLQQSGPGLVKPSQTLSLTCAISGDSVSSNSAAWNWIRQSPSRG
LEWLGKTYYRFKWYSDYAVSVKGRITINPDTSKNQFSLQLNSVTP
EDTAVFYCTRESTTYDLLAGPFDYWGQGTLVTVSS (SEQ ID NO:
42)
43. The amino acid sequence of the light chain variable region of RG6058(hG4): 
DIVMTQSPDSLAVSLGERATINCKSSQTVLYSSNNKKYLAWYQQK
PGQPPNLLIYWASTRESGVPDRFSGSGSGTDFTLTISSLQAEDVAV
YYCQQYYSTPFTFGPGTKVEIK (SEQ ID NO: 43)
44. The amino acid sequence of a linker fragment:
   GGGGSGGGGSGGGGSGGGGSG (SEQ ID NO: 44)
45. The amino acid sequence of the heavy chain of an anti-HEL&TGFβ antibody 
EVQLEQSGAELMKPGASVKISCKATGYTFTTYWIEWIKQRPGHSL
EWIGEILPGSDSTYYNEKVKGKVTFTADASSNTAYMQLSSLTSEDS
AVYYCARGDGFYVYWGQGTTLTVSSASTKGPSVFPLAPCSRSTSE
STAALGCLVKDYFPEPVTVSWNSGALTSGVHTFPAVLQSSGLYSL
SSVVTVPSSSLGTKTYTCNVDHKPSNTKVDKRVESKYGPPCPPCPA
PEAAGGPSVFLFPPKPKDTLMISRTPEVTCVVVDVSQEDPEVQFN
WYVDGVEVHNAKTKPREEQFNSTYRVVSVLTVLHQDWLNGKEY
KCKVSNKGLPSSIEKTISKAKGQPREPQVYTLPPSQEEMTKNQVS
LTCLVKGFYPSDIAVEWESNGQPENNYKTTPPVLDSDGSFFLYSRL
TVDKSRWQEGNVFSCSVMHEALHNHYTQKSLSLSLGGGGGSGG
GGSGGGGSGGGGSGIPPHVQKSVNNDMIVTDNNGAVKFPQLCKF
CDVRFSTCDNQKSCMSNCSITSICEKPQEVCVAVWRKNDENITLE
TVCHDPKLPYHDFILEDAASPKCIMKEKKKPGETFFMCSCSSDEC
NDNIIFSEEYNTSNPD (SEQ ID NO: 45)
46. The amino acid sequence of the light chain of an anti-HEL&TGFβ antibody 
DIELTQSPATLSVTPGDSVSLSCRASQSISNNLHWYQQKSHESPRL
LIKYTSQSMSGIPSRFSGSGSGTDFTLSINSVETEDFGVYFCQQSGS
WPRTFGGGTKLDIKRTVAAPSVFIFPPSDEQLKSGTASVVCLLNNF
YPREAKVQWKVDNALQSGNSQESVTEQDSKDSTYSLSSTLTLSKA
DYEKHKVYACEVTHQGLSSPVTKSFNRGEC (SEQ ID NO: 46)
47. The amino acid sequence of the heavy chain of RG6058(G1DM) 
EVQLQQSGPGLVKPSQTLSLTCAISGDSVSSNSAAWNWIRQSPSRG
LEWLGKTYYRFKWYSDYAVSVKGRITINPDTSKNQFSLQLNSVTP
EDTAVFYCTRESTTYDLLAGPFDYWGQGTLVTVSSASTKGPSVFP
LAPSSKSTSGGTAALGCLVKDYFPEPVTVSWNSGALTSGVHTFPA
VLQSSGLYSLSSVVTVPSSSLGTQTYICNVNHKPSNTKVDKKVEPK
 
SCDKTHTCPPCPAPEAAGGPSVFLFPPKPKDTLMISRTPEVTCVVV
DVSHEDPEVKFNWYVDGVEVHNAKTKPREEQYNSTYRVVSVLTV
LHQDWLNGKEYKCKVSNKALPAPIEKTISKAKGQPREPQVYTLPP
SRDELTKNQVSLTCLVKGFYPSDIAVEWESNGQPENNYKTTPPVL
DSDGSFFLYSKLTVDKSRWQQGNVFSCSVMHEALHNHYTQKSLS
LSPGK (SEQ ID NO: 47)
48. The amino acid sequence of the light chain of RG6058(G1DM) 
DIVMTQSPDSLAVSLGERATINCKSSQTVLYSSNNKKYLAWYQQK
PGQPPNLLIYWASTRESGVPDRFSGSGSGTDFTLTISSLQAEDVAV
YYCQQYYSTPFTFGPGTKVEIKRTVAAPSVFIFPPSDEQLKSGTAS
VVCLLNNFYPREAKVQWKVDNALQSGNSQESVTEQDSKDSTYSLS
STLTLSKADYEKHKVYACEVTHQGLSSPVTKSFNRGEC (SEQ ID
NO: 48)
49. The amino acid sequence of a TGF-βRII extracellular region fragment variant of TF01A: 
IPPHVQKAVNNDMIVTDNNGAVKFPQLCKFCDVRFSTCDNQKSC
MSNCSITSICEKPQEVCVAVWRKNDENITLETVCHDPKLPYHDFIL
EDAASPKCIMKEKKKPGETFFMCSCSSDECNDNIIFSEEYNTSNPD
(SEQ ID NO: 49)
50. The amino acid sequence of a TGF-βRII extracellular region fragment variant of TF01G: 
IPPHVQKGVNNDMIVTDNNGAVKFPQLCKFCDVRFSTCDNQKSC
MSNCSITSICEKPQEVCVAVWRKNDENITLETVCHDPKLPYHDFIL
EDAASPKCIMKEKKKPGETFFMCSCSSDECNDNIIFSEEYNTSNPD
(SEQ ID NO: 50)
51. The amino acid sequence of a TGF-βRII extracellular region fragment variant of TF01T: 
IPPHVQKTVNNDMIVTDNNGAVKFPQLCKFCDVRFSTCDNQKSC
MSNCSITSICEKPQEVCVAVWRKNDENITLETVCHDPKLPYHDFIL
EDAASPKCIMKEKKKPGETFFMCSCSSDECNDNIIFSEEYNTSNPD
(SEQ ID NO: 51)
52. The amino acid sequence of a TGF-βRII extracellular region fragment variant of antibody TF01t31: 
VNNDMIVTDNNGAVKFPQLCKFCDVRFSTCDNQKSCMSNCSITSI
CEKPQEVCVAVWRKNDENITLETVCHDPKLPYHDFILEDAASPKC
IMKEKKKPGETFFMCSCSSDECNDNIIFSEEYNTSNPD (SEQ ID NO:
52)
53. The amino acid sequence of a TGF-βRII extracellular region fragment variant of antibody TF01t37: 


 (SEQ ID NO: 53)
54. A variant of a TGF-βRII extracellular region fragment 
NNDMIVTDNNGAVKFPQLCKFCDVRFSTCDNQKSCMSNCSITSIC
EKPQEVCVAVWRKNDENITLETVCHDPKLPYHDFILEDAASPKCI
MKEKKKPGETFFMCSCSSDECNDNIIFSEEYNTSNPD (SEQ ID NO:
54)
55. A variant of a TGF-βRII extracellular region fragment 
NDMIVTDNNGAVKFPQLCKFCDVRFSTCDNQKSCMSNCSITSICE
KPQEVCVAVWRKNDENITLETVCHDPKLPYHDFILEDAASPKCIM
 
KEKKKPGETFFMCSCSSDECNDNIIFSEEYNTSNPD (SEQ ID NO:
55)
56. A variant of a TGF-βRII extracellular region fragment 
DMIVTDNNGAVKFPQLCKFCDVRFSTCDNQKSCMSNCSITSICEK
PQEVCVAVWRKNDENITLETVCHDPKLPYHDFILEDAASPKCIMK
EKKKPGETFFMCSCSSDECNDNIIFSEEYNTSNPD (SEQ ID NO: 56)
57. A variant of a TGF-βRII extracellular region fragment 
MIVTDNNGAVKFPQLCKFCDVRFSTCDNQKSCMSNCSITSICEKP
QEVCVAVWRKNDENITLETVCHDPKLPYHDFILEDAASPKCIMKE
KKKPGETFFMCSCSSDECNDNIIFSEEYNTSNPD (SEQ ID NO: 57)
58. A variant of a TGF-βRII extracellular region fragment 
IVTDNNGAVKFPQLCKFCDVRFSTCDNQKSCMSNCSITSICEKPQE
VCVAVWRKNDENITLETVCHDPKLPYHDFILEDAASPKCIMKEKK
KPGETFFMCSCSSDECNDNIIFSEEYNTSNPD (SEQ ID NO: 58)
59. A deleted fragment in a TGF-βRII extracellular region fragment
   IPPHVQKS (SEQ ID NO: 59)
60. A deleted fragment in a TGF-βRII extracellular region fragment
   IPPHVQKSVNNDMI (SEQ ID NO: 60)
61. A deleted fragment in a TGF-βRII extracellular region fragment
   IPPHVQKSV (SEQ ID NO: 61)
62. A deleted fragment in a TGF-βRII extracellular region fragment
   IPPHVQKSVN (SEQ ID NO: 62)
63. A deleted fragment in a TGF-βRII extracellular region fragment
   IPPHVQKSVNN (SEQ ID NO: 63)
64. A deleted fragment in a TGF-βRII extracellular region fragment
   IPPHVQKSVNND (SEQ ID NO: 64)
65. A deleted fragment in a TGF-βRII extracellular region fragment
   IPPHVQKSVNNDM (SEQ ID NO: 65)
66. A linker fragment
   GGGGSGGGGSGGGGSGGGGS (SEQ ID NO: 66)
67. The amino acid sequence of the heavy chain constant region of hIgG4WT 
ASTKGPSVFPLAPCSRSTSESTAALGCLVKDYFPEPVTVSWNSGAL
TSGVHTFPAVLQSSGLYSLSSVVTVPSSSLGTKTYTCNVDHKPSNT
KVDKRVESKYGPPCPPCPAPEFLGGPSVFLFPPKPKDTLMISRTPE
VTCVVVDVSQEDPEVQFNWYVDGVEVHNAKTKPREEQFNSTYRV
VSVLTVLHQDWLNGKEYKCKVSNKGLPSSIEKTISKAKGQPREPQ
VYTLPPSQEEMTKNQVSLTCLVKGFYPSDIAVEWESNGQPENNYK
TTPPVLDSDGSFFLYSRLTVDKSRWQEGNVFSCSVMHEALHNHYT
QKSLSLSLG (SEQ ID NO: 67)
68. The amino acid sequence of the heavy chain constant region of hIgG1WT 



 




 (SEQ ID NO: 68)
69. A linker fragment
   GGGGS (SEQ ID NO: 69)
70. A linker fragment
   GGGGSG (SEQ ID NO: 70)
71. The amino acid sequence of the heavy chain of B12-hG1 
QVQLVQSGAEVKKPGASVKVSCQASGYRFSNFVIHWVRQAPGQR
FEWMGWINPYNGNKEFSAKFQDRVTFTADTSANTAYMELRSLRS
ADTAVYYCARVGPYSWDDSPQDNYYMDVWGKGTTVIVSSASTKG
PSVFPLAPSSKSTSGGTAALGCLVKDYFPEPVTVSWNSGALTSGVH
TFPAVLQSSGLYSLSSVVTVPSSSLGTQTYICNVNHKPSNTKVDKK
VEPKSCDKTHTCPPCPAPELLGGPSVFLFPPKPKDTLMISRTPEVT
CVVVDVSHEDPEVKFNWYVDGVEVHNAKTKPREEQYNSTYRVVS
VLTVLHQDWLNGKEYKCKVSNKALPAPIEKTISKAKGQPREPQV
YTLPPSREEMTKNQVSLTCLVKGFYPSDIAVEWESNGQPENNYKT
TPPVLDSDGSFFLYSKLTVDKSRWQQGNVFSCSVMHEALHNHYT
QKSLSLSPG (SEQ ID NO: 71)
72. The amino acid sequence of the light chain of B12-hG1

72. The amino acid sequence of the light chain of B12-hG1

EIVLTQSPGTLSLSPGERATFSCRSSHSIRSRRVAWYQHKPGQAPR
LVIHGVSNRASGISDRFSGSGSGTDFTLTITRVEPEDFALYYCQVY
GASSYTFGQGTKLERKRTVAAPSVFIFPPSDEQLKSGTASVVCLLN

NFYPREAKVQWKVDNALQSGNSQESVTEQDSKDSTYSLSSTLTLS
KADYEKHKVYACEVTHQGLSSPVTKSFNRGEC (SEQ ID NO: 72)

### DETAILED DESCRIPTION

Embodiments of the present disclosure will be described in detail below with reference to examples, but those skilled in the art will appreciate that the following examples are only for illustrating the present disclosure and should not be construed as limitations to the scope of the present disclosure. The experimental procedures without specified conditions in the examples were conducted under conventional conditions or conditions recommended by the manufacturer. The used reagents or instruments without specified manufacturers were all commercially available conventional products.

The BALB/c mice were purchased from Guangdong Medical Laboratory Animal Center.

The C57 mice were purchased from Laboratory Animal Center, Guangzhou University of Chinese Medicine.

The MDA-MB-231 cells, U87-MG cells, and TF-1 cells were purchased from ATCC.

A549 was purchased from Cell Resource Center, Shanghai Institutes for Biological Sciences, Chinese Academy of Sciences.

The heavy and light chain amino acid sequences of positive control antibody tiragolumab were derived from the antibody described in WHO. Proposed INN: List 117. WHO Drug Information, 31(2): p104, 2017, and the sequences were identical to SEQ ID NOs: 40-41, respectively.

Tiragolumab and RG6058(hG1WT) are the same antibody in the present disclosure.

The sequences of positive control antibody RG6058(hG4) were derived from the antibody described in published patent No. CN108290946A, and the amino acid sequences of the heavy and light chain variable regions are identical to SEQ ID NOs: 42-43, respectively.

Positive control antibody RG6058(hG1DM) was prepared at a laboratory of Akeso Biopharma Inc., and its lot number was 20180816. The amino acid sequence of its heavy chain is set forth in SEQ ID NO: 47, and the amino acid sequence of its light chain is set forth in SEQ ID NO: 48.

The variable region sequences of the isotype control antibody used in the examples of the present disclosure, human anti-hen egg lysozyme IgG (anti-HEL, or human IgG, abbreviated as hIgG), are described in Acierno et al., Affinity maturation increases the stability and plasticity of the Fv domain of anti-protein antibodies, J Mol Biol., 2007; 374(1):130-46. The hIgG1DM and hIgG4WT used in the examples were isotype control antibodies of anti-HEL containing hG1DM and hG4WT constant region sequences and were prepared at a laboratory of Akeso Biopharma Inc.

The anti-HEL&TGFβ antibody was constructed by fusing TGFβRII protein to the C-terminus of the heavy chain of an anti-HEL antibody of subtype k (human hG4DM) and was prepared at a laboratory of Akeso Biopharma Inc. The amino acid sequence of its heavy chain is set forth in SEQ ID NO: 45, and the amino acid sequence of its light chain is set forth in SEQ ID NO: 46.

TIGIT-mFc protein (Lot No. 20171110), TGF-βRII-mFc (Lot No. 20200528), CD155-hFc-Biotin (Lot No. 20170721), CD155-mFc (Lot No. 20190726), and CD112-mFc (Lot No. 20190726) were all produced by Akeso Biopharma Inc. according to known sequences. mFc represents an Fc protein fragment of mouse IgG, and hFc represents an Fc protein fragment of human IgG.

TGF-β1 protein was purchased from Sino Biological Inc., and the catalog number was LC13DE3108.

TGF-β2 protein was purchased from Peprotech Inc., and the catalog number was 0420345 E0620.

TGF-β3 protein was purchased from Peprotech Inc., and the catalog number was 0713410 A0219.

In the following examples of the present disclosure, the 293T-TIGIT cell line used was constructed by Akeso Biopharma Inc. The 293T-TIGIT cell line was prepared by viral infection of HEK293T cells (Wuhan University). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vector used was plenti6.3/V5-TIGITFL-BSD (TIGIT, Genebank ID: NP_776160.2; vector plenti6.3/V5 TOPO was purchased from Invitrogen Inc., and the product number was K531520).

In the following examples of the present disclosure, the CHO-K1-TIGIT cell line used was constructed by Akeso Biopharma Inc. The CHO-K1-TIGIT cell line was prepared by viral infection of CHO-K1 cells (Cell Resource Center, Institute of Basic Medical Sciences, Chinese Academy of Medical Sciences). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vector used was plenti6.3/V5-TIGITFL-BSD (TIGIT, Genebank ID: NP_776160.2; vector plenti6.3/V5 TOPO was purchased from Invitrogen Inc., and the product number was K531520).

In the following examples of the present disclosure, the Jurkat-TIGIT cell line used was constructed by Akeso Biopharma Inc. The Jurkat-TIGIT cell line was prepared by viral infection of Jurkat cells (Cell Center, Chinese Academy of Sciences). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vector used was plenti6.3/V5-TIGITFL-BSD (TIGIT, Genebank ID: NP_776160.2; vector plenti6.3/V5 TOPO was purchased from Invitrogen Inc., and the product number was K531520).

In the following examples of the present disclosure, the HT1080-aCD3scFv cell line used was constructed by Akeso Biopharma Inc. The HT1080-aCD3scFv cell line was prepared by viral infection of HT-1080 cells (Cell Resource Center, Shanghai Institutes for Biological Sciences, Chinese Academy of Sciences). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vector used was pCDH-aCD3scFv-Puro (the aCD3scFv sequence was derived from an anti-human CD3 Mus musculus OKT3 hybridoma antibody; vector pCDH-CMV-MCS-EF1-Puro was purchased from Youbio, and the product number was VT1480). In the following examples of the present disclosure, the 293T-TGFβR2-Luc cell line used was constructed by Akeso Biopharma Inc. The 293T-TGFβR2-Luc cell line was prepared by viral infection of HEK293T cells (Wuhan University). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vectors used were pCDH-hTGFBR2FL-puro and pCDH-Smad3/4-Luc2P-Hygro (hTGFBR2FL Genebank ID: NP_003233.4; vector pCDH-CMV-MCS-EF1-Puro was purchased from Youbio, and the product number was VT1480; pCDH-Smad3/4-Luc2P-Hygro was constructed by Akeso Biopharma).

In the following examples of the present disclosure, the Jurkat-TIGIT-NFAT-Luc cell line used was constructed by Akeso Biopharma Inc. The Jurkat-TIGIT-NFAT-Luc cell line was prepared by viral infection of Jurkat cells (Cell Center, Chinese Academy of Sciences). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vectors used were plenti6.3/V5-TIGITFL-BSD and pCDH-NFAT-hygro (TIGIT, Genebank ID: NP_776160.2; vector plenti6.3/V5 TOPO was purchased from Invitrogen Inc., and the product number was K531520; pCDH-NFAT-hygro was constructed by Akeso Biopharma).

In the following examples of the present disclosure, the CHO-aAPC-PDL1-PVR cell line used was constructed by Akeso Biopharma Inc. The CHO-aAPC-PDL1-PVR cell line was prepared by viral infection of PD-L1 aAPC/CHO-K1 cells (purchased from Promega Inc.). The viral preparation used 3rd Generation Lentiviral Systems. See, for example, A Third Generation Lentivirus Vector with a Conditional Packaging System. Dull T, Zufferey R, Kelly M, Mandel RJ, Nguyen M, Trono D, and Naldini L. J Virol. 1998. 72(11):8463-8471. The lentivirus expression vector used was pCDH-PVRFL-Puro (PVRFL Genebank ID: NP_006496.4; vector pCDH-CMV-MCS-EF1-Puro was purchased from Youbio, and the product number was VT1480).

### Example 1: Preparation of Anti-TIGIT Antibody

### 1. Preparation of hybridoma cell line LT019

The antigen used to prepare the anti-TIGIT antibody was human TIGIT-mFc (TIGIT was Genbank ID: NP_776160.2). Spleen cells of immunized mice were fused with mouse myeloma cells to prepare hybridoma cells. With human TIGIT-hFc as an antigen, the hybridoma cells were screened by indirect ELISA to obtain hybridoma cells capable of secreting an antibody that specifically binds to TIGIT. The hybridoma cells obtained by screening were subjected to a limiting dilution assay to obtain a stable hybridoma cell line. The above hybridoma cell line was designated hybridoma cell line LT019, and the monoclonal antibody secreted by the cell line was designated 26B12.

Hybridoma cell line LT019 (also referred to as TIGIT-26B12) was deposited at the China Center for Type Culture Collection (CCTCC) on October 23, 2020. The CCTCC designation was CCTCC NO. C2020208, and the deposition address was Wuhan University, Wuhan, China, postal code: 430072.

### 2. Preparation of anti-TIGIT antibody 26B12

The LT019 cell line prepared above was cultured in a chemical defined medium (CD medium, containing 1% penicillin-streptomycin) at 37 °C with 5% CO₂. After 7 days, the cell culture supernatant was collected, subjected to high-speed centrifugation and vacuum filtration through a microfiltration membrane, and purified using a HiTrap protein A HP column to obtain antibody 26B12.

### Example 2: Sequence Analysis of Anti-TIGIT Antibody 26B12

mRNA was extracted from the LT019 cell line cultured in Example 1 according to the instructions of a RNAprep pure Cell/Bacteria Kit (Tiangen, Cat. No. DP430).

cDNA was synthesized according to the instructions of an Invitrogen SuperScript^{®} III First-Strand Synthesis System for RT-PCR kit and amplified by PCR.

The PCR amplification product was directly subjected to TA cloning according to the instructions of a pEASY-T1 Cloning Kit (Transgen CT101).

The TA cloning products were directly sequenced, and the sequencing results are as follows:

The nucleotide sequence of the heavy chain variable region is set forth in SEQ ID NO: 2, and the fragment is 363 bp in length.

The amino acid sequence it encodes is set forth in SEQ ID NO: 1 and is 121 amino acids in length.

The sequence of heavy chain HCDR1 is set forth in SEQ ID NO: 3, the sequence of HCDR2 is set forth in SEQ ID NO: 4, and the sequence of HCDR3 is set forth in SEQ ID NO: 5.

The nucleotide sequence of the light chain variable region is set forth in SEQ ID NO: 7 and is 321 bp in length.

The amino acid sequence it encodes is set forth in SEQ ID NO: 6 and is 107 amino acids in length.

The sequence of light chain LCDR1 is set forth in SEQ ID NO: 8, the sequence of LCDR2 is set forth in SEQ ID NO: 9, and the sequence of LCDR3 is set forth in SEQ ID NO: 10.

### Example 3: Design and Preparation of Anti-Human TIGIT Humanized Antibodies and Mutant Antibodies

### 1. Design of light and heavy chains of anti-human TIGIT humanized antibodies 26B12H1L1, 26B12H4L1, 26B12H2L2, 26B12H3L2, 26B12H2L3, 26B12H3L3, 26B12H1L4, and 26B12H4L4

Based on the three-dimensional crystal structure of human TIGIT protein and the sequences of antibody 26B12 obtained in Example 2, the variable region sequences of antibodies 26B12H1L1, 26B12H4L1, 26B12H2L2, 26B12H3L2, 26B12H2L3, 26B12H3L3, 26B12H1L4, and 26B12H4L4 (the antibody constant region sequences were from the NCBI database; the heavy chain constant region was the Ig gamma-1 chain C region, ACCESSION: P01857; the light chain constant region was the Ig kappa chain C region, ACCESSION: P01834) were designed.

The designed variable region sequences are shown in Table 1 below.

**Table 1: The variable region sequences of anti-human TIGIT humanized antibodies**

| **Name** | **Heavy chain variable region amino acid sequence SEQ ID NO:** | **Heavy chain variable region nucleotide sequence SEQ ID NO:** | **Light chain variable region amino acid sequence SEQ ID NO:** | **Light chain variable region nucleotide sequence SEQ ID NO:** |
|---|---|---|---|---|
| **26B12H1L1** | **11** | **12** | **19** | **20** |
| **26B12H4L1** | **17** | **18** | **19** | **20** |
| **26B12H2L2** | **13** | **14** | **21** | **22** |
| **26B12H2L3** | **13** | **14** | **23** | **24** |
| **26B12H3L2** | **15** | **16** | **21** | **22** |
| **26B12H3L3** | **15** | **16** | **23** | **24** |
| **26B12H1L4** | **11** | **12** | **25** | **26** |
| **26B12H4L4** | **17** | **18** | **25** | **26** |

The nucleotide sequences of the heavy chain variable regions of the above 8 antibodies 26B12H1L1, 26B12H4L1, 26B12H2L2, 26B12H3L2, 26B12H2L3, 26B12H3L3, 26B12H1L4, and 26B12H4L4 are all 363 bp in length, and the amino acid sequences they encode are all 121 aa in length. The nucleotide sequences of their light chain variable regions are all 321 bp in length, and the amino acid sequences they encode are all 107 aa in length.

Moreover, the above 8 antibodies had the same HCDR1-HCDR3 and LCDR1-LCDR3:
the sequence of HCDR1 is set forth in SEQ ID NO: 3, the sequence of HCDR2 is set forth in SEQ ID NO: 4, and the sequence of HCDR3 is set forth in SEQ ID NO: 5;
the sequence of LCDR1 is set forth in SEQ ID NO: 8, the sequence of LCDR2 is set forth in SEQ ID NO: 9, and the sequence of LCDR3 is set forth in SEQ ID NO: 10.

### 2. Preparation of humanized antibodies 26B12H1L1, 26B12H4L1, 26B12H2L2, 26B12H3L2, 26B12H2L3, 26B12H3L3, 26B12H1L4, and 26B12H4L4

The heavy chain constant regions were all the Ig gamma-1 chain C region (ACCESSION: P01857), and the light chain constant regions were all the Ig kappa chain C region (ACCESSION: P01834).

The heavy chain cDNA and light chain cDNA of 26B12H1L1, the heavy chain cDNA and light chain cDNA of 26B12H4L1, the heavy chain cDNA and light chain cDNA of 26B12H2L2, the heavy chain cDNA and light chain cDNA of 26B12H3L2, the heavy chain cDNA and light chain cDNA of 26B12H2L3, the heavy chain cDNA and light chain cDNA of 26B12H3L3, the heavy chain cDNA and light chain cDNA of 26B12H1L4, the heavy chain cDNA and light chain cDNA of 26B12H2L4, and the heavy chain cDNA and light chain cDNA of 26B12H4L4 were cloned into pUC57simple (provided by GenScript) vectors to obtain:
pUC57simple-26B12H1 and pUC57simple-26B12L1;
pUC57simple-26B12H4 and pUC57simple-26B12L1;
pUC57simple-26B12H2 and pUC57simple-26B12L2;
pUC57simple-26B12H3 and pUC57simple-26B12L2;
pUC57simple-26B12H2 and pUC57simple-26B12L3;
pUC57simple-26B12H3 and pUC57simple-26B12L3;
pUC57simple-26B12H1 and pUC57simple-26B12L4; and
pUC57simple-26B12H4 and pUC57simple-26B12L4, respectively.

With reference to the standard techniques described in Molecular Cloning: A Laboratory Manual (Second Edition), the heavy and light chain full-length genes synthesized by EcoRI&HindIII cleavage were subcloned into pcDNA3.1 expression vectors by cleavage with restriction enzyme EcoRI&HindIII to obtain expression plasmids pcDNA3.1-26B12H1, pcDNA3.1-26B12L1, pcDNA3.1-26B12H4, pcDNA3.1-26B12H2, pcDNA3.1-26B12L2, pcDNA3.1-26B12H3, pcDNA3.1-26B12L3, and pcDNA3.1-26B12L4, and the heavy/light chain genes of the recombinant expression plasmids were further sequenced. Subsequently, the designed gene combinations comprising corresponding light and heavy chain recombinant plasmids (pcDNA3.1-26B12H1/pcDNA3.1-26B12L1, pcDNA3.1-26B12H4/pcDNA3.1-26B12L1, pcDNA3.1-26B12H2/pcDNA3.1-26B12L2, pcDNA3.1-26B12H3/pcDNA3.1-26B12L2, pcDNA3.1-26B12H2/pcDNA3.1-26B12L3, pcDNA3.1-26B12H3/pcDNA3.1-26B12L3, pcDNA3.1-26B12H1/pcDNA3.1-26B12L4, and pcDNA3.1-26B12H4/pcDNA3.1-26B12L4) were separately co-transfected into 293F cells, and the culture solutions were then collected and purified. After the products were verified by sequencing, endotoxin-free expression plasmids were prepared and transiently transfected into HEK293 cells for antibody expression. After 7 days of culture, the cell culture solutions were collected and subjected to affinity purification on a Protein A column to obtain humanized antibodies.

Humanized antibody 26B12H2L2 is also referred to herein as 26B12H2L2(hG1WT).

### 3.Design of humanized antibodies 26B12H2L2(hG1DM) and 26B12H2L2(hG4DM)

By introducing a leucine-to-alanine point mutation to position 234 (L234A) (according to the EU numbering system; this also applies hereinafter) and a leucine-to-alanine point mutation to position 235 (L235A) in the heavy chain constant region of 26B12H2L2, the inventors obtained a humanized antibody with the mutations in its constant region: 26B12H2L2(hG1DM). The amino acid sequence of 26B12H2(hG1DM), the heavy chain of 26B12H2L2(hG1DM), is set forth in SEQ ID NO: 31, and the amino acid sequence of its light chain is set forth in SEQ ID NO: 29.

By keeping the variable regions of 26B12H2L2 unchanged, using the Ig gamma-4 chain C region as a heavy chain constant region for the antibody, and introducing a phenylalanine-to-alanine point mutation to position 234 (F234A) and a leucine-to-alanine point mutation to position 235 (L235A) in the heavy chain constant region, the inventors obtained a humanized antibody with the mutations in its constant region: 26B12H2L2(hG4DM). The amino acid sequence of 26B12H2(hG4DM), the heavy chain of 26B12H2L2(hG4DM), is set forth in SEQ ID NO: 27, and the amino acid sequence of its light chain is set forth in SEQ ID NO: 29.

Humanized antibodies 26B12H2L2(hG1DM) and 26B12H2L2(hG4DM) can be prepared with reference to the method described in step 2 above.

### Example 4: Sequence Design and Preparation of Anti-TIGIT Antibody-TGF-βR Fusion Proteins

### 1. Sequence design

The composition of the anti-TIGIT antibody-TGF-βR fusion proteins in the present disclosure is shown in Table 2 below.

**Table 2: The composition of the heavy and light chains of the anti-TIGIT antibody-TGF-βR fusion proteins**

| **Fusion proteinname** | **Heavy chain of IgG moiety** | **Linker fragment** | **TGF-βRII extracellular region fragment** | **Light chain** |
|---|---|---|---|---|
| **TF01** | **SEQ ID NO: 27** | **SEQ ID NO: 44** | **SEQ ID NO: 33** | **SEQ ID NO: 29** |
| **TF02** | **SEQ ID NO: 31** | **SEQ ID NO: 44** | **SEQ ID NO: 33** | **SEQ ID NO: 29** |

The TGF-β receptor moiety had two peptide chains, and they were linked to the C-termini of the heavy chains of the IgG moiety by linker fragments, respectively.

### 2. Expression and purification of antibodies

The heavy chain cDNA sequences of TF01 and TF02 and their light chain cDNA sequences were cloned into pUC57simple (provided by Genscript) vectors to obtain plasmids pUC57simple-TF01H and pUC57simple-TF02H, and pUC57simple-TF01L and pUC57simple-TF02L, respectively. Plasmids pUC57simple-TF01H/pUC57simple TF01L and pUC57simple-TF02H/pUC57simple TF02L were subjected to enzyme cleavage (HindIII&EcoRI). The heavy and light chains isolated by electrophoresis were separately subcloned into pcDNA3.1 vectors, and recombinant plasmids were extracted and co-transfected into 293F cells. After 7 days of cell culture, the culture solution was centrifuged at high speed. The supernatant was concentrated and loaded onto a HiTrap MabSelect SuRe column. Proteins were eluted in one step with an elution buffer. The samples of interest were isolated and buffer-exchanged into PBS. Thus, anti-TIGIT antibody-TGF-βR fusion proteins TF01 and TF02 were obtained.

### Example 5: Sequence Design and Preparation of Anti-TIGIT Antibody-TGF-βR Fusion Proteins (Mutants)

The TGF-βRII extracellular region fragment in TF01, an anti-TIGIT antibody-TGF-βR fusion protein of the present disclosure, was subjected to point mutation by mutating the serine at position 8 of the TGF-βRII extracellular region fragment (SEQ ID NO: 33) to alanine, glycine, or threonine, and the new antibodies obtained were designated TF01A, TF01G, and TF01T, respectively. These antibodies were prepared with reference to Example 4.

**Table 3: The composition of the heavy and light chains of the anti-TIGIT antibody-TGF-βR fusion proteins (mutants)**

| **Fusion protein name** | **Heavy chain of IgG moiety** | **Linker fragment** | **TGF-βRII extracellular region fragment variant** | **Light chain** |
|---|---|---|---|---|
| **TF01A** | **SEQ ID NO: 27** | **SEQ ID NO: 44** | **SEQ ID NO: 49** | **SEQ ID NO: 29** |
| **TF01G** | **SEQ ID NO: 27** | **SEQ ID NO: 44** | **SEQ ID NO: 50** | **SEQ ID NO: 29** |
| **TF01T** | **SEQ ID NO: 27** | **SEQ ID NO: 44** | **SEQ ID NO: 51** | **SEQ ID NO: 29** |

### Example 6: Sequence Design and Preparation of Anti-TIGIT Antibody-TGF-βR Fusion Proteins (Truncated Forms)

The TGF-βRII extracellular region fragment in TF01, an anti-TIGIT antibody-TGF-βR fusion protein of the present disclosure, was partially truncated by deleting the sequence IPPHVQKS to obtain a fusion protein designated TF01t31 or by deleting the sequence IPPHVQKSVNNDMI to obtain a fusion protein designated TF01t37. These fusion proteins were prepared with reference to Example 4.

**Table 4: The composition of the heavy and light chains of the anti-TIGIT antibody-TGF-βR fusion proteins (truncated forms)**

| **Fusion protein name** | **Heavy chain of IgG moiety** | **Linker fragment** | **TGF-βRII extracellular region fragment variant** | **Light chain** |
|---|---|---|---|---|
| **TF01t31** | **SEQ ID NO: 27** | **SEQ ID NO: 66** | **SEQ ID NO: 52** | **SEQ ID NO: 29** |
| **TF01t37** | **SEQ ID NO: 27** | **SEQ ID NO: 66** | **SEQ ID NO: 53** | **SEQ ID NO: 29** |

### Example 7: Determination of Binding Activity of TF01 and TF02 to Antigen or TGF-β by ELISA

### 1. Determination of binding activity of TF01, TF02, 26B12H2L2(hG4DM), and RG6058(hG4) to antigen TIGIT-mFc by indirect ELISA

The specific method was as follows:
An ELISA plate was coated with 2 µg/mL goat anti-mouse IgG Fc (Jackson, Cat. No. 115-005-071) and incubated overnight at 4 °C. The ELISA plate was then washed once with PBST and blocked with a PBS solution containing 1% BSA (blocking solution) at 37 °C for 2 h. After the blocking, the ELISA plate was washed 3 times with PBST. Then 1 µg/mL TIGIT-mFc was added, and the plate was incubated at 37 °C for 30 min and then washed 3 times with PBST. The antibodies, serially diluted in PBST solution (the dilution gradients for the antibodies are detailed in Table 5), were added. The ELISA plate to which the test antibodies were added was incubated at 37 °C for 30 min and then washed 3 times with PBST. After the washing, an HRP-labeled goat anti-human IgG (H+L) (Jackson, Cat. No. 109-035-098) secondary antibody working solution diluted at a ratio of 1:5000 was added, and the plate was then incubated at 37 °C for 30 min. After the incubation, the plate was washed 4 times with PBST. Color development was then performed with TMB (Neogen, 308177) in a dark place for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

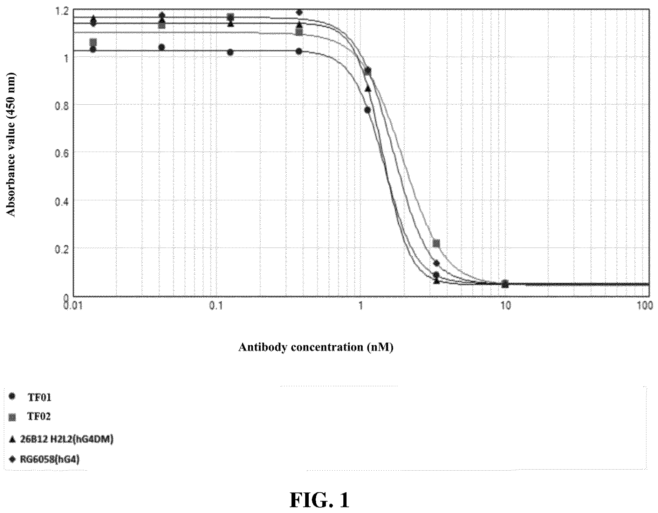

The results are shown in Table 5 and FIG. 1.

**Table 5: The detection of the binding of TF01, TF02, 26B12H2L2(hG4DM), and RG6058(hG4) to TIGIT-mFc by ELISA**

| **TIGIT-mFe (1 µg/mL), 50 µL/well** | | | | | | | | |
|---|---|---|---|---|---|---|---|---|
| **Antibody dilution gradient (nM)** | **TF01** | | **TF02** | | **26B12H2L2(hG4DM)** | | **RG6058(hG4)** | |
| | **Initial antibody concentration (µg/mL)** | | | | | | | |
| | **0.399** | | **0.399** | | **0.325** | | **0.332** | |
| **2.240** | **2.965** | **2.902** | **2.950** | **2.911** | **2.952** | **2.999** | **3.032** | **3.017** |
| **0.747** | **2.921** | **2.862** | **2.861** | **2.819** | **2.972** | **2.993** | **3.018** | **3.040** |
| **0.249** | **2.625** | **2.473** | **2.374** | **2.350** | **2.703** | **2.650** | **2.691** | **2.739** |
| **0.083** | **1.808** | **1.669** | **1.475** | **1.438** | **2.054** | **2.117** | **1.898** | **2.015** |
| **0.028** | **0.889** | **0.818** | **0.700** | **0.685** | **1.134** | **1.158** | **0.992** | **1.081** |
| **0.009** | **0.404** | **0.362** | **0.305** | **0.307** | **0.529** | **0.546** | **0.460** | **0.505** |
| **0.0031** | **0.210** | **0.178** | **0.165** | **0.167** | **0.252** | **0.265** | **0.223** | **0.261** |
| **0** | **0.109** | **0.102** | **0.097** | **0.105** | **0.098** | **0.117** | **0.113** | **0.131** |

| **Secondary antibody** | **Goat anti-human IgG (H+L), HRP (1:5000)** | | | | | | | |
|---|---|---|---|---|---|---|---|---|
| **EC₅₀ (nM)** | **0.067** | | **0.091** | | **0.046** | | **0.055** | |

It can be seen from the figure that TF01, TF02, 26B12H2L2(hG4DM), and RG6058(hG4) effectively bound to antigen TIGIT-mFc in a dose-dependent manner. The EC₅₀ values at various doses are shown in Table 3. By quantitative absorbance analysis of the antibodies that bound, curve fitting, and calculations, the binding efficiency EC₅₀ values of TF01, TF02, 26B12H2L2(hG4DM), and RG6058(hG4) (as a control) were obtained, and they were 0.067 nM, 0.091 nM, 0.046 nM, and 0.055 nM, respectively. The above experimental results show that TF01, TF02, 26B12H2L2(hG4DM), and RG6058(hG4) had the activity of effectively binding to TIGIT-mFc.

### 2. Determination of binding activity of TF01, TF02, and TGF-βRII-mFc to TGF-β1, TGF-β2, and TGF-β3 by indirect ELISA

The specific method was as follows:
ELISA plates were coated with 1 µg/mL TGF-β1, 2 µg/mL TGF-β2, and 1 µg/mL TGF-β3, respectively, and incubated overnight at 4 °C. Then the antigen-coated ELISA plates were washed once with PBST and blocked with a PBS solution containing 1% BSA (blocking solution) at 37 °C for 2 h. After the blocking, the ELISA plates were washed 3 times with PBST. The antibodies and TGF-βRII-mFc, serially diluted in PBST solution, were added. The ELISA plates to which the test antibodies and TGF-βRII-mFc were added were incubated at 37 °C for 30 min and then washed 3 times with PBST. After the washing, HRP-labeled goat anti-human IgG (H+L) (Jackson, Cat. No. 109-035-098) and HRP-labeled goat anti-mouse IgG Fc (Jackson, Cat. No. 115-035-071) secondary antibody working solutions diluted at a ratio of 1:5000 were added, and the plates were then incubated at 37 °C for 30 min. After the incubation, the plates were washed 4 times with PBST. Color development was then performed with TMB (Neogen, 308177) in a dark place for 5 min, and a stop solution was added to stop the color development reaction. The ELISA plates were immediately placed into a microplate reader, and the OD at a wavelength of 450 nm in each well of the ELISA plates was measured. The data were analyzed and processed using SoftMax Pro 6.2.1 software.

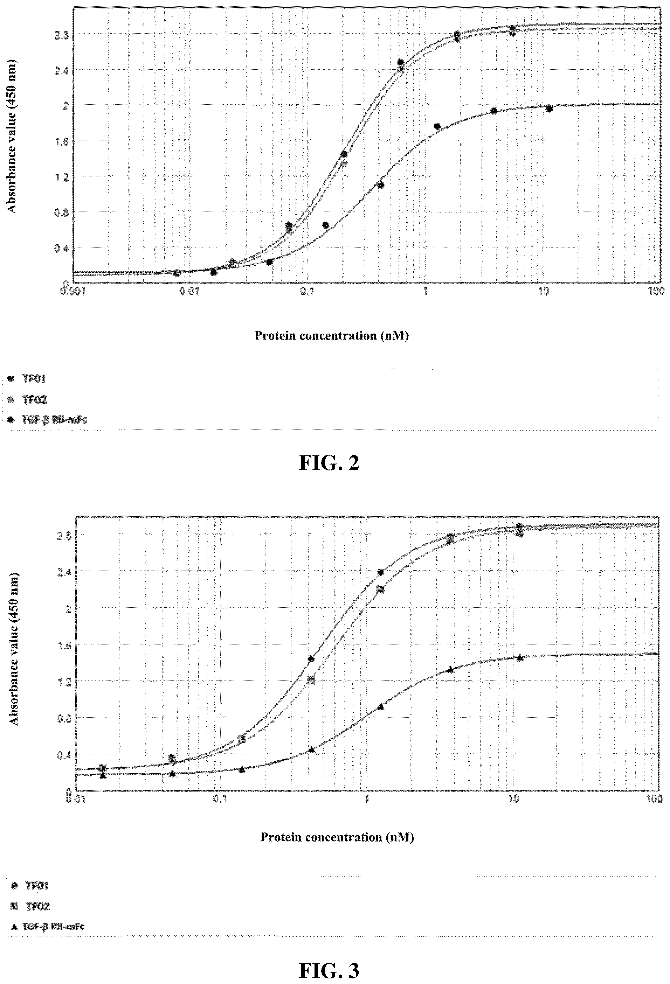

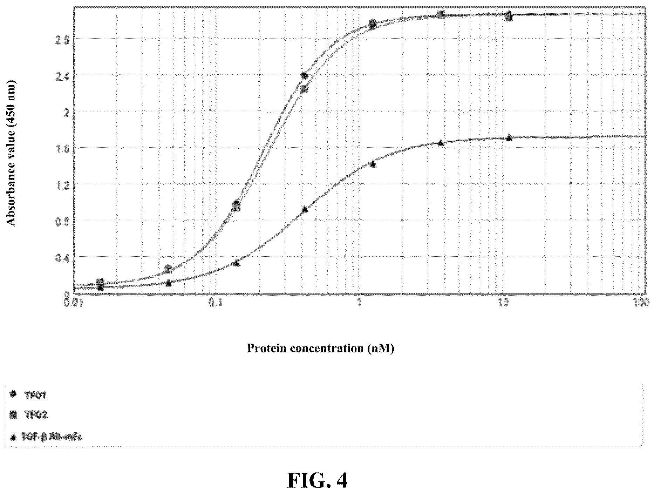

The results are shown in Tables 6, 7, and 8 and FIGs. 2, 3, and 4.

**Table 6: The detection of the binding of TF01, TF02, and TGF-βRII-mFc to TGF-β1 by ELISA**

| **Antibody dilution gradient (µg/mL)** | **TGF-β1 (1 µg/mL), 50 µL/well** | | | | | |
|---|---|---|---|---|---|---|
| | **TF01** | | **TF02** | | **TGF-β RII-mFc** | |
| **1.000** | **2.885** | **2.823** | **2.786** | **2.819** | **1.896** | **2.007** |
| **0.333** | **2.811** | **2.771** | **2.695** | **2.769** | **1.949** | **1.909** |
| **0.111** | **2.515** | **2.432** | **2.379** | **2.411** | **1.787** | **1.721** |
| **0.037** | **1.525** | **1.352** | **1.358** | **1.304** | **1.136** | **1.044** |
| **0.012** | **0.663** | **0.611** | **0.612** | **0.548** | **0.673** | **0.602** |
| **0.004** | **0.233** | **0.206** | **0.206** | **0.190** | **0.240** | **0.206** |
| **0.001** | **0.113** | **0.100** | **0.103** | **0.092** | **0.119** | **0.099** |
| **0.000** | **0.063** | **0.056** | **0.057** | **0.060** | **0.053** | **0.224** |

| **Secondary antibody** | **Goat anti-human** | | | | **Goat anti-mouse** | |
|---|---|---|---|---|---|---|
| | **IgG (H+L), HRP (1:5000), 50 µL/well** | | | | | |
| | | | | | **IgG mFC, HRP (1:5000), 50 µL/well** | |
| **EC₅₀ (nM)** | **0.207** | | **0.224** | | **0.356** | |

**Table 7: The detection of the binding of TF01, TF02, and TGF-βRII-mFc to TGF-β2 by ELISA**

| **Antibody dilution gradient (nM)** | **TGF-β2 (2 µg/mL)** | | | | | |
|---|---|---|---|---|---|---|
| | **TF01** | | **TF02** | | **TGF-β RII-mFc** | |
| **Initial protein concentration (µg/mL)** | **2.001** | | **2.000** | | **0.971** | |
| **11.23 nM** | **2.874** | **2.896** | **2.821** | **2.812** | **1.462** | **1.436** |
| **1:3** | **2.782** | **2.769** | **2.764** | **2.716** | **1.336** | **1.322** |
| **1:9** | **2.411** | **2.353** | **2.224** | **2.186** | **0.908** | **0.925** |
| **1:27** | **1.507** | **1.353** | **1.197** | **1.199** | **0.432** | **0.462** |
| **1:81** | **0.707** | **0.430** | **0.548** | **0.554** | **0.253** | **0.211** |
| **1:243** | **0.364** | **0.350** | **0.314** | **0.316** | **0.202** | **0.158** |
| **1:729** | **0.237** | **0.243** | **0.231** | **0.243** | **0.184** | **0.152** |
| **0** | **0.164** | **0.171** | **0.181** | **0.191** | **0.176** | **0.160** |

| **Secondary antibody** | **Goat anti-human IgG (H+L)-HRP (1:5000)** | | | | **Goat anti-mouse IgG Fc-HRP (1:5000)** | |
|---|---|---|---|---|---|---|
| **EC₅₀ (nM)** | **0.477** | | **0.594** | | **1.029** | |

**Table 8: The detection of the binding of TF01, TF02, and TGF-βRII-mFc to TGF-β3 by ELISA**

| **Antibody dilution gradient (nM)** | **TGF-β3(1 µg/mL)** | | | | | |
|---|---|---|---|---|---|---|
| | **TF01** | | **TF02** | | **TGF-β RII-mFc** | |
| **Initial protein concentration (µg/mL)** | **2.001** | | **2.000** | | **0.971** | |
| **11.23 nM** | **3.065** | **3.042** | **3.009** | **3.035** | **1.663** | **1.765** |
| **1:3** | **3.058** | **3.048** | **3.048** | **3.054** | **1.667** | **1.649** |
| **1:9** | **2.957** | **2.976** | **2.931** | **2.944** | **1.411** | **1.447** |
| **1:27** | **2.382** | **2.409** | **2.269** | **2.213** | **0.912** | **0.934** |
| **1:81** | **0.993** | **0.980** | **0.964** | **0.913** | **0.338** | **0.340** |
| **1:243** | **0.263** | **0.278** | **0.270** | **0.252** | **0.113** | **0.114** |
| **1:729** | **0.120** | **0.118** | **0.116** | **0.115** | **0.071** | **0.074** |
| **0** | **0.057** | **0.055** | **0.054** | **0.055** | **0.056** | **0.059** |

| **Secondary antibody** | **Goat anti-human IgG (H+L)-HRP (1:5000)** | | | | **Goat anti-mouse IgG Fc-HRP (1:5000)** | |
|---|---|---|---|---|---|---|
| **EC₅₀ (nM)** | **0.215** | | **0.234** | | **0.402** | |

By quantitative absorbance analysis of the antibodies that bound, curve fitting, and calculations, the binding efficiency EC₅₀ values of TF01 to TGF-β1, TGF-β2, and TGF-β3 were obtained, and they were 0.207 nM, 0.477 nM, and 0.215 nM, respectively; the binding efficiency EC₅₀ values of TF02 to TGF-β1, TGF-β2, and TGF-β3 were 0.224 nM, 0.594 nM, and 0.234 nM, respectively; the binding efficiency EC₅₀ values of TGF-βR2-mFc (as a positive control) to TGF-β1, TGF-β2, and TGF-β3 were 0.356 nM, 1.029 nM, and 0.402 nM, respectively.

The results show that TF01 and TF02 had the activity of effectively binding to TGF-β1, TGF-β2, and TGF-β3 in a dose-dependent manner. The results also show that TF01, TF02, and TGF-βRII-mFc all effectively bound to TGF-β1, TGF-β2, and TGF-β3, and the abilities of TF01 and TF02 to bind to TGF-β1, TGF-β2, and TGF-β3 were stronger than those of TGF-βRII-mFc.

### Example 8: Determination of Activity of TF01 and TF02 in Competing with TGF-βRII-His-Biotin for Binding to Human TGF-β1 and TGF-β3 by Competitive ELISA

Experimental steps: ELISA plates were coated with 2 µg/mL TGF-β1 and 2 µg/mL TGF-β3, respectively, and incubated overnight at 4 °C. After the incubation, the antigen-coated ELISA plates were rinsed once with PBST and then blocked with a PBS solution containing 1% BSA (ELISA plate blocking solution) for 2 h. After the blocking, the ELISA plates were washed 3 times with PBST. The antibodies and TGF-βRII-mFc, serially diluted in PBST solution, were added to the ELISA plates. The TGF-β1 ELISA plate was incubated at room temperature for 60 min and then washed 3 times with PBST, and 0.01 µg/mL TGF-βRII-His-Biotin was then added, followed by 10 min of incubation at room temperature. The TGF-β3 ELISA plate was incubated at 37 °C for 30 min and then washed 3 times with PBST, and 0.01 µg/mL TGF-βRII-His-Biotin was then added, followed by 30 min of incubation at 37 °C. After the incubation, the plates were washed 3 times with PBST. After the washing, an SA-HRP working solution diluted at a ratio of 1:4000 was added, and the plates were incubated at 37 °C for 30 min. After the incubation, the plates were washed 4 times with PBST. Color development was then performed with TMB (Neogen, 308177) in a dark place for 4 min, and a stop solution was added to stop the color development reaction. The ELISA plates were immediately placed into a microplate reader, and the OD at a wavelength of 450 nm in each well of the ELISA plates was measured. The data were analyzed and processed using SoftMax Pro 6.2.1 software.

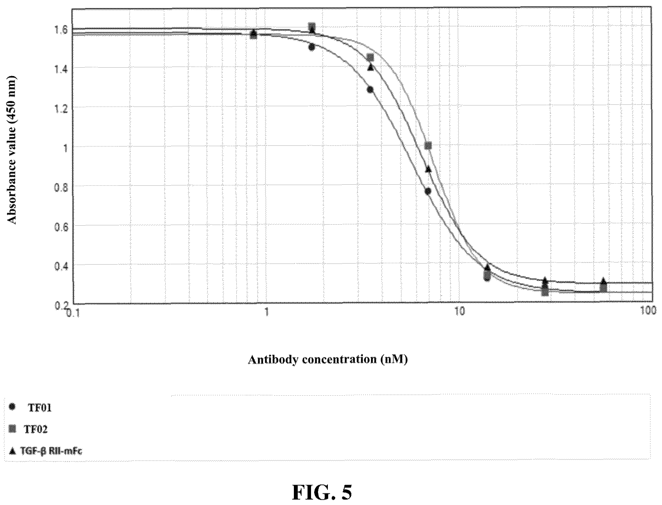

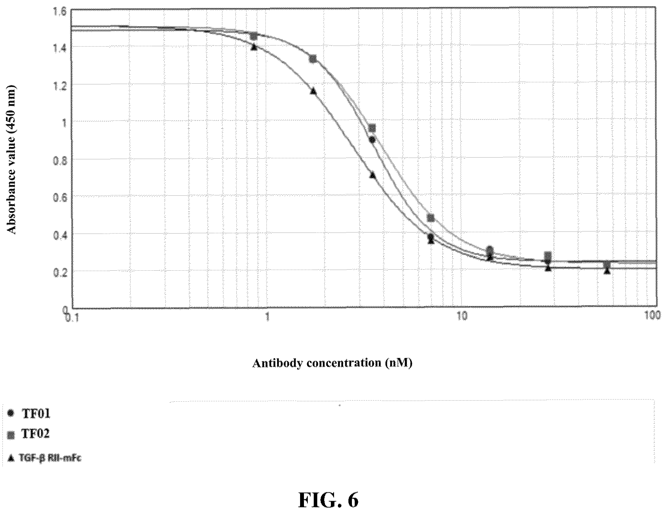

The activity of the antibodies in competing with TGF-βRII-His-Biotin for binding to TGF-β1 and TGF-β3 is shown in Tables 9 and 10 and FIGs. 5 and 6. Curves of absorbance (ordinate) vs. antibody concentration (abscissa) were fitted, and the EC₅₀ values of the antibodies competing with TGF-βRII-His-Biotin for binding to TGF-β1and TGF-β3 were calculated. The results are shown in Tables 9 and 10 below.

**Table 9: The activity of TF01 and TF02 in competing with TGF-βRII-His-Biotin for binding to TGF-β1**

| **Antibody dilution gradient (nM), 100 µL/well** | **TGF-β1, 2 µg/mL, 50 µL/well** | | | | | |
|---|---|---|---|---|---|---|
| | **TF01** | | **TF02** | | **TGF-β RII-mFc** | |
| **Initial protein concentration (µg/mL)** | **10.000** | | **9.998** | | **4.851** | |
| **56.134** | **0.252** | **0.281** | **0.256** | **0.279** | **0.294** | **0.317** |
| **28.067** | **0.275** | **0.270** | **0.222** | **0.266** | **0.305** | **0.311** |
| **14.034** | **0.324** | **0.317** | **0.316** | **0.350** | **0.396** | **0.361** |
| **7.017** | **0.714** | **0.815** | **0.961** | **1.032** | **0.933** | **0.828** |
| **3.508** | **1.267** | **1.296** | **1.385** | **1.508** | **1.435** | **1.360** |
| **1.754** | **1.491** | **1.497** | **1.590** | **1.618** | **1.601** | **1.576** |
| **0.877** | **1.561** | **1.573** | **1.524** | **1.595** | **1.609** | **1.543** |
| **0** | **1.629** | **1.561** | **1.570** | ***1.545*** | **1.609** | **1.603** |

| **TGF-βR2-His-Biotin, 0.01 µg/mL, 50 µL/well** | | | | | | |
|---|---|---|---|---|---|---|
| **Secondary antibody: SA-HRP (1:4000), 50 µL/well** | | | | | | |
| **EC₅₀ (nM)** | **5.759** | | **7.469** | | **6.475** | |

**Table 10: The activity of TF01 and TF02 in competing with TGF-βRII-His-Biotin for binding to TGF-β3**

| **Antibody dilution gradient, 50 µL/well** | **TGF-β3 (2 µg/mL), 50 µL/well** | | | | | |
|---|---|---|---|---|---|---|
| | **TF01** | | **TF02** | | **TGF-β RII-mFc** | |
| **Initial protein concentration (µg/mL)** | **10.000** | | **9.998** | | **4.851** | |
| **56.134** | **0.232** | **0.213** | **0.210** | **0.220** | **0.181** | **0.197** |
| **28.067** | **0.249** | **0.233** | **0.233** | **0.299** | **0.198** | **0.218** |
| **14.034** | **0.307** | **0.293** | **0.299** | **0.279** | **0.255** | **0.277** |
| **7.017** | **0.364** | **0.378** | **0.486** | **0.456** | **0.372** | **0.335** |
| **3.508** | **0.897** | **0.892** | **0.973** | **0.937** | **0.813** | **0.601** |
| **1.754** | **1.294** | **1.344** | **1.327** | **1.323** | **1.272** | **1.040** |
| **0.877** | **1.427** | **1.466** | **1.436** | **1.466** | **1.476** | **1.311** |
| **0** | **1.511** | **1.500** | **1.535** | **1.508** | **1.575** | **1.453** |

| **TGF-βRII-His-Biotin, 0.01 µg/mL, 50 µL/well** | | | | | | |
|---|---|---|---|---|---|---|
| **Secondary antibody** | **SA-HRP(1:4000)** | | | | | |
| **EC₅₀ (nM)** | **3.569** | | **3.879** | | **2.808** | |

By quantitative absorbance analysis of the antibodies that bound, curve fitting, and calculations, the binding efficiency EC₅₀ values of TF01, TF02, and TGF-βRII-mFc competing with TGF-βRII-His-Biotin for binding to TGF-β1 were obtained, and they were 5.579 nM, 7.469 nM, and 6.475 nM, respectively; the binding efficiency EC₅₀ values of TF01, TF02, and TGF-βRII-mFc competing with TGF-βRII-His-Biotin for binding to TGF-β3 were 3.569 nM, 3.879 nM, and 2.808 nM, respectively.

The results show that TF01 and TF02 effectively bound to TGF-β1 and TGF-β3 in a dose-dependent manner. In addition, the ability of TF01 to bind to TGF-β1 was stronger than that of TGF-βRII-His-Biotin. The ability of TF01 to compete with TGF-βRII-His-Biotin for binding to TGF-β1 was stronger than that of TGF-βRII-mFc.

### Example 9: Determination of Activity of TF01 and TF02 in Competing with CD155-hFc-Biotin for Binding to Human TIGIT-mFc by Competitive ELISA

An ELISA plate was coated with 2 µg/mL human TIGIT-mFc (TIGIT Genbank ID: NP_776160.2) and incubated overnight at 4 °C. After the incubation, the ELISA plate was blocked with a PBS solution containing 1% BSA at 37 °C for 2 h. After the blocking, the plate was washed three times and tapped dry. The antibodies were serially diluted at a ratio of 1:3 to 7 concentrations on a dilution plate, with 20.18 nM (final concentration: 10.09 nM) as the initial concentration, and a blank control was set. Then an equal volume of a 4 µg/mL (final concentration: 2 µg/mL) human CD155-hFc-Biotin solution was added. After thorough mixing, the mixtures were incubated at room temperature for 20 min. Then the reaction mixtures were added to the coated ELISA plate, and the plate was incubated at 37 °C for 30 min. After the incubation, the plate was washed three times with PBST and tapped dry. An SA-HRP (KPL, 14-30-00) working solution was added, and the plate was incubated at 37 °C for 30 min. After the incubation, the plate was washed four times and tapped dry. Color development was then performed with TMB (Neogen, 308177) in a dark place for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

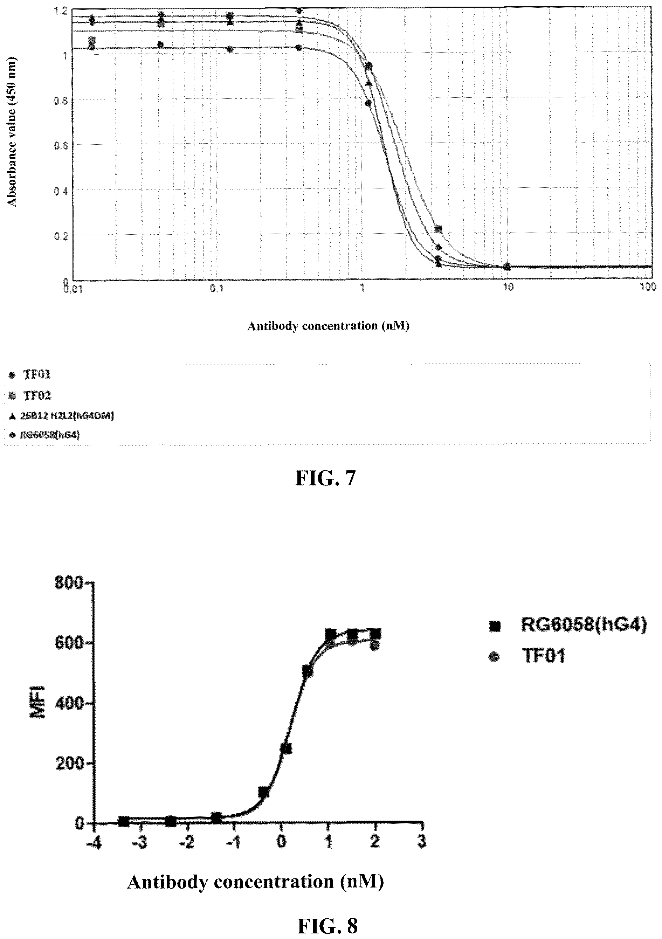

The results are shown in FIG. 7. The OD values at various doses are shown in Table 9. By quantitative absorbance analysis of the antibodies that bound and curve fitting, the binding efficiency EC₅₀ values of the antibodies were obtained (Table 11).

**Table 11: The activity of TF01, TF02, 26B12H2L2(hG4DM), and RG6058(hG4) in competing with CD155-hFc-Biotin for binding to TIGIT-mFc**

| **Antibody dilution (nM)** | **TIGIT-mFc (2 µg/mL), 50 µL/well** | | | | | | | |
|---|---|---|---|---|---|---|---|---|
| | **TF01** | | **TF02** | | **26B12H2L2 (hG4DM)** | | **RG6058(hG4)** | |
| **Initial final antibody concentration (µg/mL)** | **1.797** | | **1.797** | | **1.463** | | **1.496** | |
| **10.090** | **0.055** | **0.051** | **0.052** | **0.049** | **0.049** | **0.046** | **0.047** | **0.052** |
| **3.363** | **0.085** | **0.086** | **0.221** | **0.212** | **0.066** | **0.065** | **0.151** | **0.127** |
| **1.121** | **0.786** | **0.765** | **0.923** | **0.949** | **0.865** | **0.874** | **0.913** | **0.974** |
| **0.374** | **0.982** | **1.059** | **1.099** | **1.106** | **1.148** | **1.117** | **1.170** | **1.199** |
| **0.125** | **0.993** | **1.042** | **1.148** | **1.180** | **1.123** | **1.150** | **1.153** | **1.169** |
| **0.042** | **1.028** | **1.051** | **1.118** | **1.144** | **1.174** | **1.141** | **1.203** | **1.144** |
| 0.0138 | 1.080 | 0.979 | 1.036 | 1.083 | 1.155 | 1.165 | 1.137 | 1.139 |
| 0 | 1.001 | 1.039 | 1.011 | 1.058 | 1.095 | 1.130 | 1.182 | 1.126 |

| Primary antibody | CD155-hFc-Biotin (2 µg/mL), 50 µL/well | | | | | | | |
|---|---|---|---|---|---|---|---|---|
| Secondary antibody | SA-HRP(1:4000) | | | | | | | |
| EC₅₀ (nM) | 1.463 | | 1.963 | | 1.423 | | 1.675 | |

The EC₅₀ values of TF01, TF02, 26B12H2L2(hG4DM), and RG6058(hG4) (as a control) blocking the binding of TIGIT-mFc to its ligand CD155-hFc-Biotin were 1.463 nM, 1.963 nM, 1.423 nM, and 1.675 nM, respectively.

The results show that TF01, TF02, 26B12H2L2(hG4DM), and RG6058(hG4) (as a control) effectively blocked the binding of antigen human CD155-hFc-Biotin to its receptor human TIGIT-mFc, and the blocking efficiency showed a dose-dependent relationship; the ability of TF01 to block the binding of TIGIT-mFc to its ligand CD155-hFc-Biotin was stronger than that of the control antibody RG6058(hG4).

### Example 10: Determination of Binding Activity of TF01 to 293T-TIGIT Membrane Surface TIGIT by FACS

293T-TIGIT cells in the logarithmic growth phase were collected and transferred to a transparent 96-well V-bottom plate at 3 × 10⁵ cells/well. 1% PBSA was added, the plate was centrifuged at 350 g for 5 min, and the supernatant was removed. 100 µL of an antibody diluted in 1% PBSA (final concentrations: 100 nM, 33.33 nM, 11.11 nM, 3.7 nM, 1.23 nM, 0.41 nM, 0.041 nM, 0.0041 nM, and 0.00041 nM) was added. After gentle and thorough mixing, the plate was incubated on ice for 1 h. 1% PBSA was added, the plate was centrifuged at 350 g for 5 min, and the supernatant was removed. A 350-fold diluted FITC-labeled goat anti-human IgG secondary antibody (Jackson, Cat. No. 109-095-098) was added to resuspend the cells. After thorough mixing, the plate was incubated on ice in a dark place for 0.5 h. 1% PBSA was added, the plate was centrifuged at 350 g for 5 min, and the supernatant was removed. 400 µL of PBSA was added to resuspend the cell pellets, and the suspensions were transferred to flow cytometry tubes and analyzed by FACS Calibur.

The experimental results are shown in Table 12 and FIG. 8. Both TF01 and RG6058(hG4) (control antibody) specifically bound to 293T-TIGIT membrane surface TIGIT.

**Table 12: The determination of the binding activity of TF01 and RG6058(hG4) to 293T-TIGIT cell surface TIGIT by FACS**

| **Antibody dilution gradient (nM)** | **TF01** | **RG6058(hG4)** |
|---|---|---|
| **0.00041** | **8.83** | **9.07** |
| **0.0041** | **10.00** | **10.30** |
| **0.041** | **18.50** | **19.50** |
| **0.41** | **97.40** | **106.00** |
| **1.23** | **244.00** | **251.00** |
| **3.7** | **497.00** | **508.00** |
| **11.1** | **596.00** | **629.00** |
| **33.3** | **604.00** | **630.00** |
| **100** | **589.00** | **630.00** |
| **EC₅₀ (nM)** | **1.540** | **1.612** |
| **R2** | **0.9981** | **0.9981** |

Under the same experimental conditions, the EC₅₀ values of TF01 and RG6058(hG4) binding to 293T-TIGIT cells were 1.540 nM and 1.612 nM, respectively.

The results show that TF01 had the activity of effectively binding to 293T-TIGIT membrane surface TIGIT in a dose-dependent manner, and its binding ability was stronger than that of the control antibody RG6058(hG4).

### Example 11: Determination of Activity of TF01 in Competing with CD155 or CD112 for Binding to Cell Membrane Surface Antigen TIGIT by Competitive Flow Cytometry

293T-TIGIT cells were routinely digested, plated at 3 × 10⁵ cells/well/sample, and centrifugally washed. The antibody, corresponding serially diluted, was added at 100 µL/well, and the plate was incubated on ice for 30 min. CD155/CD112-mFc (prepared by Akeso Biopharma Inc., Lot No. 20190726/Lot No. 20190726) was added at 100 µL/well and well mixed, such that the final concentration was 10 nM/30 nM. The plate was incubated on ice for 1 h. 1% PBSA was added, the plate was centrifuged at 350 g for 5 min, and the supernatant was removed. An APC Goat anti-mouse IgG antibody (Biolegend, Cat. No. 405308) diluted at a ratio of 1:300 was added at 100 µL/well, and 100 µL of 1% PBSA was added to the blank sample. After thorough mixing, the plate was incubated on ice in a dark place for 30 min and centrifugally washed, and the cells were resuspended, transferred to flow cytometry tubes, and analyzed on a flow cytometer.

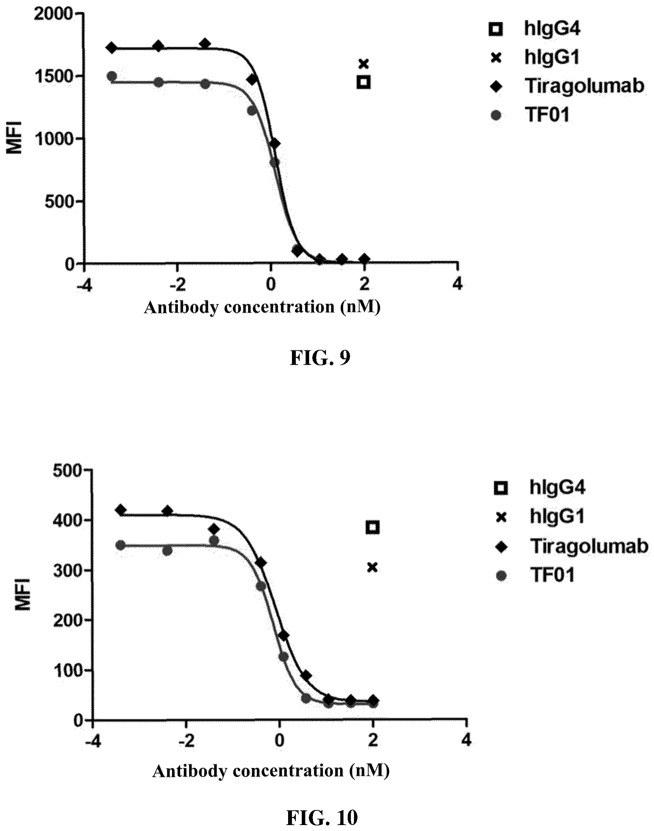

The results are shown in FIGs. 9 and 10, and the EC₅₀ values are shown in Table 13. By quantitative fluorescence analysis and curve fitting, the competitive binding EC₅₀ values of TF01 were calculated to be 1.2720 nM and 0.7445 nM.

**Table 13: FACS fluorescence intensity analysis of TF01 competing for binding to the 293T-TIGIT surface antigen**

| **Antibody dilution gradient (nM)** | **CD155** | | **CD112** | |
|---|---|---|---|---|
| | **TF01** | **Tiragolumab** | **TF01** | **Tiragolumab** |
| **0.00041** | **1499.0** | **1725.0** | **350.0** | **421.0** |
| **0.0041** | **1449.0** | **1739.0** | **339.0** | **418.0** |
| **0.041** | **1432.0** | **1754.0** | **359.0** | **382.0** |
| **0.41** | **1220.0** | **1469.0** | **267.0** | **314.0** |
| **1.23** | **802.0** | **956.0** | **125.0** | **168.0** |
| **3.7** | **113.0** | **95.8** | **42.7** | **88.1** |
| **11.1** | **25.9** | **30.4** | **33.1** | **40.6** |
| **33.3** | **25.5** | **30.0** | **33.1** | **38.2** |
| **100** | **25.0** | **31.1** | **32.5** | **38.3** |
| **EC₅₀ (nM)** | **1.2720** | **1.2760** | **0.7445** | **0.8370** |
| **R2** | **0.9958** | **0.9957** | **0.9985** | **0.9962** |

The results show that antibody TF01 effectively blocked the binding of CD155 and/or CD112 to 293T-TIGIT host cell surface TIGIT in a dose-dependent manner.

### Example 12: Determination of Affinity of TF01 for Fc Receptor FcγRI

Fc receptor FcyRI (also known as CD64) can bind to the Fc fragment of an IgG antibody, playing a role in antibody-dependent cell-mediated cytotoxicity (ADCC). The ability of a therapeutic antibody to bind to an Fc receptor influences the safety and efficacy of the antibody. In this experiment, the affinity constant of TF01 for FcyRI was determined using a Fortebio Octet molecular interaction instrument to assess the ADCC activity of the antibody.

The experimental method of determining the affinity constant of the corresponding antibody for FcyRI using a Fortebio Octet molecular interaction instrument is briefly described below: The sample dilution buffer was a solution of PBS, 0.02% Tween-20, and 0.1% BSA (pH 7.4). A 1 µg/mL FcyRI (purchased from Sinobio) solution was added to an HIS1K sensor, and the system was incubated for 50 s to immobilize FcγRI to the sensor surface. Both the association and dissociation constants of the antibody to FcyRI were determined in the buffer. The antibody concentration was 3.12-50 nM (serially diluted two-fold). The sample plate shaking speed was 1000 rpm, the temperature was 30 °C, and the frequency was 5.0 Hz. The data were analyzed by 1:1 model fitting to obtain affinity constants.

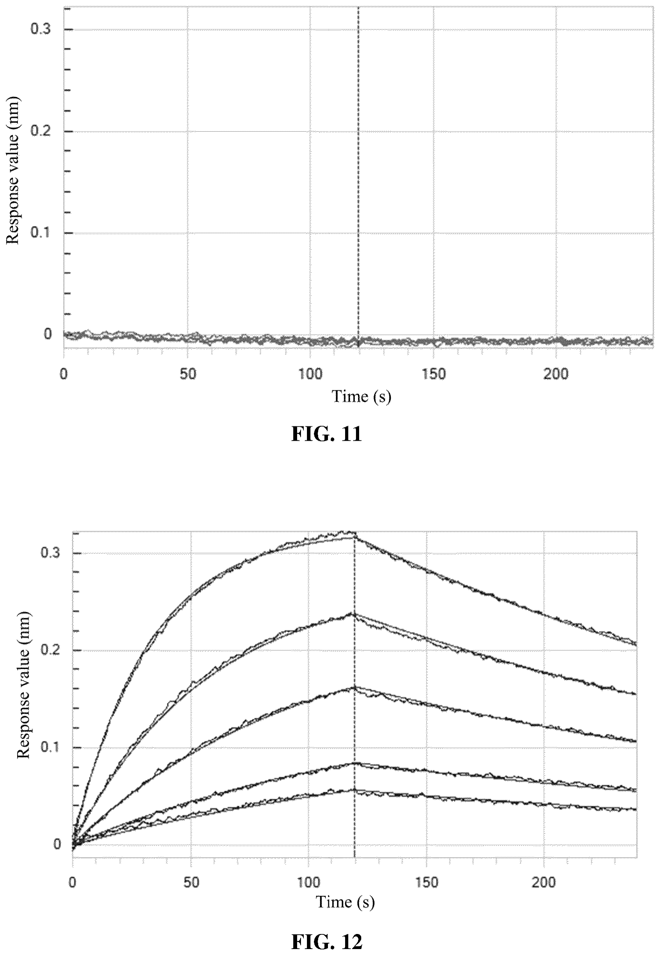

The affinity constant between FcyRI and TF01 is shown in Table 14 and FIGs. 11-12.

**Table 14: Kinetic parameters of TF01 binding to FcyRI**

| **Antibody** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **6.51E-09** | **5.58E+05** | **2.90E+03** | **3.64E-03** | **1.54E-05** | **0.30-0.37** |

N/A means that there was no binding between the antibody and the antigen, or the binding signal was extremely weak, so the results were not analyzed, and no corresponding data were obtained.

The results show that 26B12H2L2(hG1WT) bound to FcyRI with an affinity constant of 6.51E-09 M, and as there was no binding between TF01 and FcyRI or the binding signal was extremely weak, the results were not analyzed, and no corresponding data were obtained.

The results show that the binding activity of TF01 to FcγRI was effectively eliminated.

### Example 13: Determination of Affinities of TF01 for Fc Receptor FcγRIIIa and Its Subtypes

### (1) Determination of affinity constant between FcγRIIIa V158 and TF01

Fc receptor FcγRIIIa_V158 (also known as CD16a_V158) can bind to the Fc fragment of an IgG antibody, mediating an ADCC effect. In this experiment, the affinity constant of TF01 for FcγRIIIa_V158 was determined using a Fortebio Octet molecular interaction instrument to assess the ADCC activity of the antibody.

The experimental method of determining the affinity constant of the corresponding antibody using a Fortebio Octet molecular interaction instrument is briefly described below: The sample dilution buffer was PBS, 0.02% Tween-20, and 0.1% BSA, pH 7.4. 5 µg/mL FcγRIIIa_V158 was immobilized to an HIS1K sensor for 60 s, and the sensor was equilibrated in the buffer for 60 s. The FcγRIIIa_V158 immobilized to the sensor was allowed to bind to each antibody (antibody concentration: 31.25-500 nM, diluted two-fold) for 60 s, and the antibody was allowed to dissociate in the buffer for 60 s. The sample plate shaking speed was 1000 rpm, the temperature was 30 °C, and the frequency was 5.0 Hz. The data were analyzed by 1:1 model fitting to obtain affinity constants.

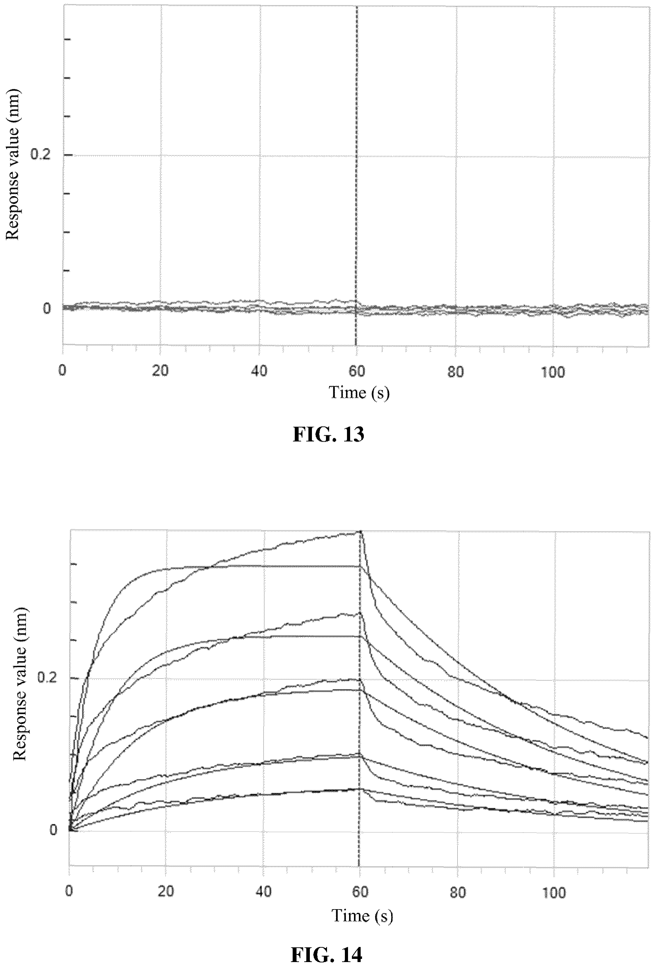

The affinity constant between FcγRIIIa_V158 and TF01 is shown in Table 15 and FIGs. 13-14.

**Table 15: Kinetic parameters of TF01 binding to FcγRIIIa_V158**

| **Antibody** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **5.54E-08** | **4.02E+05** | **1.68E+04** | **2.23E-02** | **3.84E-04** | **0.18-0.39** |

N/A means that there was no binding between the antibody and the antigen, or the binding signal was extremely weak, so the results were not analyzed, and no corresponding data were obtained.

The results show that 26B12H2L2(hG1WT) bound to FcγRIIIa_V158 with an affinity constant of 5.54E-08 M, and as there was no binding between TF01 and FcγRIIIa_V158 or the binding signal was extremely weak, the results were not analyzed.

The results show that the binding activity of TF01 to FcγRIIIa_V158 was effectively eliminated.

### (2) Determination of affinity constant between FcγRIIIa F158 and TF01

Fc receptor FcγRIIIa_F158 (also known as CD16a_F158) can bind to the Fc fragment of an IgG antibody, mediating ADCC. In this experiment, the affinity constant of TF01 for FcγRIIIa_F158 was determined using a Fortebio Octet molecular interaction instrument to assess the ADCC activity of the antibodies.

The experimental method of determining the affinity constant of TF01 for FcγRIIIa_F158 using a Fortebio Octet molecular interaction instrument is briefly described below: The sample dilution buffer was PBS, 0.02% Tween-20, and 0.1% BSA, pH 7.4. 5 µg/mL FcγRIIIa_F158 was immobilized to an HIS1K sensor for 120 s, and the sensor was equilibrated in the buffer for 60 s. The FcγRIIIa_F158 immobilized to the sensor was allowed to bind to each antibody (antibody concentration: 31.25-500 nM, diluted two-fold) for 60 s, and the antibody was allowed to dissociate in the buffer for 60 s. The sample plate shaking speed was 1000 rpm, the temperature was 30 °C, and the frequency was 5.0 Hz. The data were analyzed by 1:1 model fitting to obtain affinity constants.

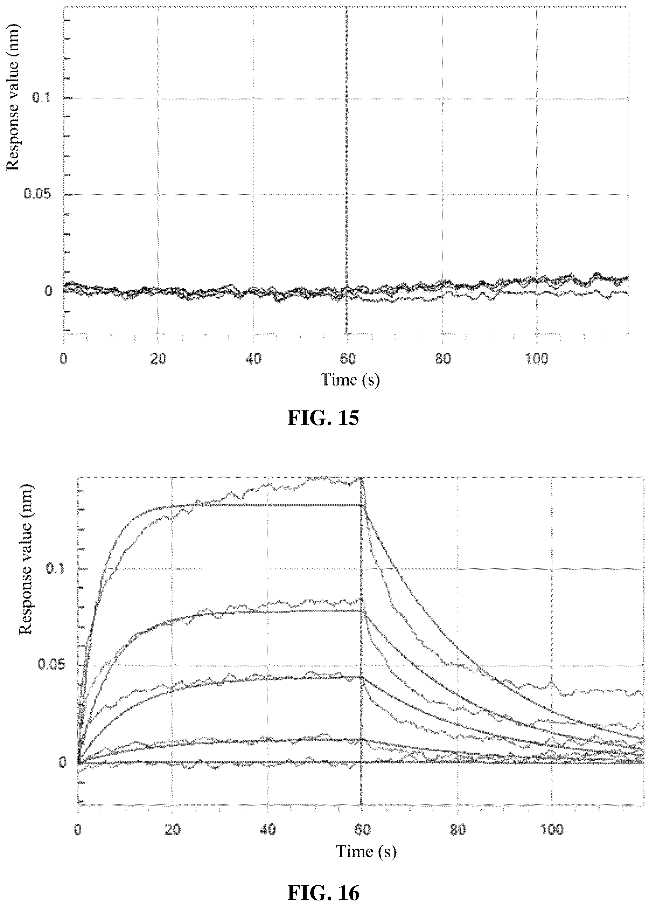

The affinity constant between FcγRIIIa_F158 and TF01 is shown in Table 16 and FIGs. 15 to 16.

**Table 16: Kinetic parameters of TF01 binding to FcγRIIIa_F158**

| **Antibody** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **9.97E-08** | **4.03E+05** | **2.28E+04** | **4.02E-02** | **7.95E-04** | **0.00-0.16** |

N/A means that there was no binding between the antibody and the antigen, or the binding signal was extremely weak, so the results were not analyzed, and no corresponding data were obtained.

The results show that 26B12H2L2(hG1WT) bound to FcγRIIIa_F158 with an affinity constant of 9.97E-08 M, and as there was no binding between TF01 and FcγRIIIa_F158 or the binding signal was extremely weak, the results were not analyzed, and no corresponding data were obtained.

The results show that the binding activity of TF01 to FcγRIIIa_F158 was effectively eliminated.

### Example 14: Determination of Affinities of TF01 for Fc Receptor FcγRIIa and Its Subtypes

### (1) Determination of affinity constant between FcγRIIa H131 and TF01

Fc receptor FcγRIIa_H131 (also known as CD32a_H131) can bind to the Fc fragment of an IgG antibody, playing a role in antibody-dependent cell-mediated cytotoxicity (ADCC). The ability of a therapeutic antibody to bind to an Fc receptor influences the safety and efficacy of the antibody. In this experiment, the affinity constant of TF01 for FcγRIIa_H131 was determined using a Fortebio Octet molecular interaction instrument to assess the abilities of the test antibodies to bind to the Fc receptor.

The experimental method of determining the affinity constant of TF01 for FcγRIIa_H131 using a Fortebio Octet molecular interaction instrument is briefly described below: The sample dilution buffer was PBS, 0.02% Tween-20, and 0.1% BSA, pH 7.4. 5 µg/mL FcγRIIa_H131 was immobilized to an NTA sensor at an immobilization height of about 1.0 nm, and the sensor was equilibrated in the buffer for 60 s. The FcγRIIa_H131 immobilized to the sensor was allowed to bind to each antibody (antibody concentration: 12.5-200 nM, serially diluted two-fold) for 60 s, and the antibody was allowed to dissociate in the buffer for 60 s. The sample plate shaking speed was 1000 rpm, the temperature was 30 °C, and the frequency was 5.0 Hz. The data were analyzed by 1:1 model fitting to obtain affinity constants.

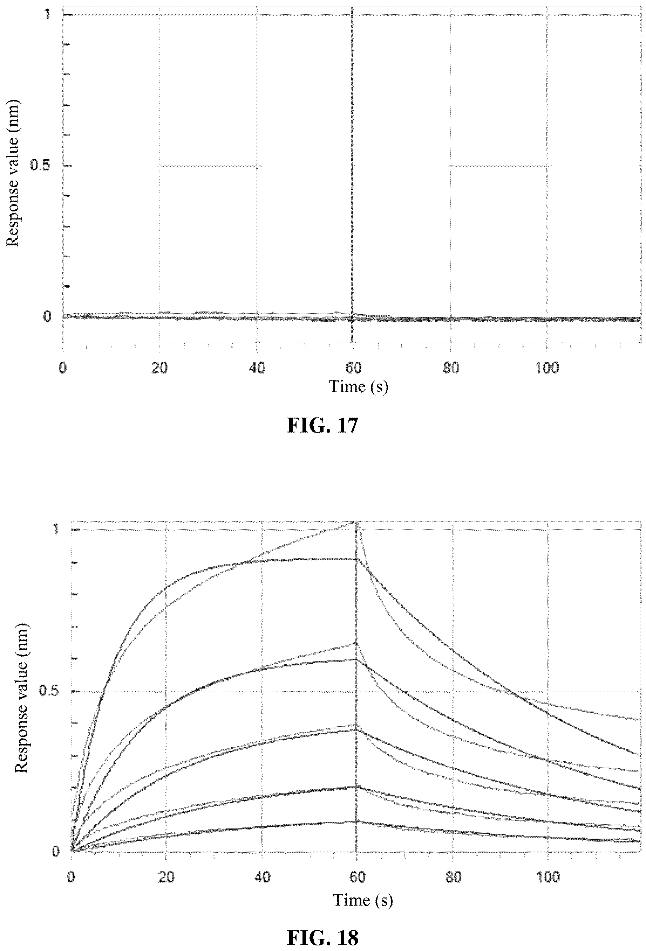

The affinity constant between FcγRIIa_H131 and TF01 is shown in Table 17 and FIGs. 17 to 18.

**Table 17: Kinetic parameters of TF01 binding to FcyRIIa H131**

| **Sample ID** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **3.90E-08** | **4.81E+05** | **1.60E+04** | **1.88E-02** | **2.82E-04** | **0.50-1.01** |

N/A means that there was no binding between the antibody and the antigen, or the binding signal was extremely weak, so the results were not analyzed, and no corresponding data were obtained.

The results show that 26B12H2L2(hG1WT) bound to FcγRIIa_H131 with an affinity constant of 3.90E-08 M, and as there was no binding between TF01 and FcγRIIa_H131 or the binding signal was extremely weak, the results were not analyzed, and no corresponding data were obtained.

The results show that the binding activity of TF01 to FcγRIIa_H131 was effectively eliminated.

### (2) Determination of affinity constant between FcγRIIa R131 and TF01

Fc receptor FcγRIIa_R131 (also known as CD32a_R131) can bind to the Fc fragment of an IgG antibody, playing a role in antibody-dependent cell-mediated cytotoxicity (ADCC). The ability of a therapeutic antibody to bind to an Fc receptor influences the safety and efficacy of the antibody. In this experiment, the affinity constant of TF01 for FcγRIIa_R131 was determined using a Fortebio Octet molecular interaction instrument to assess the abilities of the test antibodies to bind to the Fc receptor.

The experimental method of determining the affinity constant of TF01 for FcγRIIa_R131 using a Fortebio Octet molecular interaction instrument is briefly described below: The sample dilution buffer was PBS, 0.02% Tween-20, and 0.1% BSA, pH 7.4. 5 µg/mL FcγRIIa_R131 was immobilized to an NTA sensor at an immobilization height of about 1.0 nm, and the sensor was equilibrated in the buffer for 60 s. The FcγRIIa_R131 immobilized to the sensor was allowed to bind to each antibody (antibody concentration: 12.5-200 nM, serially diluted two-fold) for 60 s, and the antibody was allowed to dissociate in the buffer for 60 s. The sample plate shaking speed was 1000 rpm, the temperature was 30 °C, and the frequency was 5.0 Hz. The data were analyzed by 1:1 model fitting to obtain affinity constants.

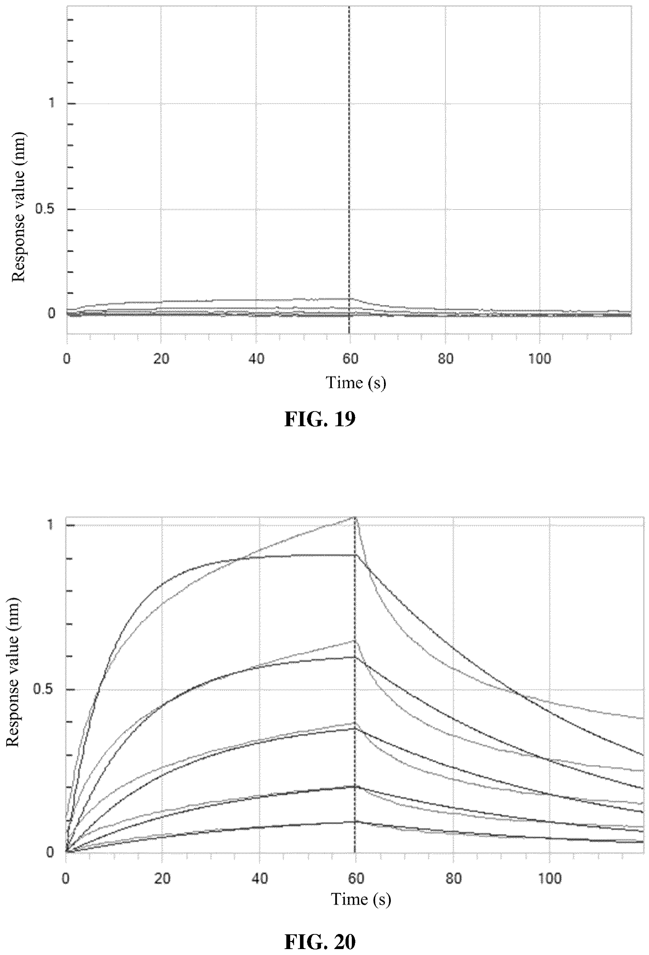

The affinity constant between FcγRIIa_R131 and TF01 is shown in Table 18 and FIGs. 19 to 20.

**Table 18: Kinetic parameters of TF01 binding to FcγRIIa_R131**

| **Antibody** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **3.22E-08** | **5.11E+05** | **1.38E+04** | **1.65E-02** | **2.14E-04** | **1.08-1.55** |

N/A means that there was no binding between the antibody and the antigen, or the binding signal was extremely weak, so the results were not analyzed, and no corresponding data were obtained.

The results show that 26B12H2L2(hG1WT) bound to FcγRIIa_H131 with an affinity constant of 3.22E-08 M, and as there was no binding between TF01 and FcγRIIa_R131 or the binding signal was extremely weak, the results were not analyzed, and no corresponding data were obtained.

The results show that the binding activity of TF01 to FcγRIIa_R131 was effectively eliminated.

### Example 15: Determination of Affinity Constant between FcγRIIb to TF01

Fc receptor FcyRIIb (also known as CD32b) can bind to the Fc fragment of an IgG antibody. In this experiment, the affinity constants of the test antibodies to FcyRIIb were determined using a Fortebio Octet molecular interaction instrument to assess the ability of TF01 to bind to the Fc receptor.

The experimental method of determining the affinity constant of TF01 for FcγRIIb using a Fortebio Octet molecular interaction instrument is briefly described below: The sample dilution buffer was PBS, 0.02% Tween-20, and 0.1% BSA, pH 7.4. 5 µg/mL FcyRIIb was immobilized to an NTA sensor at an immobilization height of about 1.0 nm, and the sensor was equilibrated in the buffer for 60 s. The hFCGR2B-his immobilized to the sensor was allowed to bind to each antibody (antibody concentration: 12.5-200 nM, serially diluted two-fold) for 60 s, and the antibody was allowed to dissociate in the buffer for 60 s. The sample plate shaking speed was 1000 rpm, the temperature was 30 °C, and the frequency was 5.0 Hz. The data were analyzed by 1:1 model fitting to obtain affinity constants.

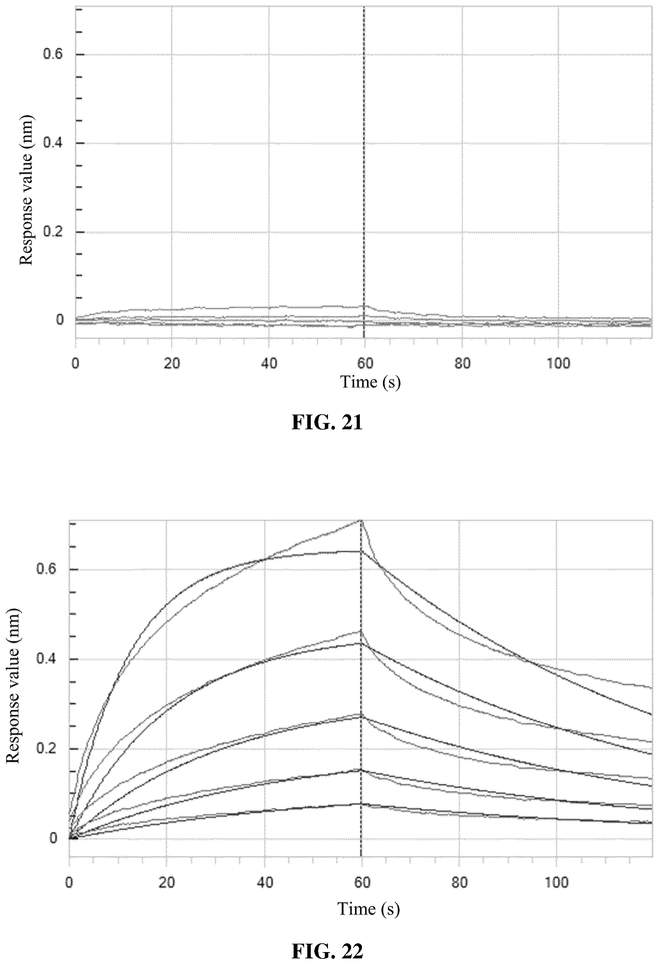

The affinity constant between FcγRIIb and TF01 is shown in Table 19 and FIGs. 21 to 22.

**Table 19: Kinetic parameters of TF01 binding to FcγRIIb**

| **Antibody** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **4.12E-08** | **3.42E+05** | **9.61E+03** | **1.41E-02** | **2.04E-04** | **0.50-0.78** |

N/A means that there was no binding between the antibody and the antigen, or the binding signal was extremely weak, so the results were not analyzed, and no corresponding data were obtained.

The results show that 26B12H2L2(hG1WT) bound to FcyRIIb with an affinity constant of 4.12E-08 M, and as there was no binding between TF01 and FcyRIIb or the binding signal was extremely weak, the results were not analyzed, and no corresponding data were obtained.

The results show that the binding activity of TF01 to FcyRIIb was effectively eliminated.

### Example 16: Determination of Affinity of TF01 for C1q

Serum complement C1q can bind to the Fc fragment of an IgG antibody, mediating a CDC effect. The ability of a therapeutic antibody to bind to C1q influences the safety and efficacy of the antibody. In this experiment, the affinity constant of TF01 for C1q was determined using a Fortebio Octet molecular interaction instrument to assess the CDC activity of these antibodies.

The experimental method of determining the affinity constant of the corresponding antibody for C1q using a Fortebio Octet molecular interaction instrument is briefly described below: The sample dilution buffer was PBS, 0.02% Tween-20, and 0.1% BSA, pH 7.4. An antibody at 50 µg/mL was immobilized to an FAB2G sensor at an immobilization height of about 2.0 nm, and the sensor was equilibrated in the buffer for 60 s. The antibody immobilized to the sensor was allowed to bind to antigen C1q (antigen concentration: 0.625-10 nM, serially diluted two-fold) for 60 s, and the antigen and the antibody were allowed to dissociate from each other in the buffer for 60 s. The sample plate shaking speed was 1000 rpm, the temperature was 30 °C, and the frequency was 5.0 Hz. The data were analyzed by 1:1 model fitting to obtain affinity constants. The data acquisition software was Fortebio Data Acquisition 7.0, and the data analysis software was Fortebio Data Analysis 7.0.

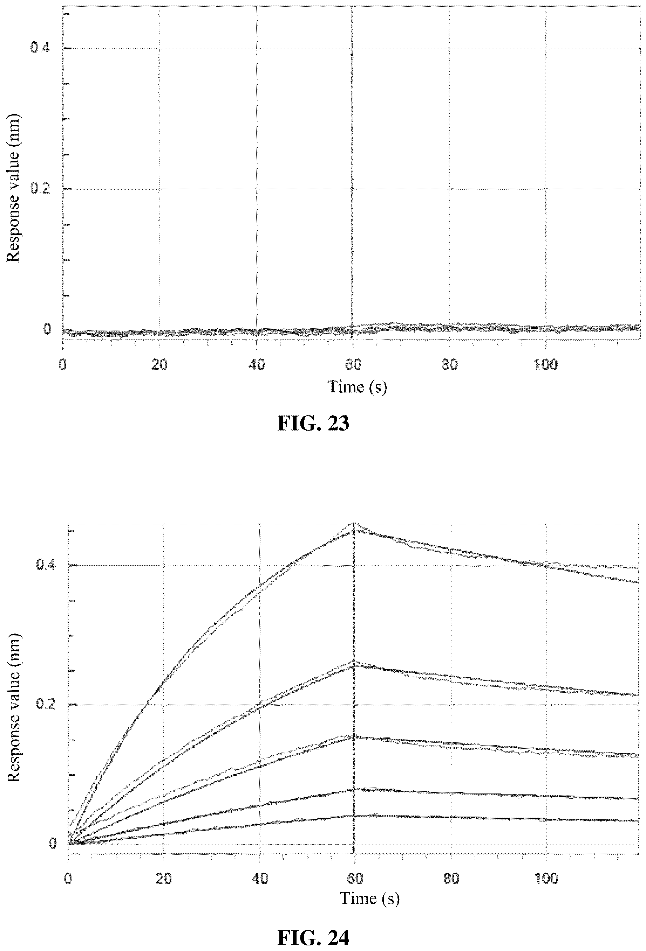

The affinity constant of TF01 for C1g is shown in Table 20 and FIGs. 23 to 24.

**Table 20: Kinetic parameters of TF01 binding to C1q**

| **Antibody** | **K_{D} (M)** | **kon (1/Ms)** | **SE (kon)** | **kdis (1/s)** | **SE (kdis)** | **Rmax (nm)** |
|---|---|---|---|---|---|---|
| **TF01** | **N/A** | **N/A** | **N/A** | **N/A** | **N/A** | **N/A** |
| **26B12H2L2 (hG1WT)** | **1.28E-09** | **2.40E+06** | **4.79E+04** | **3.05E-03** | **7.33E-05** | **0.53-0.63** |

N/A means that there was no binding between the antibody and the antigen, or the binding signal was extremely weak, so the results were not analyzed, and no corresponding data were obtained.

The results show that 26B12H2L2(hG1WT) bound to C1q with an affinity constant of 1.28E-09 M, and as there was no binding between TF01 and C1q or the binding signal was extremely weak, the results were not analyzed, and no corresponding data were obtained.

The results show that the binding activity of TF01 to C1q was effectively eliminated.

### Example 17: Antibody-Mediated Phagocytic Activity of TF01 Against CHO-K1-TIGIT Cells

The purpose of this example was to determine the antibody-dependent cellular phagocytosis (ADCP) activity. With murine macrophages as effector cells and a cell line overexpressing TIGIT as target cells, the ADCP effect mediated by the antibody was assessed.

Cryopreserved mouse bone marrow-derived macrophages (MBMMs) (derived from C57 mice) were added to DMEM + 10% FBS + 100 ng/mL M-CSF (macrophage colony stimulating factor, peprotech, Cat. No. 315-02), and the mixture was centrifuged at 1200× g for 5 min. The cells were collected, added to a DMEM + 10% FBS + 100 ng/mL M-CSF medium, and thawed overnight.

The target cells (CHO-K1-TIGIT) were collected, centrifuged at 170× g for 5 min, and washed once with PBS. The cells were counted using trypan blue. 5(6)-Carboxyfluorescein N-succinimidyl ester (CFSE, Biolegend, Cat. No. 423801) was diluted to 2.5 µM in PBS, and a proper amount of the CFSE dilution was taken to resuspend the cells (staining density: 1 × 10⁷ cells/mL). The cell suspension was incubated in an incubator for 20 min, and 6 mL of DMEM complete medium (containing 10% FBS) was added to stop the staining. The mixture was centrifuged at 170× g for 5 min, and the supernatant was discarded. 1 mL of DMEM complete medium was added, and the mixture was incubated in an incubator for 10 min. The antibody was diluted to the desired concentration in DMEM complete medium, and an isotype control antibody was designed. The target cells (1.5 × 10⁵ cells/well) were added to a 96-well V-bottom plate, and the antibodies (100 µL) were added. After thorough mixing, the plate was incubated on ice for 40 min and centrifugally washed twice (170× g, 5 min). The macrophages (MBMMs) were collected and centrifuged at 750× g for 5 min, and the supernatant was discarded. The cells were counted and resuspended in DMEM complete medium, and the concentration of macrophages was adjusted (5 × 10⁴ cells/100 µL). The cell suspension was added to the 96-well V-bottom plate containing the target cells. After resuspension and thorough mixing, the plate was incubated in an incubator at 37 °C for 2 h, and 100 µL of 1% PBSA (room temperature) was added to each well. The plate was centrifuged at 750× g for 5 min, and the supernatant was discarded. The cells were washed once with 200 µL of PBSA. An APC anti-mouse/human CD11b antibody (Biolegend, Cat. No. 101212) (500-fold diluted in PBSA) was added to the corresponding samples at 100 µL/well. After thorough mixing, the plate was incubated on ice for 40 min, and 100 µL of 1% PBSA was added to each well. The plate was centrifuged at 750× g for 5 min, and the supernatant was discarded. The cells in each well were washed once with 200 µL of PBSA, and 200 µL of 1% PBSA was added to each tube to resuspend the cells. Then the cells were analyzed using an instrument. In the analysis system, macrophages were positive for APC, and macrophages that performed phagocytosis were double positive for APC and CFSE. The phagocytosis rate was expressed in terms of the proportion of the number of double positive cells to the number of APC-positive cells, and the antibody-mediated ADCP activity was assessed based on the phagocytosis rate. The ADCP activity of each group, expressed as P%, was calculated using the following formula: P% = the number of macrophages that performed phagocytosis/the total number of macrophages × 100%

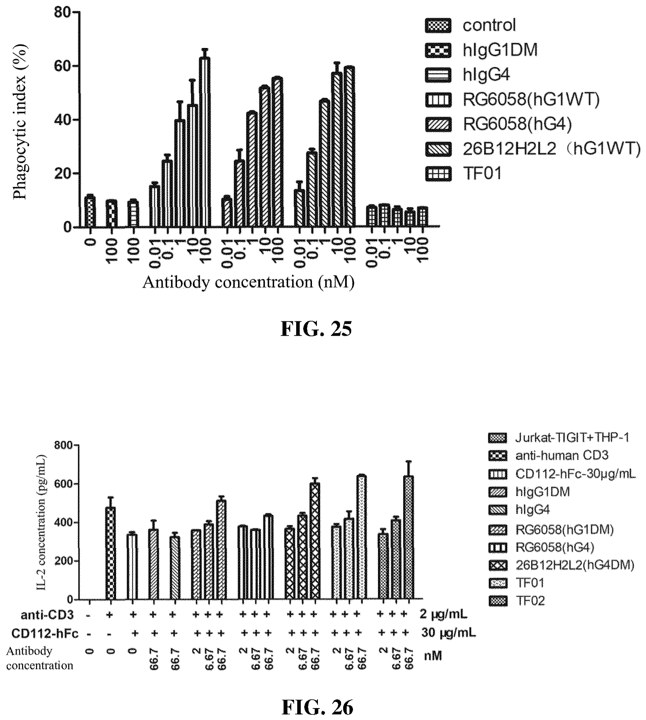

The results are shown in FIG. 25.

The results show that, at the same concentration, RG6058(hG1WT), RG6058(hG4), and 26B12H2L2(hG1WT) exhibited ADCP effects; at the same concentration, the TF01 group was comparable in phagocytosis rate to the isotype control antibody group, indicating that TF01 exhibited no ADCP effect.

The results show that the amino acid mutations introduced into TF01 effectively eliminated its ADCP effect.

### Example 18: Assessments of Blocking Activity of Anti-TIGIT Antibody-TGF-βR Fusion Proteins Against Inhibition of IL-2 Secretion in Co-Culture System by CD112/CD155

### 1. Assessment of blocking activity of anti-TIGIT antibody-TGF-βR fusion proteins against inhibition of IL-2 secretion in co-culture system of Jurkat-TIGIT and THP-1 cells by CD112

A 96-well plate (Corning, Model No. 3599) was coated with 2 µg/mL anti-human CD3 antibody (prepared by Akeso Biopharma Inc., Lot No. 20170830) at 37 °C for 2 h. The coating solution was removed, and the plate was washed once with pre-cooled PBS (300 µL). Jurkat-TIGIT cells (constructed by Akeso Biopharma Inc.) were counted and seeded into the 96-well plate at 5 × 10⁴ cells/well. The antibodies were added according to the experimental design, and the plate was incubated at 37 °C for 30 min. CD112-hFc (prepared by Akeso Biopharma Inc., Lot No. 20180209) was added according to the experimental design. THP-1 cells (purchased from Chinese Academy of Sciences, Cat. No. 3131C0001000700057) were counted and seeded into the 96-well plate at 5 × 10⁴ cells/well. The plate was incubated in an incubator for 48 h. The culture supernatant was collected, and the IL-2 content was determined using an IL-2 ELISA kit (Dakewe, Cat. No. 1110202).

The results are shown in FIG. 26. The results show that CD112 exhibited a significant inhibitory effect on IL-2 secretion. Antibodies 26B12H2L2(hG4DM), TF01, TF02, RG6058(hG1DM), and RG6058(hG4) all effectively blocked the inhibition of IL-2 secretion in the system by CD112, and TF01 and TF02 were superior in activity to positive control antibodies RG6058(hG1DM) and RG6058(hG4).

### 2. Assessment of blocking activity of anti-TIGIT antibody-TGF-βR fusion proteins against inhibition of IL-2 secretion in co-culture system of Jurkat-TIGIT and THP-1 cells by CD155

A 96-well plate (Corning, Model No. 3599) was coated with 2 µg/mL anti-human CD3 antibody (prepared by Akeso Biopharma Inc., Lot No. 20170830) at 37 °C for 2 h. The coating solution was removed, and the plate was washed once with pre-cooled PBS (300 µL). Jurkat-TIGIT cells (constructed by Akeso Biopharma Inc.) were counted and seeded into the 96-well plate at 5 × 10⁴ cells/well. The antibodies were added according to the experimental design, and the plate was incubated at 37 °C for 30 min. CD155-hFc (prepared by Akeso Biopharma Inc., Lot No. 20180209) was added according to the experimental design. THP-1 cells (purchased from Chinese Academy of Sciences, Cat. No. 3131C0001000700057) were counted and seeded into the 96-well plate at 5 × 10⁴ cells/well. The plate was incubated in an incubator for 48 h. The culture supernatant was collected, and the IL-2 content was determined using an IL-2 ELISA kit (Dakewe, Cat. No. 1110202).

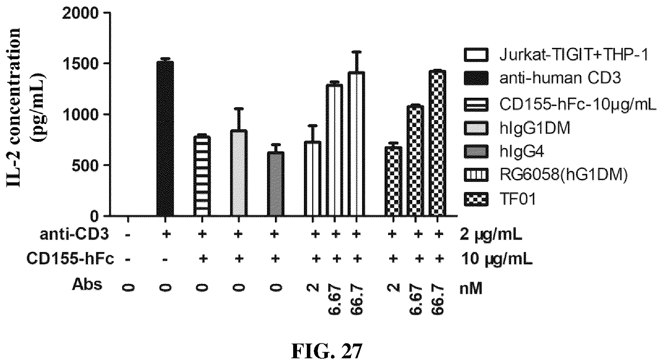

The results are shown in FIG. 27. The results show that CD155 exhibited a significant inhibitory effect on IL-2 secretion. Antibodies TF01 and RG6058(hG1DM) both effectively blocked the inhibition of IL-2 secretion in the system by CD155, and they were comparable in activity.

### Example 19: Assessment of IL-2 Secretion Promoting Bioactivity of Anti-TIGIT Antibody-TGF-βR Fusion Proteins in Co-Culture System of Jurkat-TIGIT and HT1080-aCD3scFv Cells

Jurkat-TIGIT cells (constructed by Akeso Biopharma Inc.) and HT1080-aCD3scFv cells (constructed by Akeso Biopharma Inc.) in the logarithmic growth phase were collected, counted, and plated, with 5 × 10⁴ Jurkat-TIGIT cells in each well and 1 × 10⁴ HT1080-aCD3scFv cells in each well. Diluted antibodies (antibody gradients: 3 nM, 30 nM, and 300 nM) were added, and a soluble anti-human CD28 antibody (3 µg/mL) (R&D, Cat. No. MAB342-500) was added. The plate was incubated in an incubator for 48 h. The culture supernatant was collected, and the IL-2 content was determined using an IL-2 ELISA kit (Dakewe, Cat. No. 1110202).

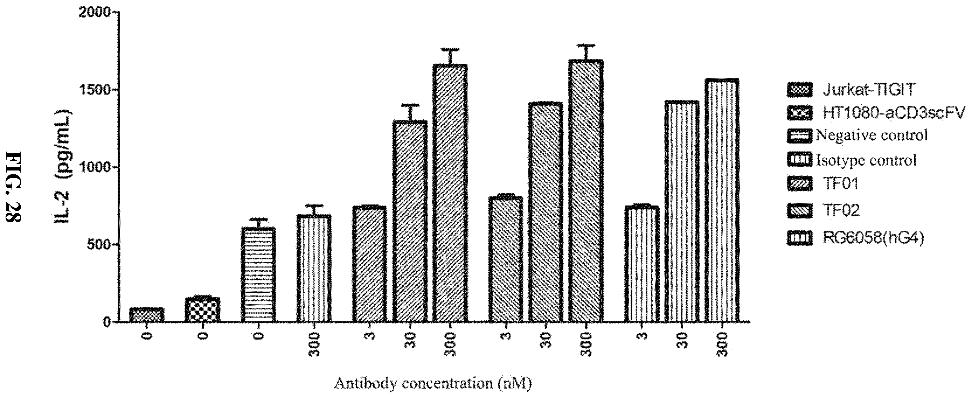

The results are shown in FIG. 28.

The results show that antibodies TF01, TF02, and RG6058(hG4) all effectively promoted IL-2 secretion in the co-culture system of Jurkat-TIGIT and HT1080-aCD3scFv cells, and they were comparable in activity.

### Example 20: Assessment of Blocking Activity of Anti-TIGIT Antibody-TGF-βR Fusion Protein Against Inhibition of IFN-γ Secretion by TGF-β1 During Secondary Immune Response of PBMCs to CMV Antigen

PBMCs (from a healthy donor) were thawed, seeded into a complete medium (RPMI 1640 + 10% FBS), and incubated overnight in an incubator. The next day, cells were collected, counted, analyzed for viability, and seeded into a 96-well round-bottom plate (Corning, Cat. No. 3799) at 2 × 10⁵ cells/well. Meanwhile, serially diluted antibodies, CMV (final concentration: 0.02 µg/mL, Mabtech, Cat. No. 3619-1), and TGF-β1 (final concentration: 3 ng/mL, Genscript, Cat. No. Z03411) were added according to the experimental design, with a final volume of 200 µL. (TGF-β1 and the antibodies were incubated at 37 °C for 10 min). The plate was incubated in an incubator for 4 days. After the 4 days, the cell culture supernatant was collected, and the IFN-γ content was determined using an IFN-y ELISA kit (Dakewe, Cat. No. 1110002).

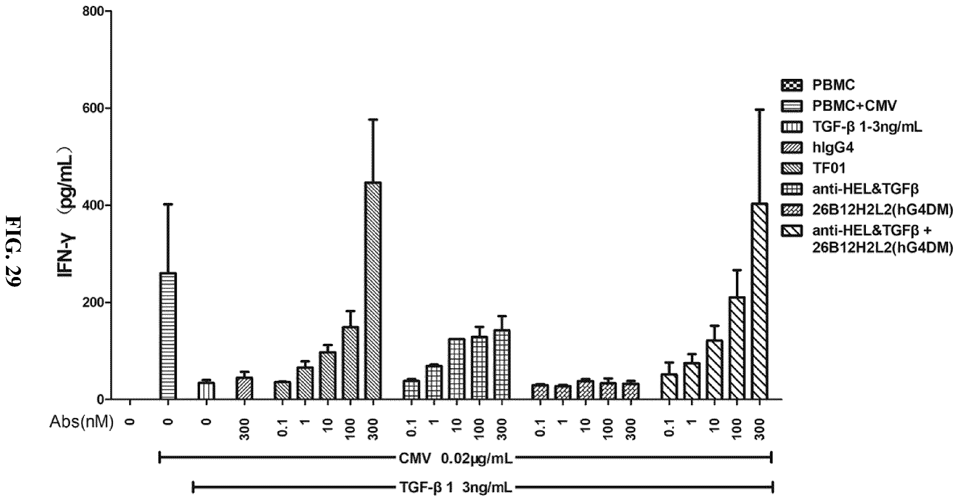

The results are shown in FIG. 29. The results show that the CMV antigen caused a secondary immune response in PBMCs, leading to IFN-**γ** secretion, and TGF-β1 exhibited a significant inhibitory effect on IFN-γ secretion in the system. Antibodies TF01 and anti-HEL&TGFβ and the combination of anti-HEL&TGFβ + 26B12H2L2(hG1DM) all effectively blocked the inhibition of IFN-γ secretion in the system by TGF-β1, whereas 26B12H2L2(hG1DM) alone did not. The blocking activity of TF01 was superior to that of control antibody anti-HEL&TGFβ and comparable to that of the combination of anti-HEL&TGFβ + 26B12H2L2(hG1DM).

### Example 21: Pharmacodynamic Assessment of Anti-TIGIT Antibody-TGF-βR Fusion Protein in Mouse Tumor Cell Subcutaneous Xenograft Model

The purpose of this example was to determine the *in vivo* anti-tumor activity of an anti-TIGIT antibody-TGF-βR fusion protein. First, MDA-MB-231 cells (purchased from ATCC) were inoculated subcutaneously into the mammary fat pad of 6.71 to 9.57-week-old female Scid Beige mice (purchased from Beijing Vital River Laboratory Animal Technology Co., Ltd.). On day 17 after the inoculation, the mice were randomized into 3 groups of 8 according to tumor volume. Each mouse was intraperitoneally injected with human peripheral blood mononuclear cells (hPBMCs) and dosed, and the day of grouping was defined as D0. The route of administration was intraperitoneal injection. The mice were dosed once weekly for 6 weeks. hPBMCs were intraperitoneally injected on day 8 after the grouping. The modeling and the specific route of administration are shown in Table 21. After the administration, the length and width of the tumors in each group were measured, and the tumor volume was calculated.

**Table 21: The dosing regimen of the anti-TIGIT antibody-TGF-βR fusion protein for treating the Scid Beige mouse MDA-MB-231 xenograft tumor model**

| **Grouping** | **Number of animals** | **Inoculation** | **Administration** |
|---|---|---|---|
| **Isotype control 30 mg/kg** | **8** | **MDA-MB-231, 2 × 10⁶ cells, Scid Beige mice, subcutaneous inoculation into the mammary fat pad; the mice were divided into groups on day 17 after the cell inoculation and intraperitoneally injected with 2.5 × 10⁶ hPBMCs; the mice were intraperitoneally injected with 3 × 10⁶ hPBMCs on day 8 after the grouping.** | **Anti-HEL(hIgG4) (produced by Akeso Biopharma Inc., Lot No. 20190910), 30 mg/kg; intraperitoneally injected once weekly for 6 consecutive weeks** |
| **TF01 3.6 mg/kg** | **8** | | **TF01 (produced by Akeso Biopharma Inc., Lot No. A130Y20210301), 3.6 mg/kg; intraperitoneally injected once weekly for 6 consecutive weeks** |
| **TF01 36 mg/kg** | **8** | | **TF01 (produced by Akeso Biopharma Inc., Lot No. A130Y20210301), 36 mg/kg; intraperitoneally injected once weekly for 6 consecutive weeks** |

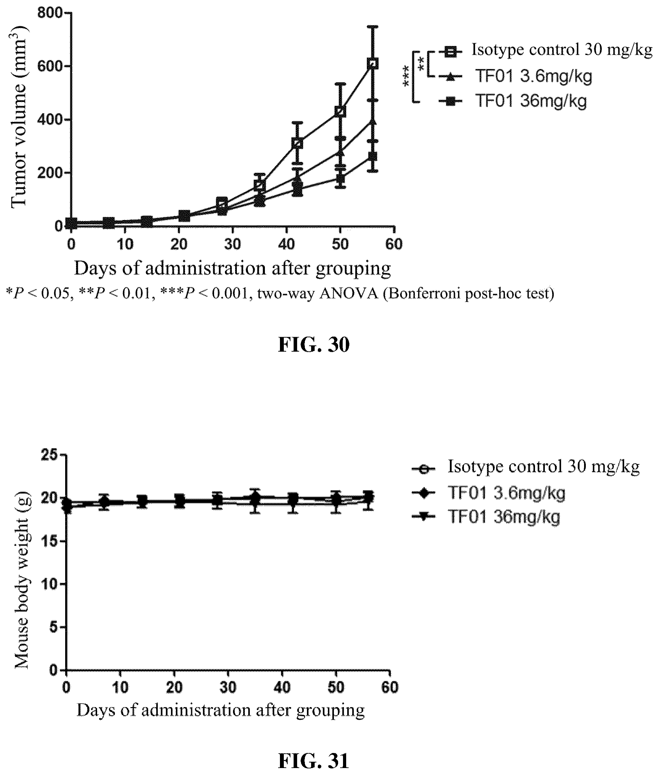

The results are shown in FIG. 30. The results show that, compared to the isotype control antibody, anti-TIGIT antibody-TGF-βR fusion protein TF01 effectively inhibited tumor growth in mice.

In addition, as shown in FIG. 31, the tumor-bearing mice exhibited good tolerance to test drug TF01, and each group had no effect on the body weight of the tumor-bearing mice.

### Example 22: Assessment of IL-2 Secretion Promoting Bioactivity of Anti-TIGIT Antibody-TGF-βR Fusion Proteins in Co-Culture System of Jurkat-TIGIT and HT1080-aCD3scFv Cells

Jurkat-TIGIT cells (constructed by Akeso Biopharma Inc.) and HT1080-aCD3scFv cells (constructed by Akeso Biopharma Inc.) in the logarithmic growth phase were collected, counted, and plated, with 5 × 10⁴ Jurkat-TIGIT cells in each well and 1 × 10⁴ HT1080-aCD3scFv cells in each well. Diluted antibodies (final antibody concentrations: 3 nM, 30 nM, and 300 nM) were added, and a soluble anti-human CD28 antibody (3 µg/mL) (R&D, Cat. No. MAB342-500) was added. The plate was incubated in an incubator for 48 h. The culture supernatant was collected, and the IL-2 content was determined using an IL-2 ELISA kit (Dakewe, Cat. No. 1110202).

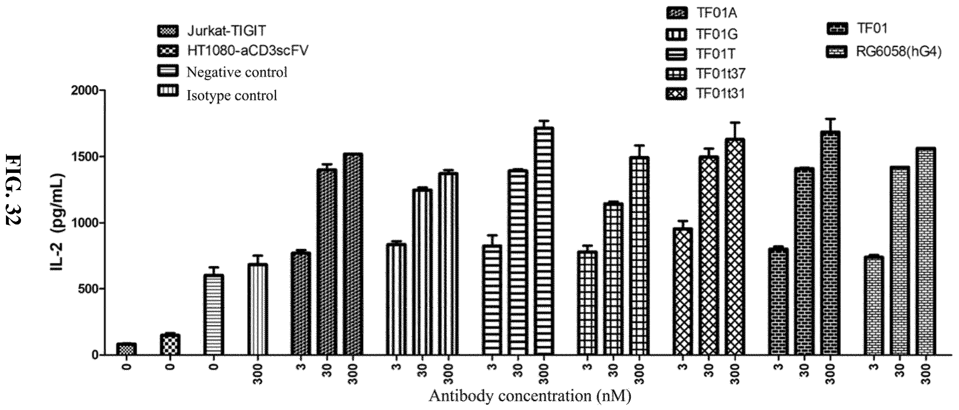

The results are shown in FIG. 32.

The results show that antibodies TF01A, TF01G, TF01T, TF01t31, and TF01t37 all effectively promoted IL-2 secretion in the co-culture system of Jurkat-TIGIT and HT1080-aCD3scFv cells, and they were comparable in activity to TF01.

### Example 23: Neutralizing Effects of Anti-TIGIT Antibody-TGF-βR Fusion Proteins on Inhibition of IL-4-Induced TF-1 Cell Proliferation by TGF-β

TF-1 cells (purchased from ATCC, Cat. No. CRL-2003) were collected, centrifuged at 110× g for 5 min, washed twice with an analysis medium (1640 + 10% FBS + 2.5 g/L glucose), resuspended, and counted, and the cell suspension (2 × 10⁴/100 µL) was added to a 96-well round-bottom plate. IL-4 (final concentration: 0.1 nM) (purchased from Peprotech, Cat. No. 200-04), TGF-β1(final concentration: 3 ng/mL) (purchased from Genscript, Cat. No. Z03411), TGF-β3 (final concentration: 3 ng/mL) (purchased from Peprotech, Cat. No. 100-36E), and the antibodies (final concentrations: 1000 nM, 100 nM, 10 nM, 1 nM, 0.1 nM, and 0.01 nM) were diluted according to the experimental design. An equal volume of antibody and TGF-β1/3 was added to each well of an 8-tube strip, and an isotype control and a blank control were designed. The strip was incubated at room temperature for 30 min. Then the pre-incubated solutions of antibodies at corresponding concentrations with TGF-β1 and TGF-β3 were added to the 96-well plate at 50 µL/well, followed by 50 µL of 0.4 nM IL-4. After thorough mixing, the plate was incubated in an incubator at 37 °C with 5% CO₂ for 72 h. After the 72 h, CCK-8 (purchased from Dojindo Laboratories, Japan, Cat. No. CK04) was added at 20 µL/well, and the plate was incubated in an incubator at 37 °C with 5% CO₂ for 4 h (color change was observed during the incubation). After thorough mixing, 150 µL of the mixture was transferred to a clean 96-well flat-bottom plate, and the OD at 450 nm was measured using a microplate reader.

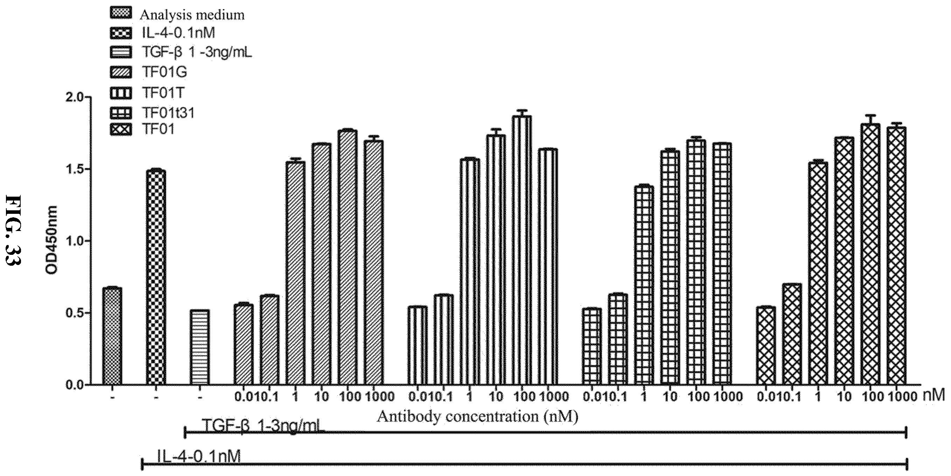

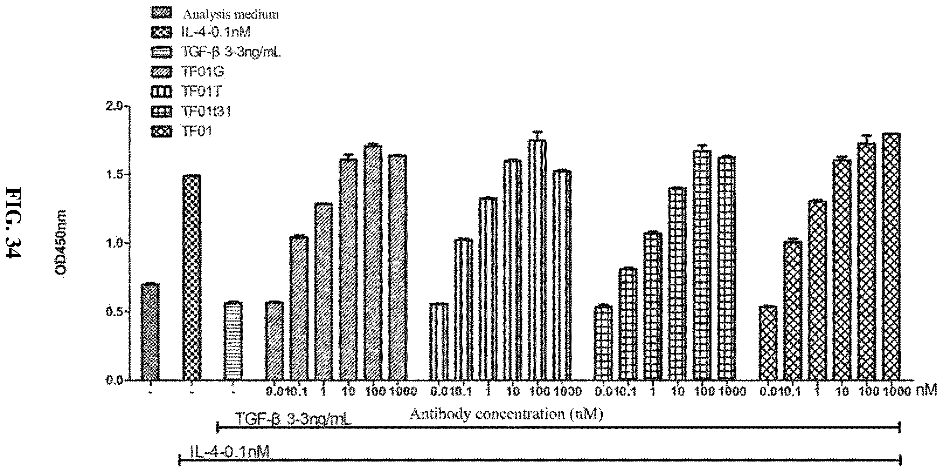

The results are shown in FIGs. 33 and 34.

The results show that antibodies TF01G, TF01T, TF01t31, and TF01 all effectively neutralized the inhibition of IL-4-induced TF-1 cell proliferation by TGF-β1 and TGF-β3, and they were comparable in activity.

### Example 24: Reporter Gene Experiment of Anti-TIGIT Antibody-TGF-βR Fusion Proteins Blocking Interaction Between TGF-β1/3 and TGF-βR

According to the experimental design, the antibodies and TGF-β1/TGF-β3 (prepared at a concentration of 4 ng/mL; final concentration: 1 ng/mL) (TGF-β1 was purchased from Genscript, Cat. No. Z03411; TGF-β3 was purchased from peprotech, Cat. No. 100-36E) were separately diluted in DMEM + 10% FBS medium. 25 µL of antibody and 25 µL of TGF-β1/TGF-β3 were added to each well of a 96-well black plate, and an isotype control and a blank control were designed. The plate was incubated in a cell incubator at 37 °C with 5% CO₂ for 30 min. 293T-TGFβR2-Luc cells (constructed by Akeso Biopharma Inc.) were collected, resuspended in a DMEM + 10% FBS medium, counted, and analyzed for viability, and 5 × 10⁴ cells/50 µL of the cell suspension was added to each well of a 96-well plate. The plate was incubated overnight in a cell incubator at 37 °C with 5% CO₂, and 50 µL of Bright-Glo^{™} Luciferase Assay Reagent (purchased from Promega, Cat. No. E2620) was added. The plate was incubated at room temperature for 30 min, and RLU (relative light units) values were read using an Envision multi-label microplate tester.

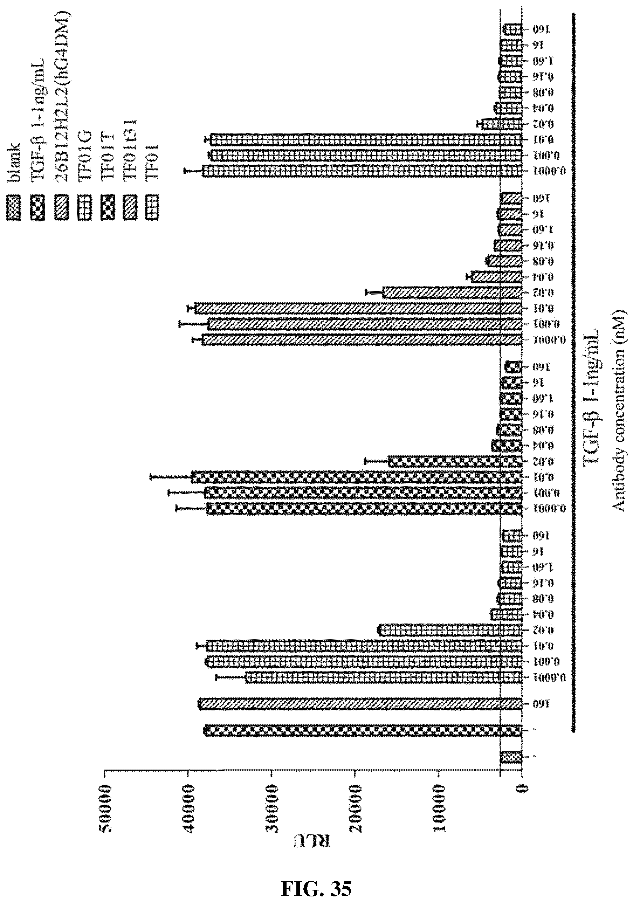

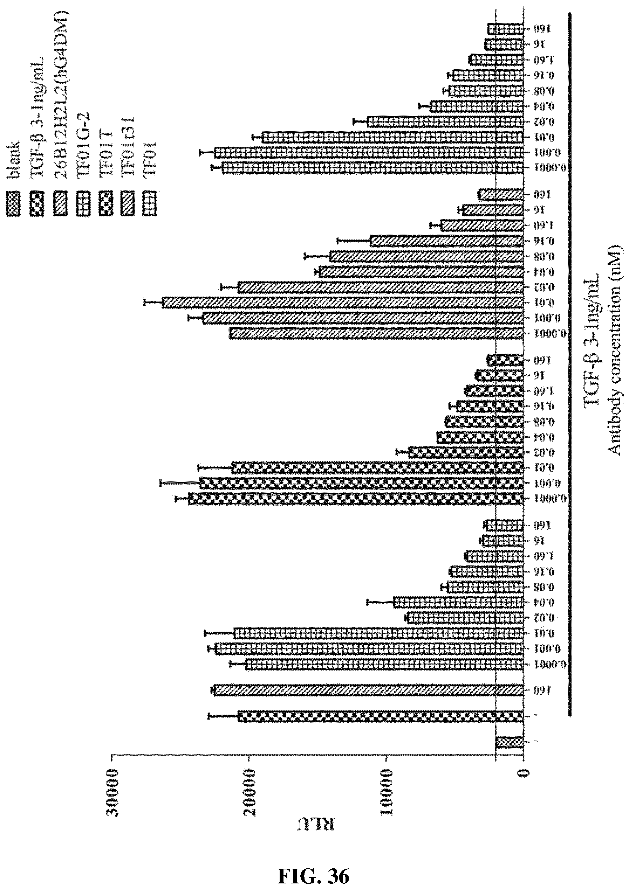

The results are shown in Table 22 and FIGs. 35 to 36.

**Table 22**

| **/** | **Factor** | **TF01G** | **TF01T** | **TF01t31** | **TF01** |
|---|---|---|---|---|---|
| **EC₅₀ (nM)** | **TGF-β1** | **0.0197** | **0.0194** | **0.0189** | **0.0153** |
| **EC₅₀ (nM)** | **TGF-β3** | **0.0163** | **0.0149** | **0.0777** | **0.0182** |

The results show that antibodies TF01G, TF01T, and TF01t31 all effectively blocked the interaction between TGF-β1/3 and TGF-βR2, and they were comparable in activity to TF01.

### Example 25: Reporter Gene Experiment of Anti-TIGIT Antibody-TGF-βR Fusion Proteins Blocking Interaction Between TIGIT and PVR

CHO-aAPC-PDL1-PVR cells (constructed by Akeso Biopharma Inc.) were seeded into a 96-well black flat-bottom plate (Corning, Model No. 3916) at 2 × 10⁴ cells/well and cultured overnight (100 µL/well) (the medium was a growth medium for the cells: Ham F-12 + 10% FBS). The next day, the medium in the plate was removed, and Jurkat-TIGIT-NFAT-Luc cells (constructed by Akeso Biopharma Inc.) were added at 1 × 10⁵ cells/well (50 µL/well) (medium: 1640 + 10% FBS). The antibodies (final concentration: 0.037 nM, 0.37 nM, 3.7 nM, 11.1 nM, 33.3 nM, 100 nM, 300 nM, 1000 nM, or 3000 nM) were added at 30 µL/well, and an isotype control group and a negative control group were set, with a final volume of 80 µL/well. The plate was incubated in an incubator for 4.5 h and then taken out and equilibrated to room temperature, and Bright-Glo^{™} Luciferase Assay System (purchased from Promega, Cat. No. E2650) was added at 80 µL/well. After the plate was incubated in a dark place for 2 min, RLU values were read.

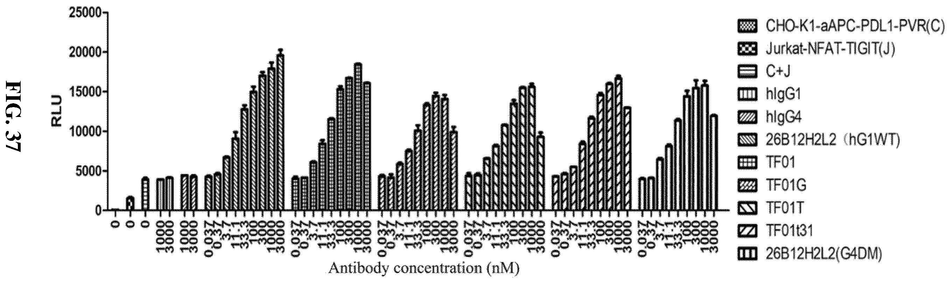

The results are shown in FIG. 37.

The results show that antibodies TF01G, TF01T, and TF01t31 all effectively blocked the interaction between TIGIT and PVR, activated the NFAT pathway of effector cells, and activated the expression of the luciferase reporter gene, and they were comparable in activity to TF01, 26B12H2L2(hG1WT), and 26B12H2L2(hG4DM).

### Example 26: Molecular Weight Analysis of Anti-TIGIT Antibody-TGF-βR Fusion Proteins

An antibody was diluted to 1 mg/mL in 50 mM ammonium bicarbonate (pH 7.8), and 50 mL of the diluted sample was taken and cleaved with 2 µL of PNGase-F (purchased from: NEB, Cat. No. P0705L) for 1 h. 10 µL of the cleaved sample was taken, and 40 µL of 6 M guanidine hydrochloride (purchased from: Sigma, Cat. No. V900385-500G) and 5 µL of 0.5 M TCEP (tris(2-carboxyethyl)phosphine, purchased from: Thermo Fisher, Cat. No. 77720) were added. After thorough mixing, the mixture was denatured and reduced at room temperature for 30 min. After C4 column separation, mass spectrometry analysis was performed.

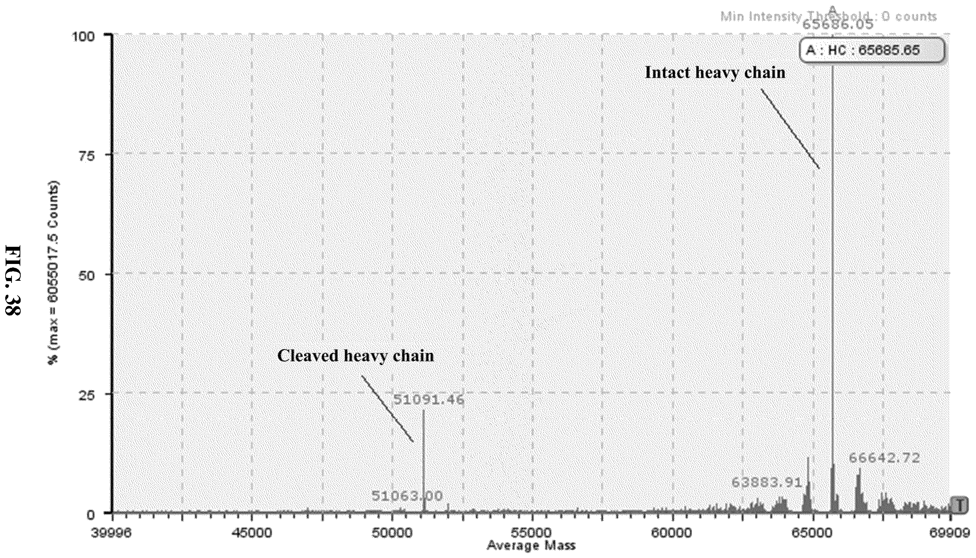

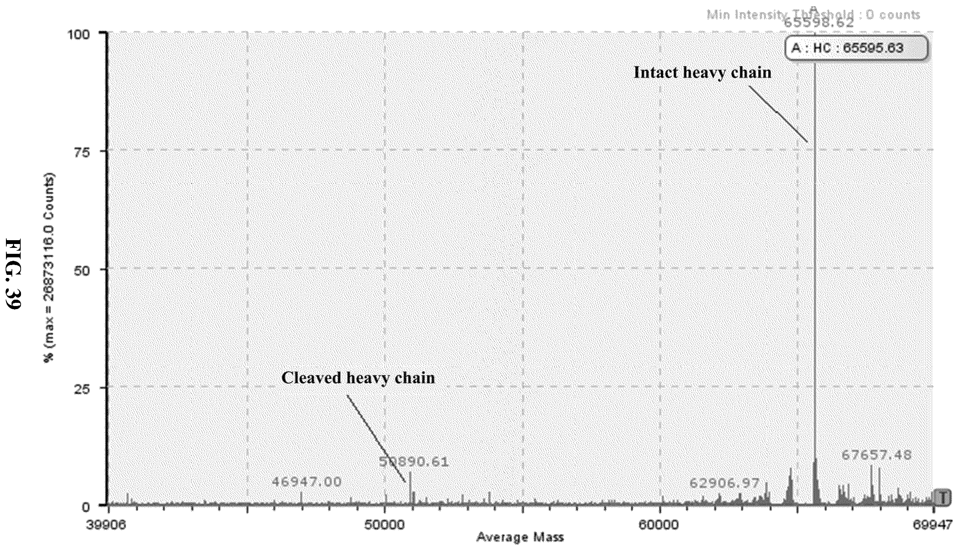

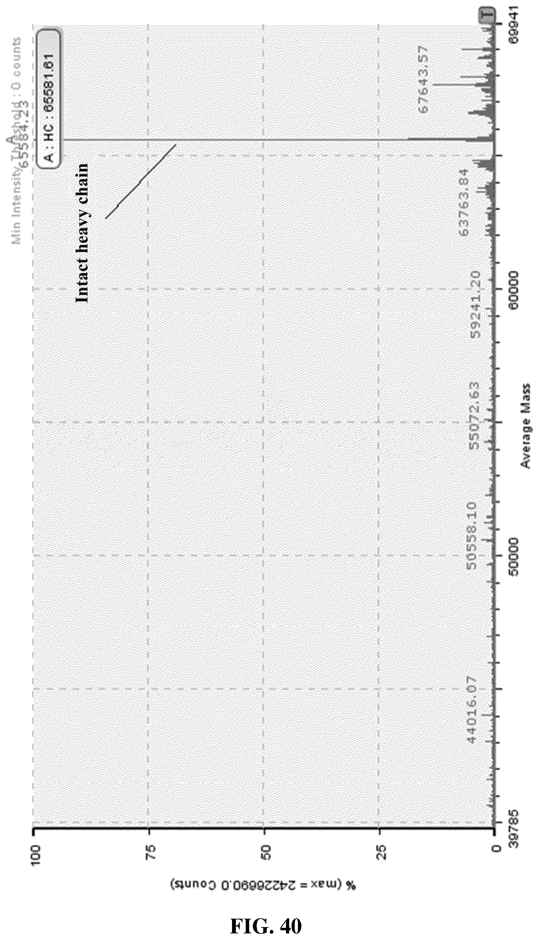

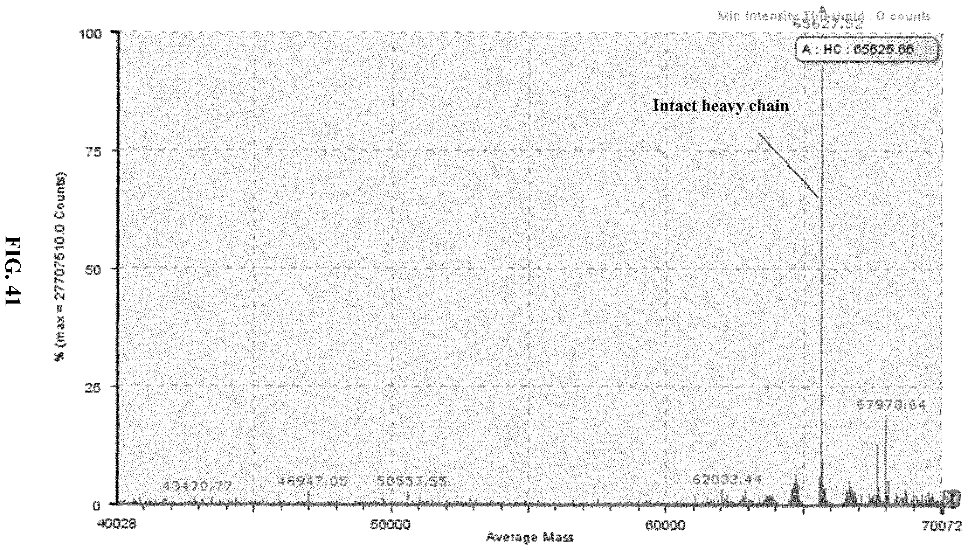

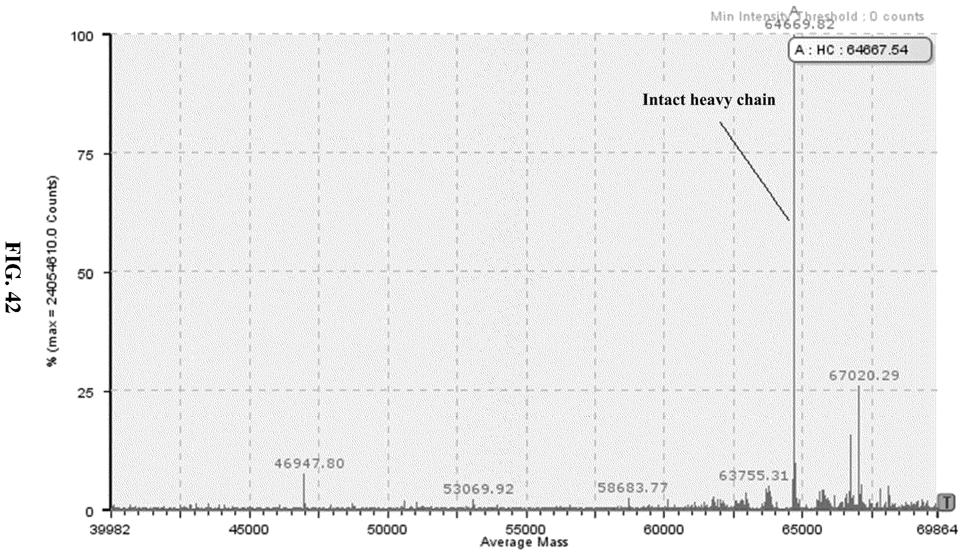

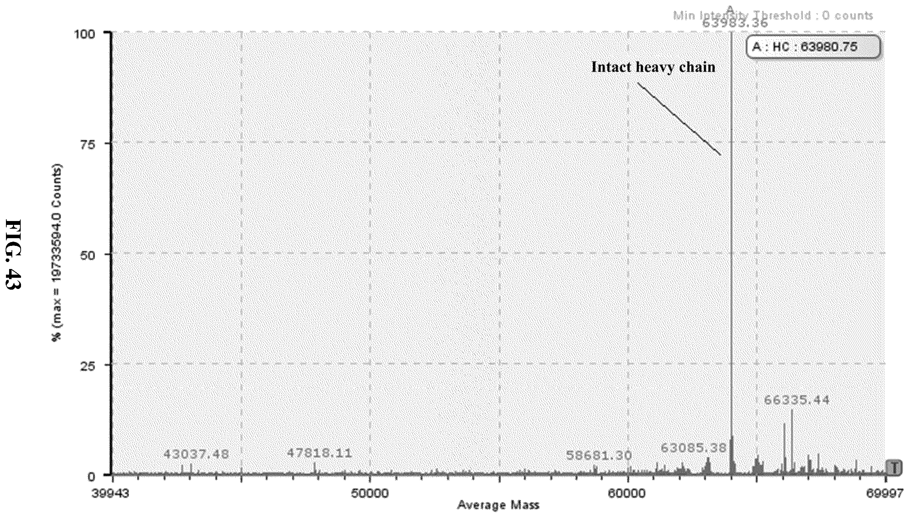

The results are shown in FIGs. 38 to 43.

The results show that: the heavy chain of antibody TF01 was cleaved, with the molecular weight reduced from 65.7 KD to 51.1 KD, while the heavy chain of antibody TF01A was cleaved to a small extent, and the heavy chains of TF01G, TF01T, TF01t31, and TF01t37 were not cleaved.

### Example 27: SEC-HPLC Analysis of Anti-TIGIT Antibody-TGF-βR Fusion Proteins

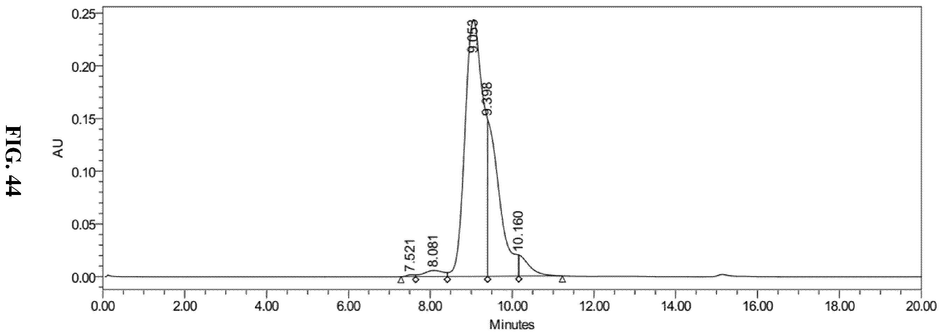

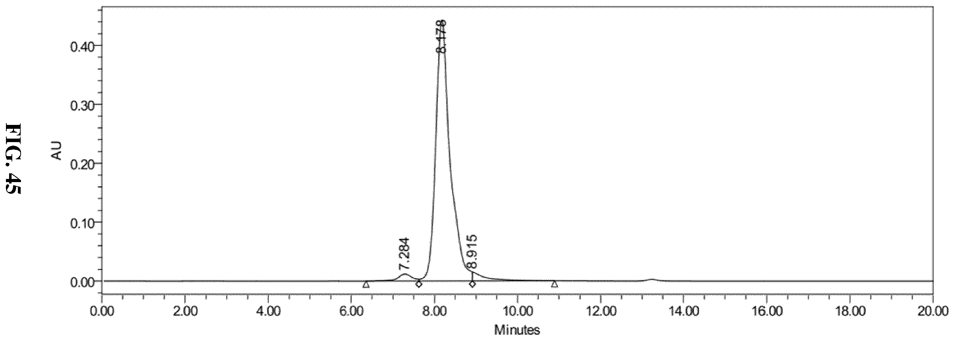

TF01 and TF01T protein samples were analyzed by SEC-HPLC using a UV detector (detection wavelength: 280 nm; mobile phase: 25 mM disodium hydrogen phosphate dodecahydrate, 25 mM anhydrous sodium dihydrogen phosphate, 300 mM sodium chloride, pH 6.5; flow rate: 0.8 mL/min; injection volume: 100 µg; run time: 20 min of isocratic elution). The results are shown in Table 23 and FIGs. 44 to 45.

**Table 23: SEC-HPLC purity analysis of the anti-TIGIT antibody-TGF-βR fusion proteins**

| **/** | **SEC-HPLC (%)** | | | | |
|---|---|---|---|---|---|
| **/** | **Peak 1** | **Peak 2** | **Peak 3** | **Peak 4** | **Peak 5** |
| **TF01** | **0.2** | **1.7** | **67.3** | **27.5** | **3.3** |
| **TF01T** | **2.6** | **94.4** | **3.0** | **/** | **/** |

The results show that TF01 exhibited significant degradation fragment characteristic peaks, whereas the engineered mutant TF01T overcame the degradation problem: the degradation fragment characteristic peaks disappeared, and the HPLC main peak purity significantly increased.

### Example 28: Determination of Binding Activity of Anti-TIGIT Antibody-TGF-βR Fusion Proteins to TGF-β by ELISA

An ELISA plate was coated with 1 µg/mL TGF-β1 and incubated overnight at 4 °C. Then the antigen-coated ELISA plate was washed once with PBST and blocked with a PBS solution containing 1% BSA (blocking solution) at 37 °C for 2 h. After the blocking, the ELISA plate was washed 3 times with PBST. The antibodies and TGF-βRII-mFc, serially diluted in PBST solution, were added. The ELISA plate to which the test antibodies and TGF-βRII-mFc were added was incubated at 37 °C for 30 min and then washed 3 times with PBST. After the washing, HRP-labeled goat anti-human IgG (H+L) (Jackson, Cat. No. 109-035-098) and HRP-labeled goat anti-mouse IgG Fc (Jackson, Cat. No. 115-035-071) secondary antibody working solutions diluted at a ratio of 1:5000 were added, and the plate was then incubated at 37 °C for 30 min. After the incubation, the plate was washed 4 times with PBST. Color development was then performed with TMB (Neogen, 308177) in a dark place for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

The results are shown in the table.

**Table 24: The detection of the binding of TF01, TF01T, and TGF-βRII-mFc to TGF-β1 by ELISA**

| **Antibody dilution gradient (µg/mL)** | **TGF-β1 (1 µg/mL), 50 µL/well** | | | | | |
|---|---|---|---|---|---|---|
| | **TF01T** | | **TF01** | | **TGF-β RII-mFc** | |
| **1.000** | **3.014** | **3.182** | **3.166** | **3.131** | **2.702** | **2.542** |
| **0.333** | **3.062** | **3.212** | **3.208** | **3.213** | **2.705** | **2.703** |
| **0.111** | **2.971** | **3.111** | **3.068** | **3.115** | **2.565** | **2.536** |
| **0.037** | **2.861** | **2.285** | **1.979** | **2.091** | **1.894** | **1.929** |
| **0.012** | **1.701** | **0.677** | **0.541** | **0.587** | **0.601** | **0.716** |
| **0.004** | **0.580** | **0.245** | **0.212** | **0.222** | **0.172** | **0.177** |
| **0.001** | **0.057** | **0.139** | **0.126** | **0.136** | **0.091** | **0.098** |
| **0.000** | **0.289** | **0.099** | **0.095** | **0.103** | **0.056** | **0.052** |
| **Secondary antibody** | **Goat anti-human** | | | | **Goat anti-mouse** | |
| | **IgG (H+L), HRP (1:5000), 50 µL/well** | | | | **IgG mFC, HRP (1:5000), 50 µL/well** | |
| **EC₅₀ (nM)** | **0.100** | | **0.164** | | **0.271** | |

By quantitative absorbance analysis of the antibodies that bound, curve fitting, and calculations, the binding efficiency EC₅₀ values of TF01T, TF01, and TGF-βRII-mFc (as a positive control) to TGF-β1 were obtained, and they were 0.100 nM, 0.164 nM, and 0.271 nM, respectively.

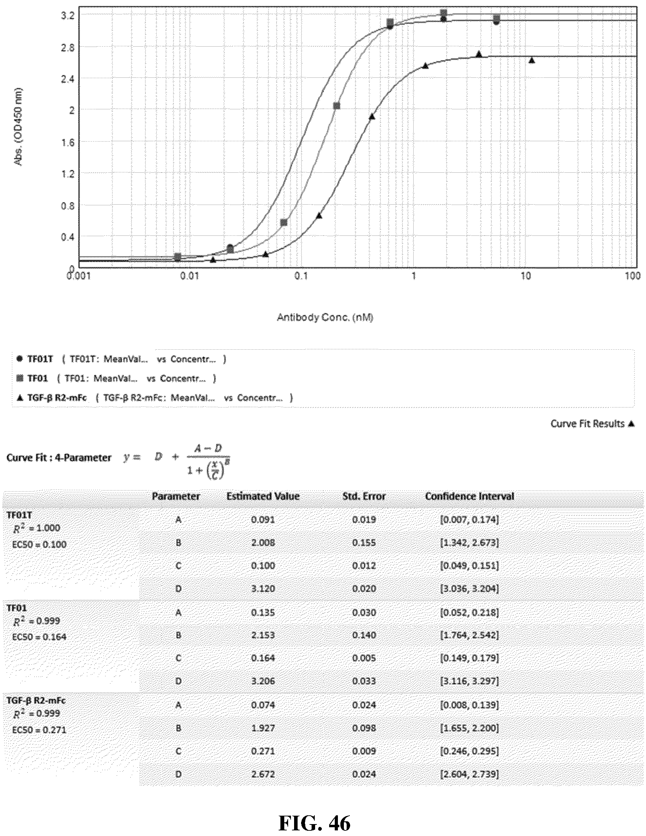

The results are shown in FIG. 46.

The results show that TF01T and TF01 had the activity of effectively binding to TGF-β1 in a dose-dependent manner. The results also show that TF01T, TF01, and TGF-βRII-mFc all effectively bound to TGF-β1, and the abilities of TF01T and TF01 to bind to TGF-β1 were stronger than that of TGF-βRII-mFc.

### Example 29: Determination of IFN-γ Secretion Promoting Bioactivity of Anti-TIGIT Antibody-TGF-βR Fusion Protein using Mixed Lymphocyte Reaction

Normal human PBMCs (from a healthy donor) were obtained by separation according to the instructions of the Ficoll-PaqueTM Plus separation solution and cryopreserved. After PBMCs were thawed and cultured overnight, the suspended cells were removed, and the adherent cells were cultured in a 1640 complete medium supplemented with 2000 U/mL GM-CSF (purchased from peprotech, Cat. No. 300-03) and 2000 U/mL IL-4 (purchased from peprotech, Cat. No. 200-04) in an incubator at 37 °C with 5% carbon dioxide for three days. A half medium change was performed, and 2000 U/mL GM-CSF, 50 ng/mL IFN-γ (purchased from sinobiological, Cat. No. 11725-HNAS-100), and 100 ng/mL LPS (purchased from SIGMA, Cat. No. L4391) were added. The mixture was incubated in an incubator at 37 °C with 5% carbon dioxide for two days. After the two days, the induced DC cells were routinely collected and cryopreserved.

Two days before the experiment, PBMCs were thawed, and after 2 h, SEB *(Staphylococcus aureus* enterotoxin antigen, purchased from Toxin technology, Cat. No. BT202) (final concentration: 0.1 µg/mL) was added, and the cells were stimulated and induced for 2 days. On the day of the experiment, the SEB-stimulated PBMCs were routinely collected and centrifuged at 250× g for 5 min, and the supernatant was removed. The cells were washed once with an analysis medium (i.e., RPMI 1640 + 10% FBS) and then resuspended and counted. On the day of the experiment, DC cells (induced from PBMCs of a healthy donor) were thawed 2 h in advance and cultured in an incubator at 37 °C with 5% carbon dioxide for 2 h. DCs were collected and centrifuged at 250× g for 5 min, and the supernatant was removed. The cells were resuspended in an analysis medium and counted. Then DC cells (1 × 10⁴/well), PBMCs (1 × 10⁵/well), and A549 human lung cancer cells (purchased from Cell Resource Center, Shanghai Institutes for Biological Sciences, Chinese Academy of Sciences, Cat. No. SCSP-503) (2 × 10⁴/well) were plated into a 96-well flat-bottom plate at 40 µL/well. According to the experimental design, TGF-β1 (final concentration: 3 ng/mL) and the antibodies (final concentration: 3 nM, 30 nM, or 300 nM) were added to the corresponding experimental group wells at 40 µL/well. After thorough mixing, the plate was incubated in an incubator for 5 days. After the 5 days, the supernatant was collected, and the IFN-γ content was determined by ELISA.

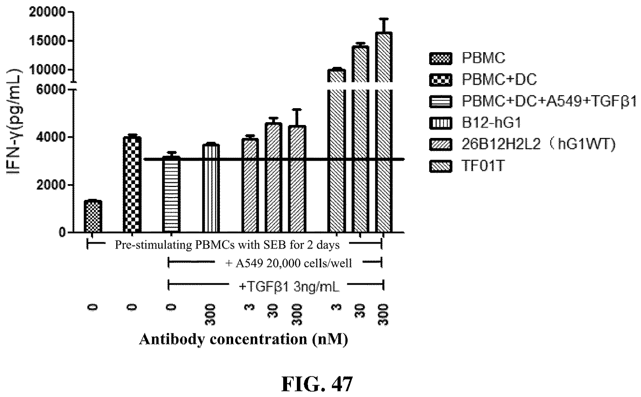

The results are shown in FIG. 47.

The results show that, compared to isotype control B12-hG1, antibodies TF01T and 26B12H2L2(hG1WT) both induced PBMCs to further secrete IFN-y, and the IFN-γ secretion promoting activity of TF01T was stronger than that of anti-TIGIT monoclonal antibody 26B12H2L2(hG1WT).

Although the specific embodiments of the present disclosure have been described in detail, those skilled in the art will appreciate that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. A fusion protein, comprising:
a first protein functional region targeting an immune checkpoint, and
a second protein functional region with TGF-β binding activity,
wherein:
the second protein functional region is a variant of a TGF-βRII extracellular region fragment, and the variant of the TGF-βRII extracellular region fragment is:
the first serine at the N-terminus of the TGF-βRII extracellular region fragment is replaced with alanine, glycine, or threonine, and/or a fragment comprising the amino acid sequence set forth in SEQ ID NO: 59 is deleted from the TGF-βRII extracellular region fragment.

2. The fusion protein according to claim 1, wherein the amino acid sequence of the TGF-βRII extracellular region fragment is set forth in SEQ ID NO: 33 or SEQ ID NO: 37.

3. The fusion protein according to any one of claims 1 to 2, wherein the amino acid sequence of the deleted fragment comprising the amino acid sequence set forth in SEQ ID NO: 59 is set forth in any one of SEQ ID NO: 59 to SEQ ID NO: 65.

4. The fusion protein according to any one of claims 1 to 3, wherein:
the amino acid sequence of the variant of the TGF-βRII extracellular region fragment is set forth in any one of SEQ ID NO: 49 to SEQ ID NO: 58.

5. The fusion protein according to any one of claims 1 to 4, wherein the immune checkpoint is selected from one or more of PD-1, PD-L1, CTLA-4, LAG3, and TIGIT.

6. The fusion protein according to any one of claims 1 to 5, wherein the first protein functional region is an anti-TIGIT antibody or an antigen-binding fragment thereof.

7. The fusion protein according to claim 6, wherein:
the anti-TIGIT antibody comprises a heavy chain variable region comprising HCDR1 to HCDR3 and a light chain variable region comprising LCDR1 to LCDR3, wherein:
the amino acid sequence of HCDR1 is set forth in SEQ ID NO: 3, the amino acid sequence of HCDR2 is set forth in SEQ ID NO: 4, and the amino acid sequence of HCDR3 is set forth in SEQ ID NO: 5, and
the amino acid sequence of LCDR1 is set forth in SEQ ID NO: 8, the amino acid sequence of LCDR2 is set forth in SEQ ID NO: 9, and the amino acid sequence of LCDR3 is set forth in SEQ ID NO: 10.

8. The fusion protein according to claim 7, wherein the amino acid sequence of the heavy chain variable region of the anti-TIGIT antibody is selected from SEQ ID NO: 1, SEQ ID NO: 11, SEQ ID NO: 13, SEQ ID NO: 15, and SEQ ID NO: 17; and
the amino acid sequence of the light chain variable region of the anti-TIGIT antibody is selected from SEQ ID NO: 6, SEQ ID NO: 19, SEQ ID NO: 21, SEQ ID NO: 23, and SEQ ID NO: 25.

9. The fusion protein according to any one of claims 7 to 8, wherein:
the amino acid sequence of the heavy chain variable region of the anti-TIGIT antibody is set forth in SEQ ID NO: 1, and the amino acid sequence of the light chain variable region is set forth in SEQ ID NO: 6;
the amino acid sequence of the heavy chain variable region of the anti-TIGIT antibody is set forth in SEQ ID NO: 11, and the amino acid sequence of the light chain variable region is set forth in SEQ ID NO: 19;
the amino acid sequence of the heavy chain variable region of the anti-TIGIT antibody is set forth in SEQ ID NO: 17, and the amino acid sequence of the light chain variable region is set forth in SEQ ID NO: 19;
the amino acid sequence of the heavy chain variable region of the anti-TIGIT antibody is set forth in SEQ ID NO: 13, and the amino acid sequence of the light chain variable region is set forth in SEQ ID NO: 21;
the amino acid sequence of the heavy chain variable region of the anti-TIGIT antibody is set forth in SEQ ID NO: 13, and the amino acid sequence of the light chain variable region is set forth in SEQ ID NO: 23;
the amino acid sequence of the heavy chain variable region of the anti-TIGIT antibody is set forth in SEQ ID NO: 15, and the amino acid sequence of the light chain variable region is set forth in SEQ ID NO: 21;
the amino acid sequence of the heavy chain variable region of the anti-TIGIT antibody is set forth in SEQ ID NO: 15, and the amino acid sequence of the light chain variable region is set forth in SEQ ID NO: 23;
the amino acid sequence of the heavy chain variable region of the anti-TIGIT antibody is set forth in SEQ ID NO: 11, and the amino acid sequence of the light chain variable region is set forth in SEQ ID NO: 25; or
the amino acid sequence of the heavy chain variable region of the anti-TIGIT antibody is set forth in SEQ ID NO: 17, and the amino acid sequence of the light chain variable region is set forth in SEQ ID NO: 25.

10. The fusion protein according to any one of claims 7 to 9, wherein the anti-TIGIT antibody or the antigen-binding fragment thereof is selected from Fab, Fab', F(ab')2, Fd, Fv, dAb, a complementarity determining region fragment, a single chain fragment variable, a humanized antibody, a chimeric antibody, and a diabody.

11. The fusion protein according to any one of claims 7 to 10, wherein:
the anti-TIGIT antibody comprises a non-CDR region, and the non-CDR region is derived from a non-murine species, e.g., from a human antibody.

12. The fusion protein according to any one of claims 7 to 11, wherein a heavy chain constant region of the anti-TIGIT antibody is an Ig gamma-1 chain C region or an Ig gamma-4 chain C region, and a light chain constant region is an Ig kappa chain C region.

13. The fusion protein according to any one of claims 7 to 12, wherein the fusion protein has a stronger ability to bind to ligand CD155-hFc-Biotin than control antibody RG6058(hG4).

14. The fusion protein according to any one of claims 7 to 13, wherein the fusion protein binds to TIGIT-mFc with an EC₅₀ of less than 0.08 nM or less than 0.10 nM; preferably, the EC₅₀ is measured by indirect ELISA.

15. The fusion protein according to any one of claims 7 to 14, wherein the fusion protein binds to TGF-β1, TGF-β2, or TGF-β3 with an EC₅₀ of less than 0.3 nM, less than 0.4 nM, less than 0.5 nM, or less than 0.6; preferably, the EC₅₀ is measured by indirect ELISA.

16. The fusion protein according to any one of claims 7 to 15, wherein the anti-TIGIT antibody is an antibody produced by hybridoma cell line LT019, and the hybridoma cell line LT019 is deposited at the China Center for Type Culture Collection (CCTCC) under CCTCC NO. C2020208.

17. The fusion protein according to any one of claims 7 to 16, wherein according to the EU numbering system,
when the heavy chain constant region of the anti-TIGIT antibody is IgG1, the heavy chain constant region has one of the following mutation combinations:
L234A and L235A;
L234A and G237A;
L235A and G237A; or
L234A, L235A, and G237A;
when the heavy chain constant region of the anti-TIGIT antibody is IgG4, the heavy chain constant region has one of the following mutation combinations:
F234A and L235A;
F234A and G237A;
L235A and G237A; or
F234A, L235A, and G237A.

18. The fusion protein according to any one of claims 7 to 17, wherein:
the amino acid sequence of a heavy chain of the anti-TIGIT antibody is set forth in SEQ ID NO: 27 or SEQ ID NO: 31, and the amino acid sequence of a light chain thereof is set forth in SEQ ID NO: 29.

19. The fusion protein according to any one of claims 1 to 18, wherein the first protein functional region and the second protein functional region are linked directly or by a linker fragment.

20. The fusion protein according to claim 19, wherein the linker fragment is a polypeptide set forth in SEQ ID NO: 69 or SEQ ID NO: 70, or a polypeptide obtained by concatenating a plurality of (e.g., 2, 3, 4, 5, or 6) polypeptides set forth in SEQ ID NO: 69, or a polypeptide obtained by concatenating a plurality of (e.g., 2, 3, 4, or 5) polypeptides set forth in SEQ ID NO: 69 and further concatenating the concatenated polypeptides and a polypeptide set forth in SEQ ID NO: 70;
preferably, the amino acid sequence of the linker fragment is set forth in SEQ ID NO: 44 or SEQ ID NO: 66.

21. The fusion protein according to any one of claims 1 to 20, wherein the first protein functional region and the second protein functional region are independently 1, 2, or more in number.

22. The fusion protein according to any one of claims 1 to 21, wherein the second protein functional region is linked to the C-terminus of the first protein functional region.

23. The fusion protein according to any one of claims 7 to 22, wherein the second protein functional region is linked to the C-terminus of the heavy chain of the anti-TIGIT antibody.

24. A fusion protein, comprising:
a first protein functional region targeting TIGIT, and
a second protein functional region with TGF-β binding activity,
wherein:
the first protein functional region is 1 in number, and the second protein functional region is 2 in number;
the first protein functional region is an anti-TIGIT antibody or an antigen-binding fragment thereof, and the second protein functional region is a variant of a TGF-βRII extracellular region fragment;
the amino acid sequence of a heavy chain of the anti-TIGIT antibody is set forth in SEQ ID NO: 27 or SEQ ID NO: 31, and the amino acid sequence of a light chain thereof is set forth in SEQ ID NO: 29;
the amino acid sequence of the variant of the TGF-βRII extracellular region fragment is set forth in any one of SEQ ID NO: 49 to SEQ ID NO: 58;
the second protein functional region is linked to the C-terminus of the heavy chain of the anti-TIGIT antibody by a linker fragment;
preferably, the amino acid sequence of the linker fragment is set forth in SEQ ID NO: 44 or SEQ ID NO: 66.

25. An isolated nucleic acid molecule, wherein the isolated nucleic acid molecule encodes the fusion protein according to any one of claims 1 to 24.

26. A vector, comprising the isolated nucleic acid molecule according to claim 25.

27. A host cell, comprising the isolated nucleic acid molecule according to claim 25 or the vector according to claim 26.

28. A conjugate, comprising a fusion protein moiety and a conjugated moiety, wherein the fusion protein moiety is the fusion protein according to any one of claims 1 to 24, and the conjugated moiety is a detectable label; preferably, the conjugated moiety is a radioisotope, a fluorescent substance, a colored substance, or an enzyme.

29. A pharmaceutical composition, comprising the fusion protein according to any one of claims 1 to 24 or the conjugate according to claim 28, wherein optionally, the pharmaceutical composition further comprises one or more pharmaceutically acceptable auxiliary materials; optionally, the pharmaceutical composition further comprises one or more anti-tumor chemotherapeutic drugs.

30. The pharmaceutical composition according to claim 29, wherein a unit dose of the pharmaceutical composition is 100 mg-1500 mg, 200 mg-1000 mg, 200 mg-800 mg, 300 mg-600 mg, 400 mg-500 mg, or 450 mg, as calculated based on the mass of the fusion protein therein.

31. Use of the fusion protein according to any one of claims 1 to 24 or the conjugate according to claim 28 in the preparation of a medicament for treating and/or preventing a tumor, wherein:
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

32. The fusion protein according to any one of claims 1 to 24 or the conjugate according to claim 28 for use in the treatment and/or prevention of a tumor, wherein:
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

33. A method for treating or preventing a tumor, comprising a step of administering to a subject in need thereof an effective amount of the fusion protein according to any one of claims 1 to 24 or the conjugate according to claim 28, wherein:
preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, B-lymphoma, plasma cell cancer, renal cell cancer, prostate cancer, and pancreatic ductal adenocarcinoma;
preferably, the non-small cell lung cancer is advanced non-small cell lung cancer.

34. A variant of a TGF-βRII extracellular region fragment, wherein the variant is:
the first serine at the N-terminus of the TGF-βRII extracellular region fragment is replaced with alanine, glycine, or threonine; or,
a fragment comprising the amino acid sequence set forth in SEQ ID NO: 59 is deleted from the TGF-βRII extracellular region fragment;
preferably, the amino acid sequence of the TGF-βRII extracellular region fragment is set forth in SEQ ID NO: 33 or SEQ ID NO: 37.

35. The variant of the TGF-βRII extracellular region fragment according to claim 34, wherein the amino acid sequence of the deleted fragment comprising the amino acid sequence set forth in SEQ ID NO: 59 is set forth in any one of SEQ ID NO: 59 to SEQ ID NO: 65.

36. The variant of the TGF-βRII extracellular region fragment according to any one of claims 34 to 35, wherein:
the amino acid sequence of the variant of the TGF-βRII extracellular region fragment is set forth in any one of SEQ ID NO: 49 to SEQ ID NO: 58.
